# EUROPEAN PATENT APPLICATION

(11) **EP 4 740 942 A1**
(43) Date of publication of application: **13.05.2026**
(21) Application number: 24835292.4
(22) Date of filing: 28.06.2024
(51) Int. Cl.: A61K 31/166, C07C 307/10, A61K 31/444, A61P 35/00

(54) **AROMATIC AMIDE DERIVATIVE AND USE THEREOF**

(30) Priority: 06.07.2023 CN 202310825593
(71) Applicant: HUAJIAN FUTURE (CHENGDU) TECHNOLOGY CO., LTD., Sichuan 611130 (CN)
(72) Inventor: DU, Fengtian, Chengdu, Sichuan 611130 (CN); YANG, Xiangyu, Chengdu, Sichuan 611130 (CN); GUO, Na, Chengdu, Sichuan 611130 (CN); LUO, Shuai, Chengdu, Sichuan 611130 (CN); TIAN, Jingyu, Chengdu, Sichuan 611130 (CN); QIAN, Qingqing, Chengdu, Sichuan 611130 (CN)
(74) Representative: Kador & Partner Part mbB
(86) International application number: PCT/CN2024/102442
(87) International publication number: WO 2025/007811

(57) **Abstract**

An aromatic amide derivative, a preparation method therefor, and use of the compound in preventing and/or treating a disease, especially in tumor treatment. The aromatic amide derivative is a MEK/RAF inhibitor having advantages such as relatively good activity and safety, and shows good application prospects in preventing and/or treating a tumor related to a MAPK pathway.

## Description

### TECHNICAL FIELD

The present invention belongs to the field of medicinal chemistry, and relates to an aromatic amide derivative, a preparation method therefor, and use of the compound in preventing and/or treating a disease, especially in tumor treatment.

### BACKGROUND ART

Aromatic amide structure is widely found in drug molecules, which is an important pharmacophore, may interact with a drug target protein, and can improve drug performance and the like. For example, all the following marketed drugs or molecular structures in the clinical stage contain an aromatic amide structure.

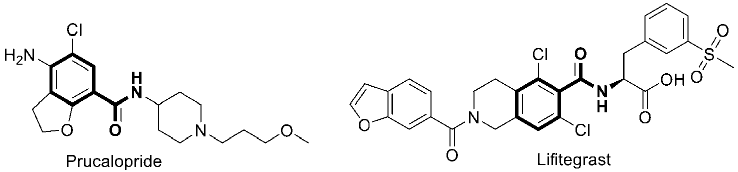

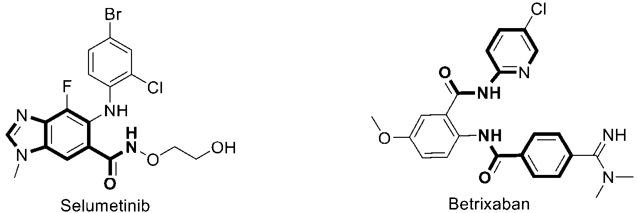

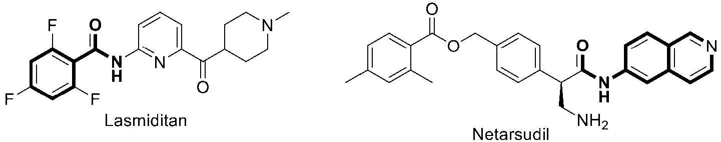

RAS-RAF-MEK-ERK cascade reaction is an important signal pathway, which is one of the most actively studied pathways. Abnormal activation of this signal pathway will affect the proliferation, differentiation, apoptosis and metastasis of normal cells. In addition to lung cancer, the abnormal activation of this pathway has a larger proportion and a greater influence in high mortality cancer types, such as pancreatic cancer (> 90%) and colorectal cancer (> 50%). RAF is a serine/threonine protein kinase, which is usually activated by a RAS protein kinase. MEK is a bispecific kinase, which may phosphorylate a serine/threonine residue and a tyrosine residue. The activated MEK activates a downstream ERK, so as to make the kinase enter cell nuclei to regulate various transcription factors and induce the expression of some genes, thereby causing cell proliferation. The blockage of only a single target of this pathway is often insufficient to achieve an ideal anti-tumor effect, and a single-target drug has defects such as easy mutation and drug resistance, and great toxic and side effects. At present, the Raf and MEK inhibitors are used in combination to block two key targets of this pathway, so as to overcome drug resistance caused by the reactivation of this pathway, thereby delaying the emergence of drug resistance.

It is an effective anti-tumor strategy to develop a RAF and MEK dual-target inhibitor. At present, among the RAF/MEK inhibitors under research, VS-6766 (related literatures such as WO2007091736) has made rapid clinical progress, which has shown efficacy in the treatment of various tumors activated by a MAPK pathway, and related adverse reactions have also been reported in clinical experiments; and another type of compound also shows activity in a lung cancer model (related literatures such as WO2021149776).

The development of the RAF and MEK dual-target inhibitor is to improve the inhibitory activity and effectiveness of the compound on one hand, and needs to pay attention to the safety on the other hand. It is of great significance to develop a new RAF and MEK dual-target inhibitor with better efficacy and higher safety.

### SUMMARY

The present invention discloses an aromatic amide MEK/RAF inhibitor, a stereoisomer thereof, a tautomer thereof or a pharmaceutically acceptable salt thereof, which has advantages such as good activity and safety, and shows good application prospects in preventing and/or treating a tumor related to a MAPK pathway.

The present invention provides a compound represented by formula (A), or a stereoisomer, a tautomer or a pharmaceutically acceptable salt thereof, 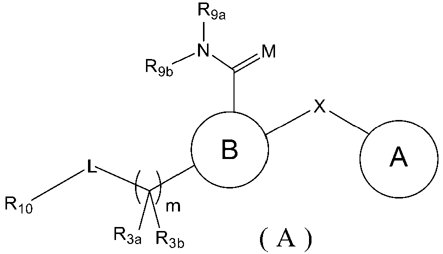
wherein, M is selected from oxygen or sulfur;
X is selected from a bond, oxygen, sulfur, -C(O)-, -NR₁C(O)-, -C(O)NR₁-, -NR₁S(O)-, -S(O)NR₁-, -NR₁SO₂-, -SO₂NR₁-, -NR₁-, or -CR₁R₂-;
each of ring A and ring B is independently selected from substituted or unsubstituted cycloalkyl, substituted or unsubstituted heterocyclyl, substituted or unsubstituted aryl, and substituted or unsubstituted heteroaryl;
L is selected from a bond, oxygen, sulfur, -C(O)-, -NR₁C(O)-, -C(O)NR₁-, -NR₁S(O)-, -S(O)NR₁-, -NR₁SO₂-, -SO₂NR₁-, -NR₁-, substituted or unsubstituted cycloalkyl, substituted or unsubstituted heterocyclyl, substituted or unsubstituted aryl, and substituted or unsubstituted heteroaryl;
R₁₀ is selected from hydrogen, deuterium, halogen, aldehyde group, cyano, hydroxyl, -ORₐ, -SRₐ, -NRₐR_{b}, -C(O)Rₐ, -S(O)Rₐ, -S(O)₂Rₐ, -(CR₂ₐR_{2b})ₚSO₂NRₐR_{b}, -(CR₂ₐR₂₈)ₚNRₐSO₂NR_{d}R_{c}, -(CR₂ₐR_{2b})ₚNRₐSO₂R_{b}, -(CR₂ₐR_{2b})ₚC(O)NRₐR_{b}, -(CR₂ₐR_{2b})ₚNRₐC(O)NR_{b}R_{c}, -(CR₂ₐR_{2b})ₚNRₐC(O)R_{b}, substituted or unsubstituted amino, substituted or unsubstituted alkyl, substituted or unsubstituted deuterated alkyl, substituted or unsubstituted haloalkyl, substituted or unsubstituted alkoxy, substituted or unsubstituted alkenyl, substituted or unsubstituted alkynyl, substituted or unsubstituted cycloalkyl, substituted or unsubstituted heterocyclyl, substituted or unsubstituted aryl, and substituted or unsubstituted heteroaryl;
each of R₉ₐ and R_{9b} is independently selected from hydrogen, deuterium, halogen, aldehyde group, cyano, hydroxyl, -ORₐ, -SRₐ, -NRₐR_{b}, -C(O)Rₐ, -S(O)Rₐ, -S(O)₂Rₐ, substituted or unsubstituted amino, substituted or unsubstituted alkyl, substituted or unsubstituted deuterated alkyl, substituted or unsubstituted haloalkyl, substituted or unsubstituted alkoxy, substituted or unsubstituted alkenyl, substituted or unsubstituted alkynyl, substituted or unsubstituted cycloalkyl, substituted or unsubstituted heterocyclyl, substituted or unsubstituted aryl, and substituted or unsubstituted heteroaryl; or two adjacent substituents together form a substituted or unsubstituted 3-15 membered cyclic structure;
each of R₁, R₂, R₂ₐ, R_{2b}, R₃ₐ, R_{3b}, Rₐ, R_{b}, and R_{c} is independently selected from hydrogen, deuterium, halogen, aldehyde group, cyano, hydroxyl, -ORₐ, -SRₐ, -NRₐR_{b}, -C(O)Rₐ, -S(O)Rₐ, -S(O)₂Rₐ, substituted or unsubstituted amino, substituted or unsubstituted alkyl, substituted or unsubstituted deuterated alkyl, substituted or unsubstituted haloalkyl, substituted or unsubstituted alkoxy, substituted or unsubstituted alkenyl, substituted or unsubstituted alkynyl, substituted or unsubstituted cycloalkyl, substituted or unsubstituted heterocyclyl, substituted or unsubstituted aryl, and substituted or unsubstituted heteroaryl; or two adjacent substituents together form a substituted or unsubstituted 3-15 membered cyclic structure;
m is selected from 0, 1, 2, 3, or 4; and
p is selected from 0, 1, 2, 3, or 4.

The present invention provides a compound represented by formula (A-1), or a stereoisomer, a tautomer or a pharmaceutically acceptable salt thereof, 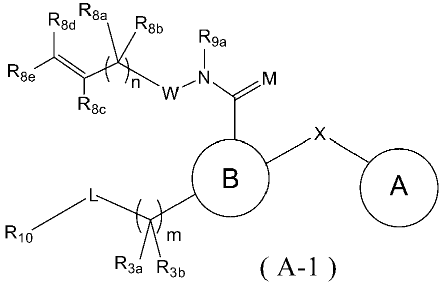
wherein, W is selected from a bond, oxygen, or sulfur;
M is selected from oxygen or sulfur;
X is selected from a bond, oxygen, sulfur, -C(O)-, -NR₁C(O)-, -C(O)NR₁-, -NR₁S(O)-, -S(O)NR₁-, -NR₁SO₂-, -SO₂NR₁-, -NR₁-, or -CR₁R₂-;
each of ring A and ring B is independently selected from substituted or unsubstituted cycloalkyl, substituted or unsubstituted heterocyclyl, substituted or unsubstituted aryl, and substituted or unsubstituted heteroaryl;
L is selected from a bond, oxygen, sulfur, -C(O)-, -NR₁C(O)-, -C(O)NR₁-, -NR₁S(O)-, -S(O)NR₁-, -NR₁SO₂-, -SO₂NR₁-, -NR₁-, substituted or unsubstituted cycloalkyl, substituted or unsubstituted heterocyclyl, substituted or unsubstituted aryl, and substituted or unsubstituted heteroaryl;
R₁₀ is selected from hydrogen, deuterium, halogen, aldehyde group, cyano, hydroxyl, -ORₐ, -SRₐ, -NRₐR_{b}, -C(O)Rₐ, -S(O)Rₐ, -S(O)₂Rₐ, -(CR₂ₐR_{2b})ₚSO₂NRₐR_{b}, -(CR₂ₐR_{2b})ₚNRₐSO₂NR_{b}R_{c}, -(CR₂ₐR_{2b})ₚNRₐSO₂R_{b}, -(CR₂ₐR_{2b})ₚC(O)NRₐR_{b}, -(CR₂ₐR₂₆)ₚNRₐC(O)NR_{d}R_{c}, -(CR₂ₐR_{2b})ₚNRₐC(O)R_{b}, substituted or unsubstituted amino, substituted or unsubstituted alkyl, substituted or unsubstituted deuterated alkyl, substituted or unsubstituted haloalkyl, substituted or unsubstituted alkoxy, substituted or unsubstituted alkenyl, substituted or unsubstituted alkynyl, substituted or unsubstituted cycloalkyl, substituted or unsubstituted heterocyclyl, substituted or unsubstituted aryl, and substituted or unsubstituted heteroaryl;
each of R₁, R₂, R₂ₐ, R_{2b}, R₃ₐ, R_{3b}, Rₐ, R_{b} and R_{c} is independently selected from hydrogen, deuterium, halogen, aldehyde group, cyano, hydroxyl, -ORₐ, -SRₐ, -NRₐR_{b}, -C(O)Rₐ, -S(O)Rₐ, -S(O)₂Rₐ, substituted or unsubstituted amino, substituted or unsubstituted alkyl, substituted or unsubstituted deuterated alkyl, substituted or unsubstituted haloalkyl, substituted or unsubstituted alkoxy, substituted or unsubstituted alkenyl, substituted or unsubstituted alkynyl, substituted or unsubstituted cycloalkyl, substituted or unsubstituted heterocyclyl, substituted or unsubstituted aryl, and substituted or unsubstituted heteroaryl; or two adjacent substituents together form a substituted or unsubstituted 3-15 membered cyclic structure;
each of R₈ₐ, R_{8b}, R_{8c}, R_{8d}, R₈ₑ and R₉ₐ is independently selected from hydrogen, deuterium, halogen, aldehyde group, cyano, hydroxyl, -ORa, -SRₐ, -NRₐR_{b}, -C(O)Rₐ, -S(O)Rₐ, -S(O)₂Rₐ, substituted or unsubstituted amino, substituted or unsubstituted alkyl, substituted or unsubstituted deuterated alkyl, substituted or unsubstituted haloalkyl, substituted or unsubstituted alkoxy, substituted or unsubstituted alkenyl, substituted or unsubstituted alkynyl, substituted or unsubstituted cycloalkyl, substituted or unsubstituted heterocyclyl, substituted or unsubstituted aryl, and substituted or unsubstituted heteroaryl; or two adjacent substituents together form a substituted or unsubstituted 3-15 membered cyclic structure;
m is 0, 1, 2, 3, or 4;
n is 1, 2, 3, 4, or 5; and
p is 0, 1, 2, 3, or 4.

The present invention provides a compound represented by formula (I), or a stereoisomer, a tautomer or a pharmaceutically acceptable salt thereof, 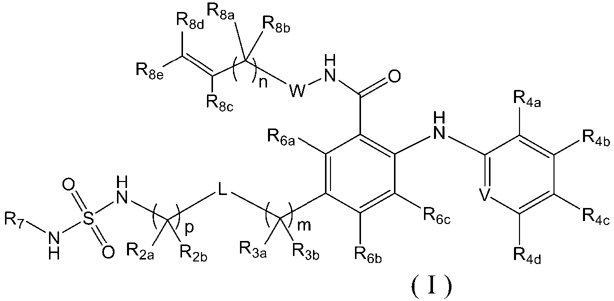
wherein, W is selected from a bond, oxygen, or sulfur;
V is selected from nitrogen or CR₄ₑ;
L is selected from -NR₁C(O)-, -C(O)NR₁-, C₃₋₆ cycloalkyl, 5-10 membered heterocyclyl, C₆₋₁₀ aryl, or 5-10 membered heteroaryl;
R₁ is selected from hydrogen, halogen, C₁₋₆ alkyl, C₁₋₆ deuterated alkyl, C₁₋₆ haloalkyl, C₁₋₆ alkoxy, C₂₋₆ alkenyl, C₂₋₆ alkynyl, or C₃₋₆ cycloalkyl;
each of R₂ₐ, R_{2b}, R₃ₐ, R_{3b}, R₄ₐ, R_{4b}, R_{4c}, R_{4d}, R₄ₑ, R₆ₐ, R_{6b} and R_{6c} is independently selected from hydrogen, deuterium, halogen, aldehyde group, cyano, amino, hydroxyl, C₁₋₆ alkyl, C₁₋₆ deuterated alkyl, C₁₋₆ haloalkyl, C₁₋₆ alkoxy, C₂₋₆ alkenyl, C₂₋₆ alkynyl or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl or C₂₋₆ alkynyl, wherein the alkyl, alkoxy, alkenyl, alkynyl, and cycloalkyl are optionally substituted with one or more substituents selected from deuterium, halogen, amino, C₁₆ alkyl, C₁₋₆ alkoxy, C₂₋₆ alkenyl, C₂₋₆ alkynyl, and C₃₋₆ cycloalkyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl, C₁₋₆ haloalkyl, or C₃₋₆ cycloalkyl;
m is 0, 1, 2, or 3;
n is 1, 2, 3, or 4; and
p is 0, 1, 2, or 3.

The present invention provides a compound represented by formula (II), or a stereoisomer, a tautomer or a pharmaceutically acceptable salt thereof, 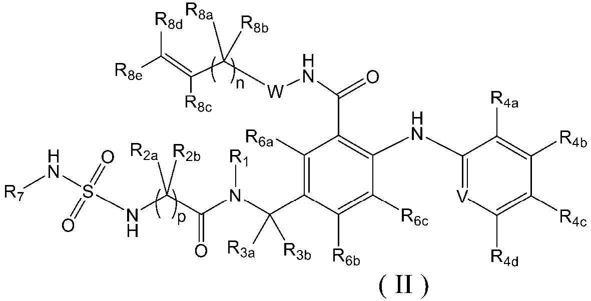
wherein, W is selected from a bond, oxygen, or sulfur;
V is selected from nitrogen or CR₄ₑ;
R₁ is selected from hydrogen, halogen, C₁₋₆ alkyl, C₁₋₆ deuterated alkyl, C₁₋₆ haloalkyl, C₁₋₆ alkoxy, C₂₋₆ alkenyl, C₂₋₆ alkynyl, or C₃₋₆ cycloalkyl;
each of R₂ₐ, R_{2b}, R₃ₐ, R_{3b}, R₄ₐ, R_{4b}, R_{4c}, R_{4d}, R₄ₑ, R₆ₐ, R_{6b} and R_{6c} is independently selected from hydrogen, deuterium, halogen, aldehyde group, cyano, amino, hydroxyl, C₁₋₆ alkyl, C₁₋₆ deuterated alkyl, C₁₋₆ haloalkyl, C₁₋₆ alkoxy, C₂₋₆ alkenyl, C₂₋₆ alkynyl, or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl, or C₂₋₆ alkynyl, wherein the alkyl, alkoxy, alkenyl, alkynyl, and cycloalkyl are optionally substituted with one or more substituents selected from deuterium, halogen, amino, C₁₋₆ alkyl, C₁₋₆ alkoxy, C₂₋₆ alkenyl, C₂₋₆ alkynyl, and C₃₋₆ cycloalkyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl, C₁₋₆ haloalkyl, or C₃₋₆ cycloalkyl;
n is 1, 2, 3, or 4; and
p is 0, 1, 2, or 3.

The present invention provides a compound as follows,or a stereoisomer, a tautomer or a pharmaceutically acceptable salt thereof,

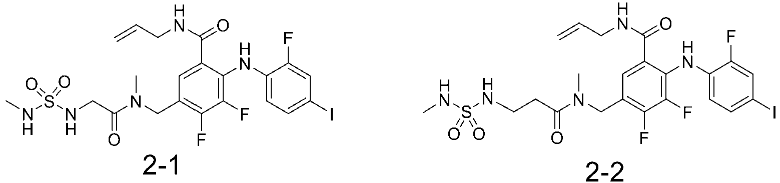

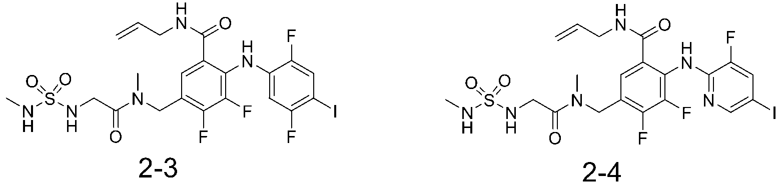

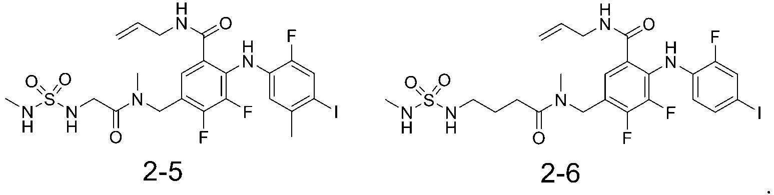

The present invention provides a compound represented by formula (III), or a stereoisomer, a tautomer or a pharmaceutically acceptable salt thereof, 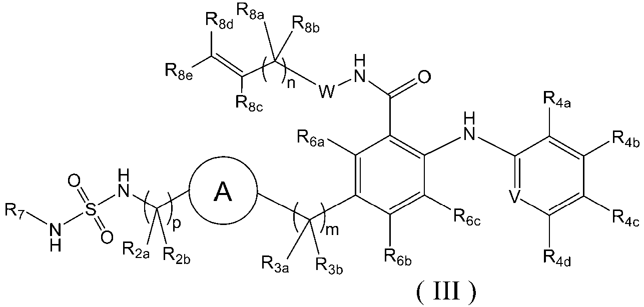
wherein, W is selected from a bond, oxygen, or sulfur;
V is selected from nitrogen or CR₄ₑ;
ring A is selected from C₆₋₁₀ aryl or 5-10 membered heteroaryl;
each of R₂ₐ, R_{2b}, R₃ₐ, R_{3b}, R₄ₐ, R_{4b}, R_{4c}, R_{4d}, R₄ₑ, R₆ₐ, R_{6b} and R_{6c} is independently selected from hydrogen, deuterium, halogen, aldehyde group, cyano, amino, hydroxyl, C₁₋₆ alkyl, C₁₋₆ deuterated alkyl, C₁₋₆ haloalkyl, C₁₋₆ alkoxy, C₂₋₆ alkenyl, C₂₋₆ alkynyl, or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl, or C₂₋₆ alkynyl, wherein the alkyl, alkoxy, alkenyl, alkynyl, and cycloalkyl are optionally substituted with one or more substituents selected from deuterium, halogen, amino, C₁₋₆ alkyl, C₁₋₆ alkoxy, C₂₋₆ alkenyl, C₂₋₆ alkynyl, and C₃₋₆ cycloalkyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl, C₁₋₆ haloalkyl, or C₃₋₆ cycloalkyl;
m is 0, 1, 2, or 3;
n is 1, 2, 3, or 4; and
p is 0, 1, 2, or 3.

The present invention provides a compound as follows, or a stereoisomer , a tautomer or a pharmaceutically acceptable salt thereof,

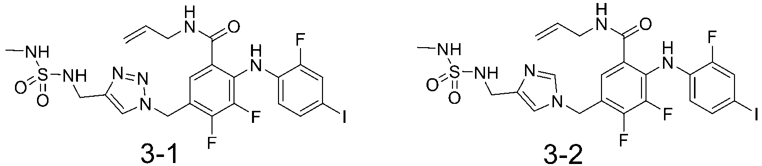

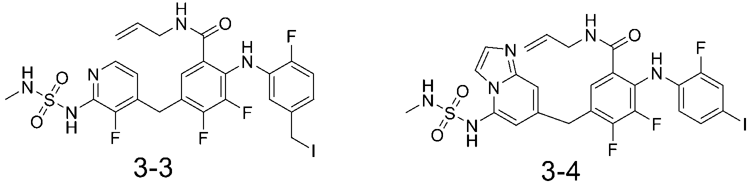

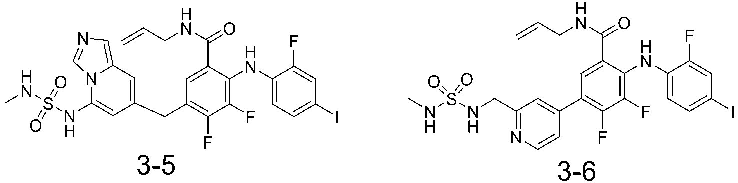

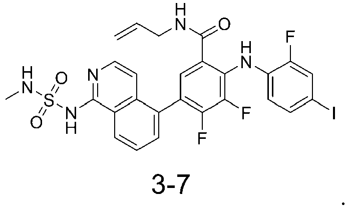

The present invention provides a compound represented by formula (IV), or a stereoisomer, a tautomer or a pharmaceutically acceptable salt thereof, 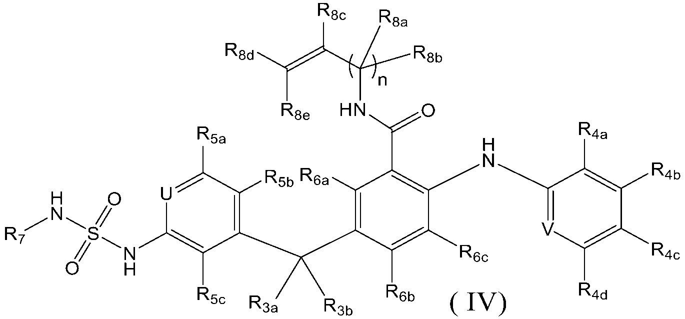
wherein, U is selected from nitrogen or CR_{5d};
V is selected from nitrogen or CR₄ₑ;
each of R₃ₐ, R3b, R₄ₐ, R4b, R_{4c}, R_{4d}, R₄ₑ, R₅ₐ, R_{5b}, R_{8c}, R_{5d}, R₆ₐ, R_{6b} and R_{6c} is independently selected from hydrogen, deuterium, halogen, aldehyde group, cyano, amino, hydroxyl, C₁₋₆ alkyl, C₁₋₆ deuterated alkyl, C₁₋₆ haloalkyl, C₁₋₆ alkoxy, C₂₋₆ alkenyl, C₂₋₆ alkynyl, or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl, or C₂₋₆ alkynyl, wherein the alkyl, alkoxy, alkenyl, alkynyl and cycloalkyl are optionally substituted with one or more substituents selected from deuterium, halogen, amino, C₁₋₆ alkyl, C₁₋₆ alkoxy, C₂₋₆ alkenyl, C₂₋₆ alkynyl and C₃₋₆ cycloalkyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl, C₁₋₆ haloalkyl or C₃₋₆ cycloalkyl; and
n is 1, 2, 3, or 4.

The present invention provides a compound represented by formula (IV-1), or a stereoisomer, a tautomer or a pharmaceutically acceptable salt thereof, 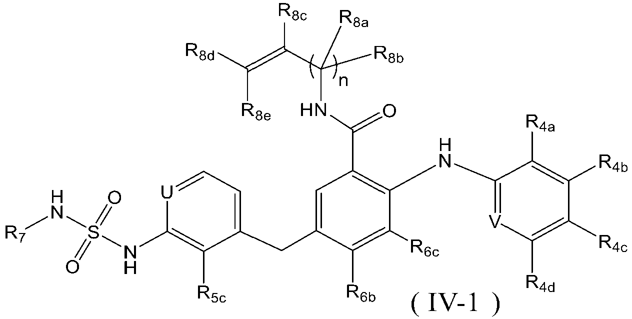
wherein, U is selected from nitrogen or CH;
V is selected from nitrogen or CR₄ₑ;
each of R₄ₐ, R_{4b}, R_{4c}, R_{4d} and R₄ₑ is independently selected from hydrogen, deuterium, halogen, aldehyde group, hydroxyl, C₁₋₆ alkyl, C₁₋₆ alkoxy, C₂₋₆ alkenyl, C₂₋₆ alkynyl or C₃₋₆ cycloalkyl;
each of R_{5c}, R_{6b} and R_{6c} is independently selected from hydrogen, halogen or C₁₋₆ alkyl;
R₇ is selected from hydrogen, C₁₋₆ alkyl, C₁₋₆ deuterated alkyl or C₃₋₆ cycloalkyl;
each of R₈ₐ, R_{8b}, R_{8c}, R_{8d} and R₈ₑ is independently selected from hydrogen, deuterium or C₁₋₆ alkyl; and
n is 1, 2, or 3.

The present invention provides a compound as follows, or a stereoisomer, a tautomer or a pharmaceutically acceptable salt thereof,

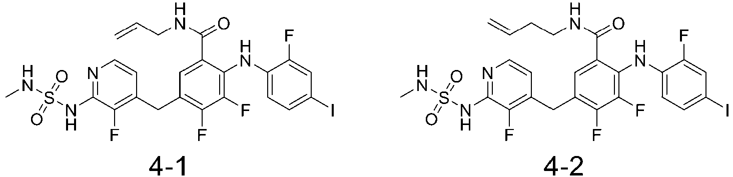

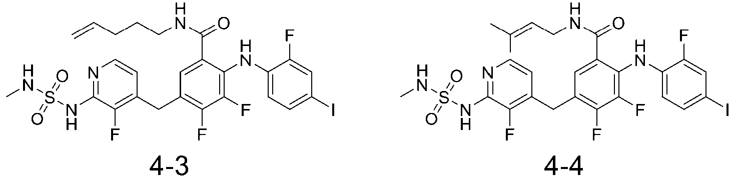

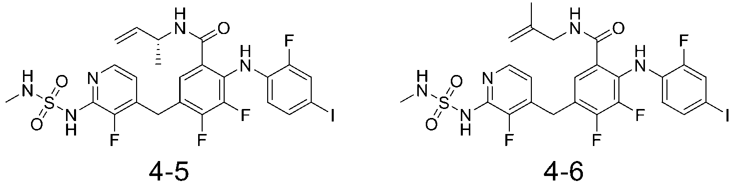

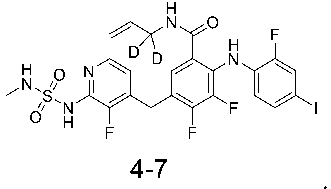

The present invention provides a compound as follows, or a stereoisomer, a tautomer or a pharmaceutically acceptable salt thereof,

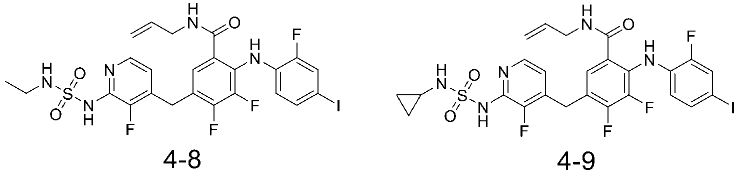

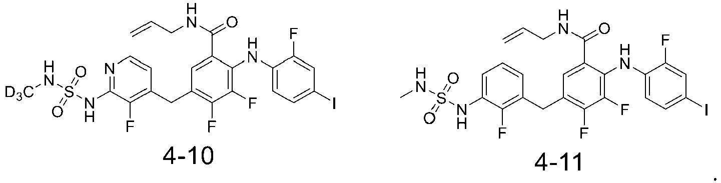

The present invention provides a compound as follows, or a stereoisomer, a tautomer or a pharmaceutically acceptable salt thereof,

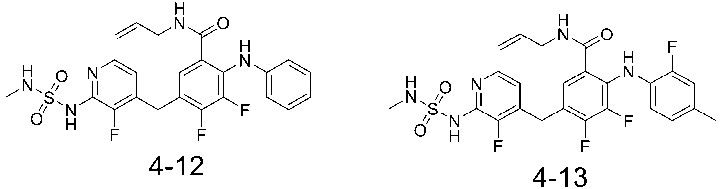

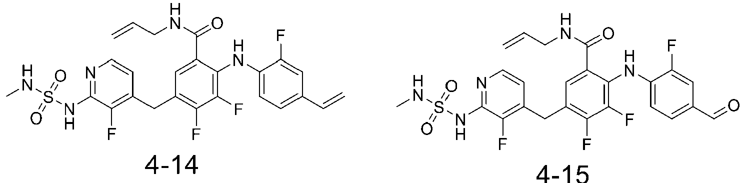

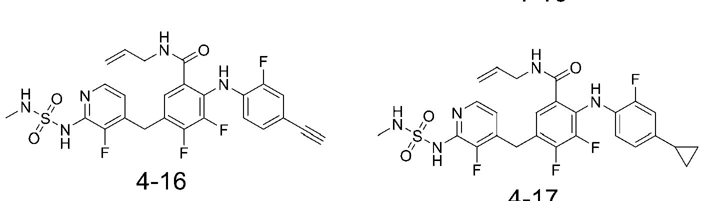

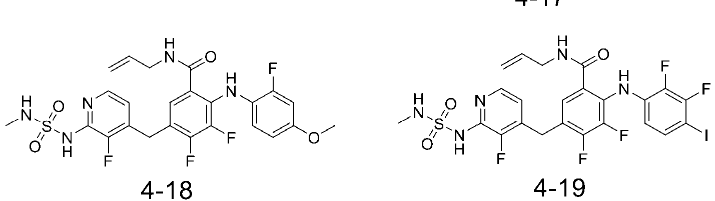

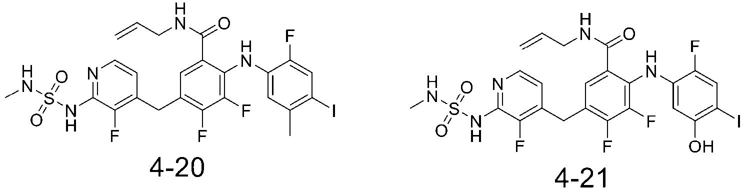

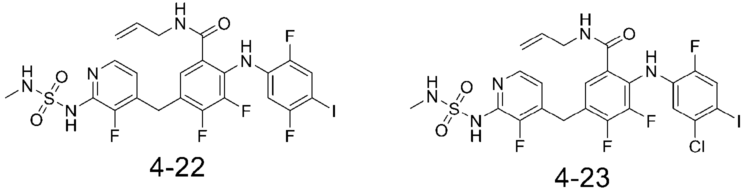

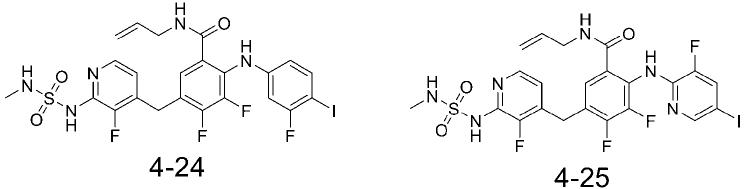

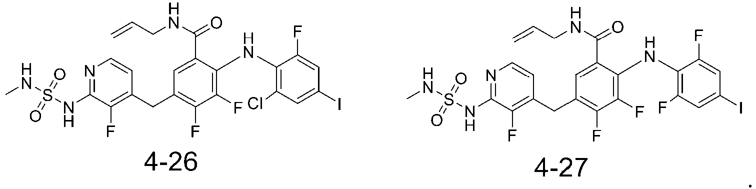

The present invention provides a compound represented by formula (V), or a stereoisomer, a tautomer or a pharmaceutically acceptable salt thereof, 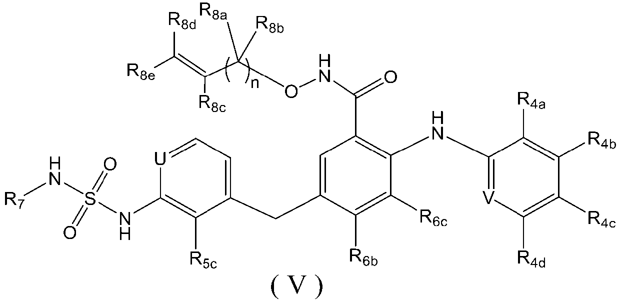
wherein, U is selected from nitrogen or CH;
V is selected from nitrogen or CR₄ₑ;
each of R₄ₐ, R_{4b}, R_{4c}, R_{4d}, R₄ₑ, R_{8c}, R_{6b} and R_{6c} is independently selected from hydrogen, deuterium, halogen, aldehyde group, cyano, amino, hydroxyl, C₁₋₆ alkyl, C₁₋₆ deuterated alkyl, C₁₋₆ haloalkyl, C₁₋₆ alkoxy, C₂₋₆ alkenyl, C₂₋₆ alkynyl, or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl, or C₂₋₆ alkynyl, wherein the alkyl, alkoxy, alkenyl, alkynyl, and cycloalkyl are optionally substituted with one or more substituents selected from deuterium, halogen, amino, C₁₋₆ alkyl, C₁₋₆ alkoxy, C₂₋₆ alkenyl, C₂₋₆ alkynyl and C₃₋₆ cycloalkyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl, C₁₋₆ haloalkyl or C₃₋₆ cycloalkyl; and
n is 1, 2, 3, or 4.

The present invention provides a compound as follows, or a stereoisomer, a tautomer or a pharmaceutically acceptable salt thereof,

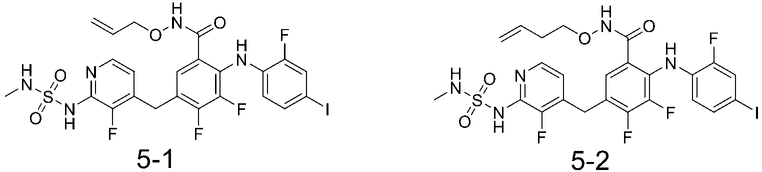

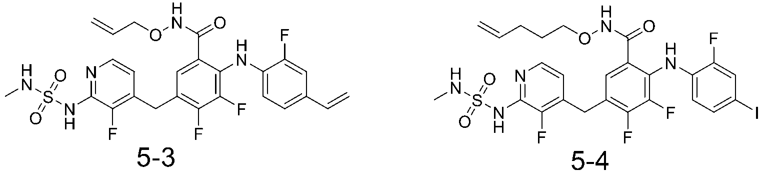

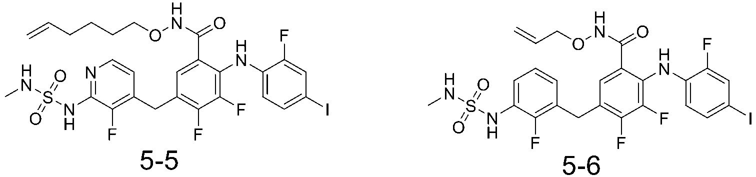

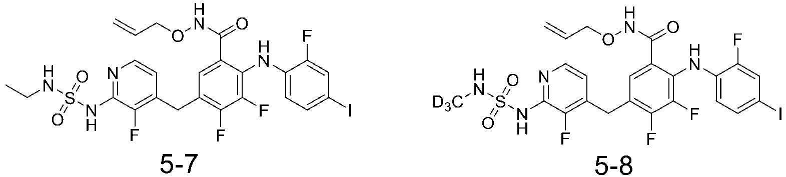

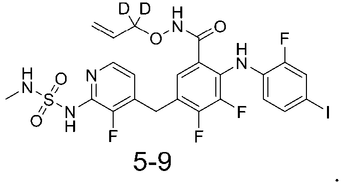

The present invention provides a pharmaceutical composition, which comprises a therapeutically effective amount of the compound, or the stereoisomer, the tautomer or the pharmaceutically acceptable salt thereof according to any one of items of the present invention, and a pharmaceutically acceptable carrier.

The present invention provides use of the compound, or the stereoisomer, the tautomer or the pharmaceutically acceptable salt thereof according to any one of items of the present invention, or the pharmaceutical composition of the present invention in the preparation of a medicament for preventing and/or treating a tumor.

The present invention provides use of the compound, or the stereoisomer, the tautomer or the pharmaceutically acceptable salt thereof according to any one of items of the present invention, or the pharmaceutical composition of the present invention in the preparation of a medicament for preventing and/or treating a disease mediated by RAF and/or MEK, wherein the disease is preferably a tumor.

According to the use in the present invention, the tumor comprises kidney cancer, liver cancer, breast cancer, pancreatic cancer, prostate cancer, melanoma, leukemia, malignant lymphoma, ovarian cancer, head and neck cancer, lung cancer, colorectal cancer, bladder cancer, uterine cancer, and the like.

The present invention further provides a method for inhibiting a RAF/MEK protein in cells, which includes contacting the cells with an effective amount of the compound, or the stereoisomer, the tautomer or the pharmaceutically acceptable salt thereof according to the present invention, or the pharmaceutical composition of the present invention.

### Detailed description

All the technical and scientific terms used in the specification have the same meanings as those commonly understood by those of ordinary skills in the art.

The term "hydrogen" herein refers to H.

The term "deuterium" herein refers to D.

The term "nitrogen" herein refers to N.

The term "cyano" herein refers to -CN.

The term "halogen" herein refers to -F, -Cl, -Br and -I.

The term "aldehyde group" herein refers to -CHO.

The term "hydroxyl" herein refers to -OH.

The term "amino" or "amine" interchangeably refers to -NR₂ group, wherein each R is, for example, H or a substituent. In some embodiments, amino is further substituted to form an ammonium ion, such as NR³⁺. An ammonium moiety is specifically included in the definition of "amino" or "amine". The substituent may be, for example, alkyl, deuterated alkyl, alkoxy, cycloalkyl, heterocycloalkyl, aryl, heteroaryl, amide or carboxylate. An R group may be further substituted by one or more (for example, 1 to 4) groups selected from halogen, cyano, alkenyl, alkynyl, alkyl, alkoxy, cycloalkyl, heterocycloalkyl, aryl, heteroaryl, urea, carbonyl, carboxylate, amine and amide.

The term "alkyl" herein refers to a saturated aliphatic hydrocarbon group having multiple carbon atoms, preferably 1-10 carbon atoms, and more preferably 1-6 carbon atoms, and the term includes straight-chain and branched-chain hydrocarbon groups. When there is restriction on a number of carbon atoms in front of alkyl, such as C₁₋₆ alkyl, it means that the alkyl contains 1-6 carbon atoms. Non-limiting examples of alkyl include methyl, ethyl, n-propyl, isopropyl, n-butyl, isobutyl, sec-butyl, tert-butyl, n-pentyl, neopentyl, n-hexyl, and the like. The alkyl herein may be optionally substituted by one or more of the following substituents: deuterium, fluorine, chlorine, bromine, iodine, cyano, nitro, hydroxyl, carboxyl, amino, alkyl, alkoxy, acyl, acyloxy, oxo, amide group, ester group, amine, sulfonyl, sulfinyl, cycloalkyl, heterocyclyl, cycloalkenyl, heterocycloalkyl, alkenyl, alkenoxy, alkynyl, cycloalkoxy, heterocycloalkyloxy, aryloxy, heteroaryloxy, aryl or heteroaryl.

The term "deuterated alkyl" herein refers to alkyl obtained by substituting the "alkyl" as defined above with deuterium. Non-limiting examples of deuterated alkyl include deuterated methyl, deuterated ethyl and the like.

The term "haloalkyl" herein refers to alkyl obtained by substituting the "alkyl" as defined above with halogen, wherein the halogen includes fluorine, chlorine, bromine, iodine and the like. Non-limiting examples of haloalkyl include monofluoromethyl, difluoromethyl, trifluoromethyl, trifluoroethyl, and the like.

The term "alkoxy" refers to a -ORₐ group, wherein Rₐ is the alkyl as defined above. When there is restriction on a number of carbon atoms in front of alkoxy, such as C₁₋₆ alkoxy, it means that the alkoxy contains 1-6 carbon atoms. Examples of the alkoxy include, but are not limited to, methoxy, ethoxy, isopropoxy, and the like.

Alkenyl is an unsaturated hydrocarbon group containing a carbon-carbon double bond. The term "alkenyl" herein refers to an alkyl group containing a carbon-carbon double bond in a molecule, wherein the alkyl is as defined above, and has 2 to 10 carbon atoms, preferably 2 to 8 carbon atoms, and more preferably 2 to 6 carbon atoms. When there is restriction on a number of carbon atoms in front of alkenyl, such as C₂₋₆ alkenyl, it means that the alkenyl contains 2-6 carbon atoms. The alkenyl herein may be optionally substituted by one or more of the following substituents: deuterium, fluorine, chlorine, bromine, iodine, cyano, nitro, hydroxyl, carboxyl, amino, oxo, alkyl, alkoxy, acyl, amide group, ester group, amine, sulfonyl, sulfinyl, cycloalkyl, cycloalkenyl, heterocycloalkyl, cycloalkoxy, mercapto, alkylmercapto, deuterated alkylmercapto, sulfonyl, sulfinyl, silyl, phosphonyl, deuterated alkyl, heterocyclyl, aryl, heteroaryl, alkynyl, alkenyl and arylalkyl. Non-limiting examples of alkenyl include, but are not limited to, vinyl, propenyl, allyl, isopropenyl, butenyl, isobutenyl, deuterated vinyl, deuterated propenyl, deuterated allyl, and the like.

Alkynyl is an unsaturated hydrocarbon group containing a carbon-carbon triple bond. The term "alkynyl " herein refers to an alkyl group containing a carbon-carbon triple bond in a molecule, wherein the alkyl is as defined above, and has 2 to 10 carbon atoms, preferably 2 to 8 carbon atoms, and more preferably 2 to 6 carbon atoms. When there is restriction on a number of carbon atoms in front of alkynyl, such as C₂₋₆ alkynyl, it means that the alkynyl contains 2-6 carbon atoms. The alkynyl herein may be optionally substituted by one or more of the following substituents: deuterium, fluorine, chlorine, bromine, iodine, cyano, nitro, hydroxyl, carboxyl, amino, alkyl, oxo, alkoxy, acyl, amide group, ester group, amine, sulfonyl, sulfinyl, cycloalkyl, cycloalkenyl, heterocycloalkyl, cycloalkoxy, mercapto, alkylmercapto, deuterated alkylmercapto, sulfonyl, sulfinyl, silyl, phosphonyl, deuterated alkyl, heterocyclyl, aryl, heteroaryl, alkynyl, alkenyl and arylalkyl. Non-limiting examples of alkynyl include, but are not limited to, ethynyl, propargyl, 1-propargyl, 2-propargyl, 1-, 2- or 3-butynyl, deuterated 1-propargyl, deuterated 2-propargyl, deuterated propargyl, and the like.

The term "cycloalkyl" refers to a stable monovalent nonaromatic monocyclic or polycyclic hydrocarbon group consisting of carbon atoms and hydrogen atoms only, which may include a fused ring system or a bridged ring system having 3 to 15 carbon atoms, preferably 3 to 10 carbon atoms, more preferably 3 to 8 and 3 to 6 carbon atoms, and is saturated or unsaturated and may be linked to the rest of the molecule by a single bond via any suitable carbon atom. When there is restriction on a number of carbon atoms in front of cycloalkyl, such as C₃₋₆ cycloalkyl, it means that the cycloalkyl contains 3-6 carbon atoms. The cycloalkyl includes, but is not limited to, cyclopropyl, cyclobutyl, cyclopentyl, cyclohexyl, cycloheptyl, cyclooctyl, 1H-indenyl, 2,3-dihydroindenyl, 1,2,3,4-tetrahydro-naphthyl, 5,6,7,8-tetrahydro-naphthyl, 8,9-hydro-7H-benzocyclo heptene-6-yl, 6,7,8,9-tetrahydro-5H-benzocyclo heptenyl, 5,6,7,8,9,10-hexahydro-benzocyclo octenyl, fluorenyl, bicyclo[2.2.1]heptyl, 7,7-dimethyl-bicyclo[2.2.1]heptyl, bicyclo[2.2.1]heptenyl, bicyclo[2.2.2]octyl, bicyclo[3.1.1]heptyl, bicyclo[3.2.1]octyl, bicyclo[2.2.2]octenyl, bicyclo[3.2.1]octenyl, adamantyl, octahydro-4,7-methylene-1H-indenyl, octahydro-2,5-methylene-cyclopentadienyl, and the like. The cycloalkyl herein may be optionally substituted by one or more of the following substituents: deuterium, fluorine, chlorine, bromine, iodine, cyano, nitro, hydroxyl, oxo, carboxyl, amino, alkyl, alkoxy, acyl, amide group, ester group, amine, sulfonyl, sulfinyl, cycloalkyl, heterocyclyl, cycloalkenyl, heterocycloalkyl, alkenyl, alkynyl, cycloalkoxy, aryl and heteroaryl.

The term "heterocyclyl" refers to a substituted or unsubstituted and saturated or unsaturated aromatic ring or non-aromatic ring containing at least 1 to 5 heteroatoms selected from N, O or S. The aromatic ring or non-aromatic ring may be a 3-10 membered monocyclic ring, a 4-20 membered spirocyclic ring, a fused ring or a bridged ring, wherein N and S optionally substituted in the ring of the heterocyclyl may be oxidized into various oxidation states, preferably a 3-12 membered and 5-10 membered heterocyclic ring. Non-limiting examples of the heterocyclyl include oxiran-2-yl, oxetan-2-yl, oxolan-2-yl, oxan-2-yl, oxepan-2-yl, oxocan-2-yl, aziridin-2-yl, azetidin-2-yl, azolidin-2-yl, piperidin-2-yl, azirin-2-yl, 1,3-dioxolan-2-yl, 1,4-dioxolan-2-yl, 1,3-dithiolan-2-yl, 1,3-dioxan-2-yl, 1,3-dithian-2-yl, azepinyl, morpholinyl, piperazinyl, pyridinyl, furanyl, thiophenyl, pyrrolyl, pyranyl, N-alkylpyrrolyl, pyrimidinyl, pyrazinyl, pyridazinyl, imidazolyl, piperidinyl, thiomorpholinyl, dihydropyranyl, thiadiazolyl, oxazolyl, oxadiazolyl, pyrazolyl, 1,4-dioxinyl, and the like. The heterocyclyl herein may be optionally substituted by one or more of the following substituents: deuterium, fluorine, chlorine, bromine, iodine, cyano, nitro, hydroxyl, carboxyl, amino, alkyl, alkoxy, acyl, amide group, ester group, amine, sulfonyl, sulfinyl, cycloalkyl, heterocyclyl, cycloalkenyl, heterocycloalkyl, alkenyl, alkynyl, cycloalkoxy, aryl and heteroaryl.

The term "aryl" herein refers to a 6-14 membered full-carbon monocyclic or fused polycyclic (for example, a ring sharing a pair of adjacent carbon atoms) group with a conjugated π-electron system, preferably a 6-10 membered ring, for example, phenyl and naphthyl, more preferably phenyl. A ring of the aryl may be fused on a ring of heteroaryl, heterocyclyl or cycloalkyl, wherein a ring linked to the parent structure is the ring of the aryl. The aryl herein may be substituted or unsubstituted, and in the case of being substituted, a substituent is preferably one or more of the following groups: deuterium, fluorine, chlorine, bromine, iodine, cyano, nitro, hydroxyl, carboxyl, amino, alkyl, alkoxy, acyl, amide group, ester group, sulfonyl, sulfinyl, cycloalkyl, heterocycloalkyl, cycloalkenyl, alkenyl, alkynyl, heterocyclyl, cycloalkoxy, aryl and heteroaryl.

The term "heteroaryl" herein refers to an aromatic group consisting of 5 to 10 atoms and containing at least one heteroatom selected from N, O or S. This term may have a single ring (non-limiting examples include furan, thiophene, imidazole, triazole, pyrazole, pyridine, pyrazine, oxazole, thiazole, and the like) or multiple fused rings (non-limiting examples include benzothiophene, benzofuran, indole, isoindole, and the like), wherein the fused ring may or may not be an aromatic group containing heteroatoms, provided that a linking point is an atom of an aromatic heteroaryl group. The heteroaryl herein may be optionally substituted by one or more of the following substituents: deuterium, fluorine, chlorine, bromine, iodine, cyano, nitro, hydroxyl, amino, oxo, alkyl, alkoxy, acyl, acyloxy, amide group, ester group, amine, sulfonyl, sulfinyl, cycloalkyl, heterocycloalkyl, cycloalkenyl, alkenyl, alkynyl, heterocyclyl, cycloalkoxy, aryl and heteroaryl.

The present invention further includes isotopically labeled compounds of the present invention, such as having the same structure as disclosed above, but one or more atoms in this structure are substituted by atoms with the same proton number but different neutron number. Examples of isotopes combined in the compounds of the present invention include isotopes of hydrogen, carbon, nitrogen, oxygen, sulfur, fluorine, chlorine and iodine, such as ²H, ³H, ¹³C, ¹⁴C, ¹⁵N, ¹⁸O, ¹⁷O, ³⁵S, ¹⁸F, ³⁶Cl and ¹³¹I respectively. The compounds of the present invention, their stereoisomers, tautomers or pharmaceutically acceptable salts, and the compounds in the above forms containing the above isotopes and/or other atomic isotopes are all included within the scope of the present invention. Some isotopically labeled compounds of the present invention, such as those compounds labeled by ³H or ¹⁴C, may be used in a drug tissue distribution experiment. Therefore, these ³H or ¹⁴C isotopes are particularly preferred because of easy preparation and detection. Some compounds of the present invention substituted by heavier isotopes, such as ²H and ¹⁸O, have some therapeutic advantages due to better metabolic stability, such as increased in-vivo half-life period, reduced dosage, and other comprehensive performances. Therefore, ²H and ¹⁸O are also preferred in some cases.

The term "optional" or "optionally" means that the subsequently described event or situation may, but does not necessarily, occur, and the description includes the case in which the event or situation occurs and the case in which the event or situation does not occur.

The term "compound of the present invention" (unless otherwise specified) refers to the compounds as shown in formula (A), formula (A-1) and formula (I)-formula (V) and all their pure and mixed stereoisomers, geometric isomers, tautomers, solvates, hydrates, prodrugs, isotopically labeled compounds and any pharmaceutically acceptable salts. The solvate of the compound of the present invention refers to a compound combined with stoichiometric and non-stoichiometric solvents or a salt thereof, such as a hydrate, an ethanol solvate and a methanol solvate. The compound may also exist in one or more crystalline states, such as serving as a cocrystal and a polymorph, or may exist in an amorphous solid state. All such forms are covered by the claims.

The term "pharmaceutically acceptable" means that a substance or composition must be chemically and/or toxicologically compatible with other ingredients constituting the preparation and/or mammals treated with the preparation.

The term "can optionally be substituted by ..." means that the structure is unsubstituted or substituted by one or more substituents according to the present invention. The term "substituted" herein means that any group is monosubstituted or polysubstituted by a specified substituent to an extent that such monosubstitution or polysubstitution (including multi-substitution in the same part) is chemically permitted, and each substituent may be located in any available position on the group and may be linked by any available atom on the substituent. "Any available position" refers to any position on the group that may be chemically obtained by a method known in the art or a method taught herein, and does not produce excessively unstable molecules. When there are two or more substituents on any group, each substituent is defined independently of any other substituent, so that the substituents may be the same or different.

In various parts of the specification, the substituents of the compounds of the present invention are disclosed in the form of groups or ranges, which specifically means that the present invention includes a sub-combination of each member or each individual in the member of such groups and ranges. For example, the term "C₁₋₆ alkyl" specifically means that methyl, ethyl, C₃ alkyl, C₄ alkyl, C₅ alkyl and C₆ alkyl are separately disclosed.

The term "stereoisomer" herein refers to a compound with different chirality having one or more stereocenters. The stereoisomer includes an enantiomer, a diastereomer, a conformational isomer (rotational isomer), a geometric isomer (cis/trans) isomer, an atropisomer, and the like.

The term "tautomer" herein refers to a structure with different energy, and an isomeride may cross a low energy barrier to achieve mutual conversion. For example, a proton tautomer includes a tautomer of interconversion through protolysis, such as an enol-ketone tautomer and an imine-enamine tautomer, or a tautomeric form containing a heteroaryl group linked to a ring atom of ring-NH-moiety and ring=N-moiety, such as pyrazole, imidazole, benzimidazole, triazole and tetrazole. A valence tautomer includes a tautomer of interconversion through recombination of some bonding electrons.

The compounds of the present invention may be used in the form of salts, such as the "pharmaceutically acceptable salts" derived from inorganic or organic acids. These salts include, but are not limited to: acetate, adipate, alginate, citrate, aspartate, sodium salt, benzoate, benzenesulfonate, bisulfate, butyrate, camphorate, camphorsulfonate, digluconate, cyclopentanepropionate, sodium dodecylbenzenesulfonate sulfate, ethanesulfonate, glucosyl heptanoate, glycerophosphate, hemisulfate, heptanoate, caproate, fumarate, hydrochloride, hydrobromide, hydroiodide, 2-hydroxyethylsulfonate, lactate, maleate, methanesulfonate, hydrochloride, 2-naphthalenesulfonate, oxalate, pectate, sulfate, 3-phenylpropionate, picrate, pivalate, propionate, succinate, tartrate, thiocyanate, p-toluenesulfonate, quinate, and the like.

In the present application, "tumor" or "cancer" includes, but is not limited to: leukemia, malignant lymphoma, multiple myeloma, myelodysplastic syndrome and other blood and lymph cancers; and brain tumor, neurotumor, head and neck cancer, esophageal cancer, gastric cancer, colorectal cancer, lung cancer, thyroid cancer, breast cancer, gallbladder cancer, pancreatic cancer, liver cancer, prostate cancer, ovarian cancer, uterine cancer, testis cancer, ovarian cancer, renal cell cancer, bladder cancer, renal pelvis-ureter cancer, malignant melanoma, skin cancer, colorectal cancer, breast cancer, kidney cancer and other solid tumors.

### DETAILED DESCRIPTION

The present invention is further described hereinafter with reference to embodiments, but the present invention is not limited by the embodiments. Throughout the present application, multiple embodiments of the compound and method of the present invention are mentioned herein. The multiple embodiments are intended to provide illustrative examples and shall not be construed as descriptions of alternatives. Meanwhile, it should be noted that the embodiments (including various methods and parameters) discussed herein are only for illustrating the present invention, and do not limit the scope of protection of the present invention in any way. In order to describe the present invention, specific embodiments are listed hereinafter. However, it should be understood that the present invention is not limited to these embodiments, and the following embodiments only provide the method for practicing the present invention and do not limit the scope of the present invention in any way.

A process of a preparation method for a compound as shown in formula (IV), a stereoisomer thereof, a tautomer thereof or a pharmaceutically acceptable salt thereof according to the present invention is as follows.

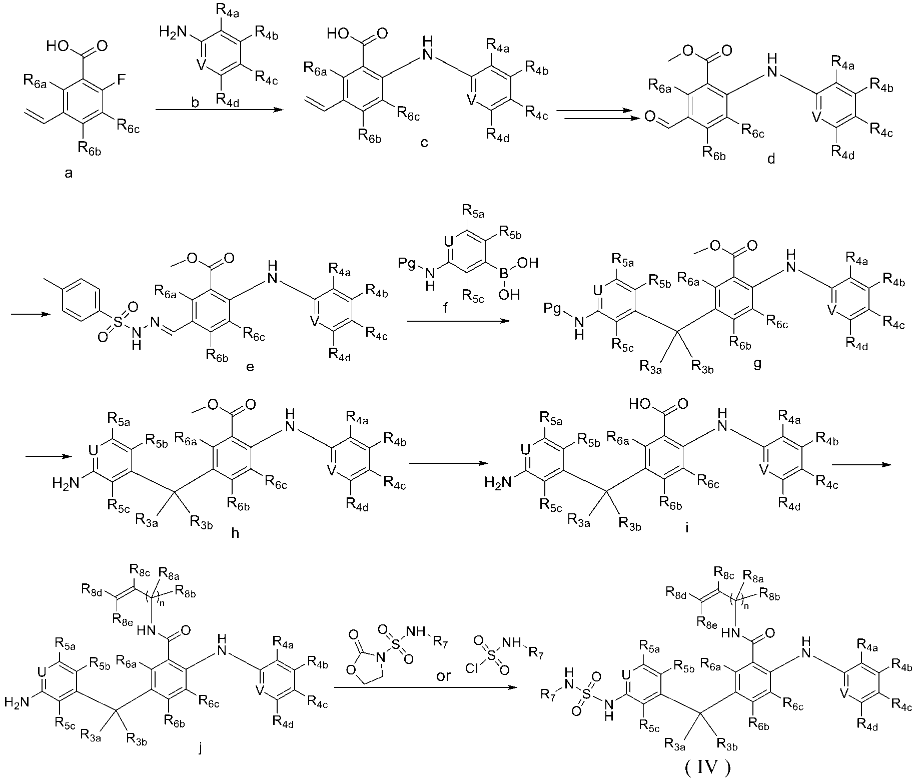

Under alkaline conditions, fluorobenzene derivative a reacts with aromatic amine derivative b to generate compound c, which then undergoes methylation and oxidation to yield compound d. Aldehyde derivative d reacts with aryl sulfonylhydrazine to give compound e. Under alkaline conditions, hydrazine derivative e reacts with arylboronic acid derivative f to generate compound g. Under acidic conditions, compound g is deprotected to generate compound h. Ester derivative h is hydrolyzed in the presence of a hydroxide to produce compound i. In the presence of a condensing agent, benzoic acid derivative i reacts with the corresponding amine or amine hydrochloride to generate compound j. Under alkaline conditions, compound j reacts with the corresponding sulfonyl compound to obtain the compound of general formula I. The definitions of each substituent are as previously described.

The compounds provided by the present invention may be prepared by standard synthesis methods well known in the art, and the specification provides general methods for preparing the compounds of the present invention. The starting materials or reagents are usually commercially available, such as those purchased through Alfa Aesar^{®}, Sigma-Aldrich^{®}, TCI, and other companies, or prepared by methods well known to those skilled in the art.

The compounds of the present invention and the corresponding preparation methods are further explained and enumerated hereinafter through the embodiments and the preparations. It should be understood that, although typical or preferred reaction conditions (such as reaction temperature and time, molar ratios of reactants, and reaction solvents) are given in specific embodiments, other reaction conditions may also be used by those skilled in the art. The optimum reaction conditions may vary depending on a specific reaction substrate or solvent used, but the conditions may be determined by those skilled in the art through routine optimization.

Structures of the compounds in the following embodiments were characterized by nuclear magnetic resonance (NMR) and/or mass spectrometry (MS). Bruker Ascend 400 MHz NMR spectrometer was used, the compounds were dissolved in appropriate deuterated reagents, and analyzed by 1H-NMR with TMS as an internal standard at ambient temperature. A NMR chemical shift (δ) was in a unit of ppm, and the following abbreviations were used: s, single peak; d, double peak; t, triple peak; q, quadruple peak; m, multiple peaks; and brs, broad single peak.

The starting materials in reaction, the intermediates and the compounds of the embodiments may be separated and purified by precipitation, filtration, crystallization, evaporation, distillation, chromatography (such as silica gel column chromatography and preparative chromatography), and other technologies.

### Preparation of intermediate 1

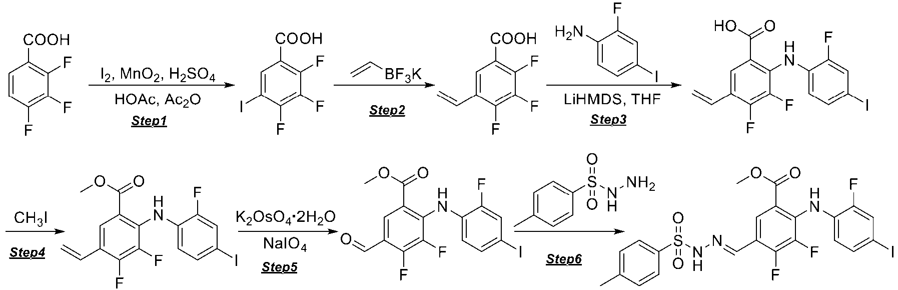

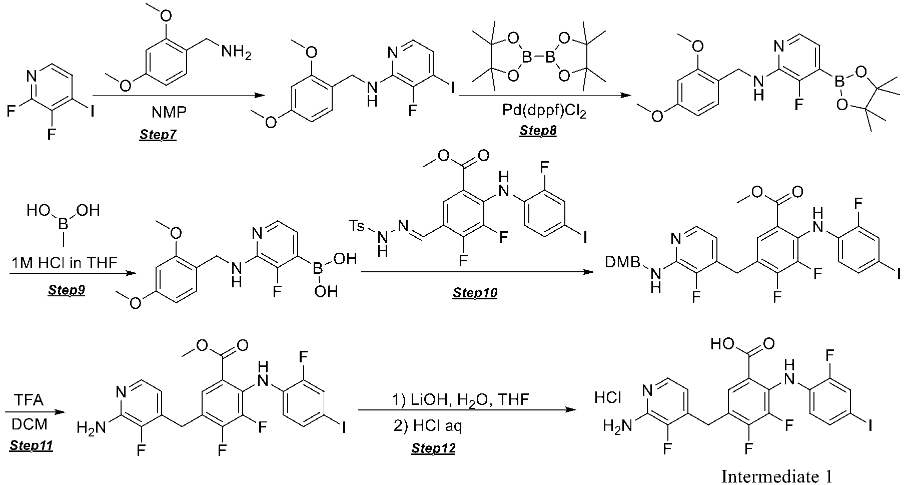

### Step 1: preparation of 2,3,4-trifluoro-5-iodobenzoic acid

Acetic acid (928 mL) and acetic anhydride (538.0 g) were added into a 3 L three-neck flask, and cooled to 0°C in an ice bath. Concentrated sulfuric acid (2116.0 g) was slowly added to the system, and an internal temperature of the system was kept below 40°C. After finishing the dropwise addition, the system was cooled to room temperature again. Subsequently, 2,3,4-trifluorobenzoic acid (115.0 g), iodine (66.9 g) and manganese dioxide (68.7 g) were added. After finishing the addition, the system was heated to 50°C to react for 3 hours. After stopping heating, and then cooling to room temperature, iodine (66.9 g) and manganese dioxide (68.7 g) were added, and subsequently, the system was heated to 50°C to react for 3 hours. After stopping heating, and then cooling the system to room temperature, iodine (33.4 g) and manganese dioxide (34.4 g) were added, and subsequently, the system was heated to 50°C again to react for 21 hours. After stopping reacting, and then cooling to room temperature, the system was slowly poured into ice water for quenching the reaction, and extracted with dichloromethane. Organic phases were combined, washed with sodium sulfite aqueous solution and saturated brine respectively, and dried with anhydrous sodium sulfate. The organic phase was concentrated, then n-heptane was added, the mixture was stirred in an ice bath to precipitate a large number of solids, and subjected to suction filtration. The filter cake was washed with n-heptane to obtain 178.1 g of white solids. ESI-MS: [M - H]⁻ = 300.9.

### Step 2: preparation of 2,3,4-trifluoro-5-vinylbenzoic acid

2,3,4-trifluoro-5-iodobenzoic acid (89.0 g), potassium vinyltrifluoroborate (47.4 g), potassium phosphate (93.8 g), methanol (1780 mL), water (890 mL) and [1,1'-bis(diphenylphosphino)ferrocene]dichloropalladium (21.6 g) were sequentially added into a 3 L three-neck flask. The system was subjected to nitrogen purging, and heated to 60°C to react for 3 hours. After cooling to room temperature, the system was filtered with diatomaceous earth. The filtrate was collected, concentrated, and 1 M HCl was added to adjust a pH value of the system to 4-5. Subsequently, the system was extracted with methyl tert-butyl ether. Organic phases were combined, washed with saturated brine, dried and concentrated. N-heptane and dichloromethane were added to the concentrate, the mixture was stirred at room temperature for 2 hours, and filtered. The filter cake was washed with n-heptane to obtain 48.2 g of white solids. ESI-MS: [M - H]⁻ = 201.0.

### Step 3: preparation of 3,4-difluoro-2-((2-fluoro-4-iodophenyl)amino)-5-vinylbenzoic acid

2,3,4-trifluoro-5-vinylbenzoic acid (65.0 g), 2-fluoro-4-iodoaniline (76.3 g) and tetrahydrofuran (1300 mL) were added into a 3 L three-neck flask. Subsequently, the system was subjected to nitrogen purging, and cooled to -15°C to -20°C. Lithium bis(trimethylsilyl)amide (1 M, 965 mL) was slowly added to the system, and in this period, it was ensured that the temperature of the system was not higher than -15°C. After finishing the dropwise addition, the temperature of the system was slowly increased to room temperature, then 1 M hydrochloric acid was added to adjust a pH value of the system to 1-2, and the system was extracted with methyl tert-butyl ether. Organic phases were combined, washed with saturated brine, dried and concentrated. N-heptane was slowly added to the concentrate to precipitate solids, and filtered to obtain 99.0 g of off-white solids. ESI-MS: [M - H]⁻ = 418.0.

### Step 4: preparation of methyl 3,4-difluoro-2-((2-fluoro-4-iodophenyl)amino)-5-vinylbenzoate

3,4-difluoro-2-((2-fluoro-4-iodophenyl)amino)-5-vinylbenzoic acid (20.0 g) and N,N-dimethylformamide (200 mL) were added into a 1 L three-neck flask, and the system was stirred at room temperature until a clear solution was obtained. Potassium carbonate (7.9 g) and methyl iodide (8.1 g) were added to the system. Subsequently, the system reacted at room temperature for 12 hours. Suspended substances in the system were removed by filtration, and a filtrate was collected. The filtrate was slowly dropwise added into water to precipitate solids. After finishing the dropwise addition, the system was continuously stirred for 1 hour, and subjected to suction filtration. The filter cake was washed with 250 mL of ethanol to obtain 15.1 g of light yellow solids.

### Step 5: preparation of methyl 3,4-difluoro-2-((2-fluoro-4-iodophenyl)amino)-5-formylbenzoate

Methyl 3,4-difluoro-2-((2-fluoro-4-iodophenyl)amino)-5-vinylbenzoate (11.6 g) and tetrahydrofuran (200 mL) were added into a 1 L three-neck flask, and stirred in an ice bath until a clear solution was obtained. Potassium osmate dihydrate (494 mg) was added to the system, and the mixture was continuously stirred in an ice bath to react. After 15 minutes, water (200 mL), sodium periodate (17.2 g) and 2,6-dimethylpyridine (2.9 g) were added to the system, and the system was heated to room temperature and stirred to react for 4 hours. The reaction system was poured into a saturated sodium sulfite aqueous solution, and extracted with ethyl acetate. Organic phases were combined, washed with saturated brine, and concentrated to obtain a solid crude product. The crude product was washed with anhydrous ethanol, and filtered to obtain 9.0 g of light brown solids. ¹H NMR (400 MHz, DMSO-*d*₆): δ 10.00 (s, 1H), 9.52 (s, 1H), 8.21 (dd, *J*₁ = 7.2 Hz, *J*₂ = 1.2 Hz, 1H), 7.71 (dd, *J*₁ = 10.4 Hz, *J*₂ = 1.6 Hz, 1H), 7.54-7.51 (m, 1H), 7.13-7.07 (m, 1H), 3.86 (s, 3H). ESI-MS: [M + H]⁺ = 436.0.

### Step 6: preparation of methyl (E)-3,4-difluoro-2-((2-fluoro-4-iodophenyl)amino)-5-((2-p-toluenesulfonylhydrazinyl)methyl)benz oate

Methyl 3,4-difluoro-2-((2-fluoro-4-iodophenyl)amino)-5-formylbenzoate (50.7 g), p-toluenesulfonyl hydrazide (21.7 g) and ethanol (1500 mL) were added into a 2 L three-neck flask, and the system was heated to 50°C to react for 5 hours. The system was cooled to 0°C in an ice bath to precipitate a large number of solids, and subjected to suction filtration. The filter cake was washed with ethanol to obtain 53.1 g of light yellow solids. ¹H NMR (400 MHz, DMSO-*d*₆): δ 11.76 (s, 1H), 8.97 (s, 1H), 8.00-7.99 (m, 2H), 7.75 (d, *J =* 8.0 Hz, 2H), 7.64 (dd, *J*₁ = 10.4 Hz, *J*₂ = 1.6 Hz, 1H), 7.43 (d, *J =* 8.4 Hz, 3H), 6.92-6.89 (m, 1H), 3.85 (s, 3H), 2.37 (s, 3H). ESI-MS: [M + H]⁺ = 604.0.

### Step 7: preparation of N-(2,4-dimethoxybenzyl)-3-fluoro-4-iodopyridin-2-amine

2,3-difluoro-4-iodopyridine (45.0 g), (2,4-dimethoxyphenyl)methylamine (78.0 g), N-methylpyrrolidone (675 mL) and triethylamine (56.7 g) were added into a 1 L single-neck flask, and the system was subjected to nitrogen purging. The system was heated to 100°C to react for 2 hours. After cooling to room temperature, the system was poured into water, and extracted with methyl tert-butyl ether. Organic phases were combined, washed with saturated brine, dried and concentrated to obtain a crude product. The crude product was washed with methyl tert-butyl ether/petroleum ether (1/4) to obtain 56.8 g of off-white solids. ¹H NMR (400 MHz, DMSO-*d*₆): δ 7.45 (d, *J* = 5.6 Hz, 1H), 7.11-7.08 (m, 1H), 7.03 (d, *J* = 8.4 Hz, 1H), 6.89-6.86 (m, 1H), 6.54 (d, *J* = 2.4 Hz, 1H), 6.43 (dd, *J*₁ = 8.4 Hz, *J*₂ = 2.4 Hz, 1H), 4.44 (d, *J =* 6.0 Hz, 2H), 3.80 (s, 3H), 3.72 (s, 3H).

### Step 8: preparation of N-(2,4-dimethoxybenzyl)-3-fluoro-4-(4,4,5,5-tetramethyl-1,3,2-dioxaborolan-2-yl)pyridin-2-amine

N-(2,4-dimethoxybenzyl)-3-fluoro-4-iodopyridin-2-amine (56.5 g), bis(pinacolato)diboron (147.8 g), potassium pivalate (44.9 g), toluene (1400 mL), dimethyl sulfoxide (20 mL) and [1,1'-bis(diphenylphosphino)ferrocene]dichloropalladium (10.6 g) were added into a 2 L three-neck flask, and the system was subjected to nitrogen purging. Subsequently, the system was heated to 55°C to react for 15 hours. After cooling to room temperature, the system was poured into water, and extracted with ethyl acetate. Organic phases were combined, dried with anhydrous sodium sulfate and concentrated. Subsequently, the concentrate was diluted with n-hexane, dried and filtered with diatomite, and the filtrate was concentrated to obtain a product. The product in this step was directly used in the next step of reaction without further purification. ¹H NMR (400 MHz, DMSO-*d*₆): δ 7.75 (dd, *J*₁ = 4.8 Hz, *J*₂ = 0.8 Hz, 1H), 7.01 (d, *J* = 8.4 Hz, 1H), 6.87-6.84 (m, 1H), 6.59-6.56 (m, 1H), 6.54 (d, *J* = 2.4 Hz, 1H), 6.42 (dd, *J*₁ = 8.4 Hz, *J*₂ = 2.4 Hz, 1H), 4.45 (d, *J =* 6.0 Hz, 2H), 3.80 (s, 3H), 3.72 (s, 3H), 1.30 (s, 12H).

### Step 9: preparation of (2-((2,4-dimethoxybenzyl)amino)-3-fluoropyridin-4-yl)boronic acid

N-(2,4-dimethoxybenzyl)-3-fluoro-4-(4,4,5,5-tetramethyl-1,3,2-dioxaborolan-2-yl)pyridin-2-a mine and THF (500 mL) were added into a 2 L single-neck flask, and stirred until a clear solution was obtained. 1 M hydrochloric acid aqueous solution was slowly added to the system, and stirred at room temperature to react for 1 hour. The system was extracted with petroleum ether, and an aqueous phase was collected. Methylboric acid (17.4 g) was added to the aqueous phase, and the mixture was stirred at room temperature to react for 1 hour. Subsequently, the system was extracted with an ethyl acetate/petroleum ether (3/1) mixed solvent, and an aqueous phase was collected. Ethyl acetate was added to the aqueous phase, and a sodium bicarbonate aqueous solution was added to adjust a pH value of the system to 8. Organic phases were collected, washed with water, dried and concentrated to obtain 37.0 g of off-white solids. ESI-MS: [M + H]⁺ = 307.1.

### Step 10: preparation of methyl 5-((2-((2,4-dimethoxybenzyl)amino)-3-fluoropyridin-4-yl)methyl)-3,4-difluoro-2-((2-fluoro-4-iodo phenyl)amino)benzoate

(2-((2,4-dimethoxybenzyl)amino)-3-fluoropyridin-4-yl)boronic acid (15.0 g), methyl (*E*)-3,4-difluoro-2-((2-fluoro-4-iodophenyl)amino)-5-((2-p-toluenesulfonylhydrazinyl)methyl)benz oate (35.4 g), potassium carbonate (8.1 g) and toluene (600 mL) were added into a 1 L single-neck flask, and the system was subjected to nitrogen purging. The system was heated to 100°C to react for 5 hours. After cooling to room temperature, saturated ammonium chloride aqueous solution was added to the system, and the mixture was extracted with ethyl acetate. Organic phases were combined, dried with anhydrous sodium sulfate, filtered and subjected to rotary evaporation to remove the solvent. Subsequently, the crude product was purified by silica gel column chromatography to obtain 17.9 g of light yellow liquid. ¹H NMR (400 MHz, DMSO-*d*₆): δ 8.65 (s, 1H), 7.73 (d, *J =* 7.2 Hz, 1H), 7.68 (d, *J =* 5.2 Hz, 1H), 7.62 (dd, *J*₁ = 10.8 Hz, *J*₂ = 3.0 Hz, 1H), 7.39 (dd, *J*₁ = 8.4 Hz, *J*₂ = 0.8 Hz, 1H), 7.03 (d, *J =* 8.4 Hz, 1H), 6.92-6.88 (m, 1H), 6.80-6.75 (m, 1H), 6.54 (d, *J =* 2.4 Hz, 1H), 6.42 (dd, *J*₁ = 8.4 Hz, *J*₂ = 2.4 Hz, 1H), 6.38 (t, *J =* 4.8 Hz, 1H), 4.44 (d, *J* = 6.0 Hz, 2H), 4.01 (s, 2H), 3.80 (d, *J=* 1.2 Hz, 6H), 3.72 (s, 3H). ESI-MS: [M + H]⁺ = 682.1.

### Step 11: preparation of methyl 5-((2-amino-3-fluoropyridin-4-yl)methyl)-3,4-difluoro-2-((2-fluoro-4-iodophenyl)amino)benzoate

Methyl 5-((2-((2,4-dimethoxybenzyl)amino)-3-fluoropyridin-4-yl)methyl)-3,4-difluoro-2-((2-fluoro-4-iodo phenyl)amino)benzoate (10.0 g) and dichloromethane (150 mL) were added into a 500 mL single-neck flask, and stirred at room temperature until a clear solution was obtained. Subsequently, the system was placed in an ice bath, and trifluoroacetic acid (150 mL) was slowly added. After finishing the addition, the system was heated to room temperature and stirred to react for 1.5 hours. Saturated sodium carbonate aqueous solution was slowly added to the system to adjust a pH value to 8, and the mixture was extracted with dichloromethane. Organic phases were combined, dried with anhydrous sodium sulfate, filtered and subjected to rotary evaporation to remove the solvent. Subsequently, the crude product was purified by silica gel column chromatography to obtain 5.8 g of light yellow solids. ¹H NMR (400 MHz, DMSO-*d*₆): δ 8.64 (s, 1H), 7.71 (d, *J =* 7.2 Hz, 1H), 7.67 (d, *J* = 5.2 Hz, 1H), 7.62 (dd, *J*₁ = 10.8 Hz, *J*₂ = 1.6 Hz, 1H), 7.39 (dd, *J*₁ = 8.4 Hz, *J*₂ = 1.2 Hz, 1H), 6.80-6.74 (m, 1H), 6.40 (t, *J =* 4.8 Hz, 1H), 6.22 (s, 2H), 3.99 (s, 2H), 3.79 (s, 3H). ESI-MS: [M + H]⁺ = 532.0.

### Step 12: preparation of 5-((2-amino-3-fluoropyridin-4-yl)methyl)-3,4-difluoro-2-((2-fluoro-4-iodophenyl)amino)benzoic acid hydrochloride

Methyl 5-((2-amino-3-fluoropyridin-4-yl)methyl)-3,4-difluoro-2-((2-fluoro-4-iodophenyl)amino)benzoate (6.9 g), tetrahydrofuran (100 mL) and water (50 mL) were sequentially added into a 1 L single-neck flask, and the system was placed in an ice bath and stirred. Lithium hydroxide monohydrate (1.6 g) was slowly added to the system. After finishing the addition, the system was heated to room temperature and stirred to react for 2.5 hours. Subsequently, hydrochloric acid aqueous solution (6 M, 20 mL) was added to the system to adjust a pH value to 1-2, stirred for 5 minutes, and concentrated under reduced pressure to remove the tetrahydrofuran. Subsequently, the concentrate was diluted with water and stirred for 10 minutes, and filtered. A filter cake was collected, and washed once with water and once with methyl tert-butyl ether respectively to obtain 6.2 g of magenta solids. ESI-MS: [M + H]⁺ = 518.0.

### Preparation of intermediate 2

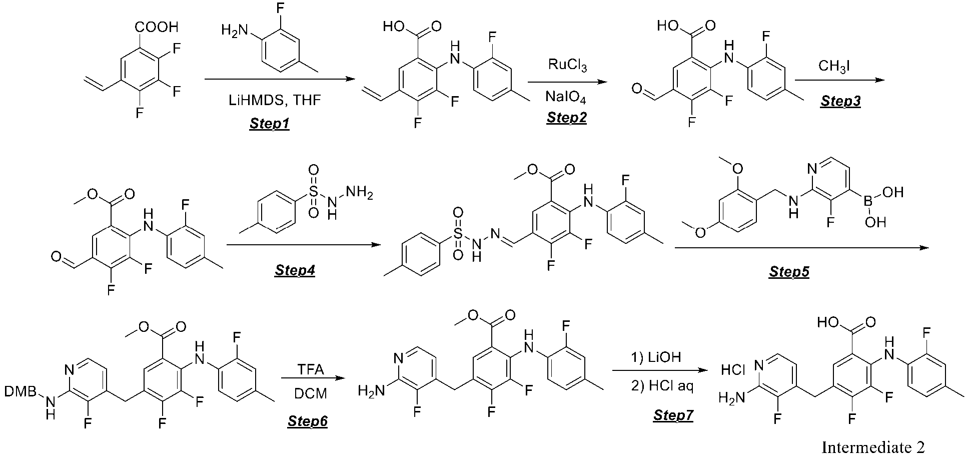

### Step 1: preparation of 3,4-difluoro-2-((2-fluoro-4-methylphenyl)amino)-5-vinylbenzoic acid

2,3,4-trifluoro-5-vinylbenzoic acid (24.2 g), 2-fluoro-4-methylaniline (15.0 g) and tetrahydrofuran (485 mL) were added into a 2 L three-neck flask. Subsequently, the system was subjected to nitrogen purging, and cooled to -70°C to -80°C. Lithium bis(trimethylsilyl)amide (1 M, 360 mL) was slowly dropwise added to the system, and in this period, it was ensured that a temperature of the system was not higher than -70°C. After finishing the dropwise addition, the temperature of the system was slowly increased to room temperature, 600 mL of hydrochloric acid (1 M) was added to adjust a pH value to 1-2, and extracted with methyl tert-butyl ether. Organic phases were combined, washed with saturated brine, dried and concentrated. N-heptane was slowly added to the concentrate, the mixture was stirred at room temperature for 30 minutes, and filtered to obtain 30.4 g of light yellow solids. ESI-MS: [M - H]⁻ = 306.1.

### Step 2: preparation of 3,4-difluoro-2-((2-fluoro-4-methylphenyl)amino)-5-formylbenzoic acid

Water (100 mL), tetrahydrofuran (300 mL) and acetonitrile (300 mL) were added into a 2 L three-neck flask, and stirred at room temperature. Subsequently, concentrated sulfuric acid (2.0 g) was slowly dropwise added to the reaction flask. After the temperature of the system was returned to room temperature, sodium periodate (34.9 g) and anhydrous ruthenium trichloride (0.5 g) were slowly added to the system, and the mixture was continuously stirred at room temperature to react. After 5 minutes, 3,4-difluoro-2-((2-fluoro-4-methylphenyl)amino)-5-vinylbenzoic acid (10.0 g) was added to the system, and the mixture was stirred at room temperature to react for 2 hours. Insoluble substances were removed by filtration. A filtrate was collected, and extracted with ethyl acetate. Organic phases were combined, washed with a saturated sodium sulfite aqueous solution, and then washed with saturated brine. The organic phase was collected and dried, filteried, and concentrated to obtain 7.0 g of yellow solids. ESI-MS: [M - H]⁻ = 308.1.

### Step 3: preparation of methyl 3,4-difluoro-2-((2-fluoro-4-methylphenyl)amino)-5-formylbenzoate

3,4-difluoro-2-((2-fluoro-4-methylphenyl)amino)-5-formylbenzoic acid (3.1 g) and N,N-dimethylformamide (31 mL) were added into a 250 mL three-neck flask, and the system was stirred at room temperature until a clear solution was obtained. Potassium carbonate (1.5 g) and methyl iodide (1.6 g) were added to the system, and then heated to 50°C to react for 4 hours. Suspended substances were removed by filtration. A filtrate was collected. The filtrate was slowly dropwise added into water to precipitate solids. After finishing the dropwise addition, the system was continuously stirred for 1 hour, and subjected to suction filtration. The filter cake was washed with water and dried to obtain 1.3 g of yellow solids. ESI-MS: [M + H]⁺ = 324.1.

### Step 4: preparation of methyl (E)-3,4-difluoro-2-((2-fluoro-4-methylphenyl)amino)-5-((2-p-toluenesulfonylhydrazinyl)methyl)be nzoate

Methyl 3,4-difluoro-2-((2-fluoro-4-methylphenyl)amino)-5-formylbenzoate (1.8 g), p-toluenesulfonyl hydrazide (1.1 g) and ethanol (54 mL) were added into a 2 L three-neck flask, and the system was heated to 50°C to react for 2 hours. The system was cooled to 0°C in an ice bath to precipitate a large number of solids, and subjected to suction filtration. The filter cake was washed with ethanol to obtain 1.7 g of light yellow solids. ¹H NMR (400 MHz, DMSO-*d*₆): δ 11.69 (s, 1H), 9.04 (s, 1H), 8.01-7.98 (m, 2H), 7.75 (d, *J =* 8.0 Hz, 2H), 7.43 (d, *J =* 8.0 Hz, 2H), 7.09-7.03 (m, 2H), 6.93 (d, *J* = 8.0 Hz, 1H), 3.87 (s, 3H), 2.38 (s, 3H), 2.28 (s, 3H). ESI-MS: [M + H]⁺ = 492.1.

### Step 5: preparation of methyl 5-((2-((2,4-dimethoxybenzyl)amino)-3-fluoropyridin-4-yl)methyl)-3,4-difluoro-2-((2-fluoro-4-meth ylphenyl)amino)benzoate

(2-((2,4-dimethoxybenzyl)amino)-3-fluoropyridin-4-yl)boronic acid (2.7 g), methyl (*E*)-3,4-difluoro-2-((2-fluoro-4-methylphenyl)amino)-5-((2-p-toluenesulfonylhydrazinyl)methyl)be nzoate (3.6 g), potassium carbonate (1.2 g) and toluene (144 mL) were added into a 1 L single-neck flask, and the system was subjected to nitrogen purging. The system was heated to 100°C to react for 4 hours. After cooling to room temperature, saturated ammonium chloride aqueous solution was added to the system, and the mixture was extracted with ethyl acetate. Organic phases were combined, dried with anhydrous sodium sulfate, filtered and subjected to rotary evaporation to remove the solvent. Subsequently, the crude product was purified by silica gel column chromatography to obtain 3.2 g of light yellow liquid. ESI-MS: [M + H]⁺ = 570.2.

### Step 6: preparation of methyl 5-((2-amino-3-fluoropyridin-4-yl)methyl)-3,4-difluoro-2-((2-fluoro-4-methylphenyl)amino)benzoat e

Methyl 5-((2-((2,4-dimethoxybenzyl)amino)-3-fluoropyridin-4-yl)methyl)-3,4-difluoro-2-((2-fluoro-4-meth ylphenyl)amino)benzoate (3.2 g) and dichloromethane (60 mL) were added into a 250 mL single-neck flask, and stirred at room temperature until a clear solution was obtained. Subsequently, the system was placed in an ice bath, and trifluoroacetic acid (60 mL) was slowly added. After finishing the addition, the system was heated to room temperature and stirred to react for 1.5 hours. Saturated sodium carbonate aqueous solution was slowly added to adjust a pH value to be 8, and the mixture was extracted with dichloromethane. Organic phases were combined, dried with anhydrous sodium sulfate, filtered and subjected to rotary evaporation to remove the solvent. Subsequently, the crude product was purified by silica gel column chromatography to obtain 2.1 g of light yellow solids. ESI-MS: [M + H]⁺ = 420.1.

### Step 7: preparation of 5-((2-amino-3-fluoropyridin-4-yl)methyl)-3,4-difluoro-2-((2-fluoro-4-methylphenyl)amino)benzoic acid hydrochloride

Methyl 5-((2-amino-3-fluoropyridin-4-yl)methyl)-3,4-difluoro-2-((2-fluoro-4-methylphenyl)amino)benzoat e (2.5 g), tetrahydrofuran (60 mL) and water (30 mL) were sequentially added into a 250 mL single-neck flask, and the system was placed in an ice bath and stirred. Lithium hydroxide monohydrate (0.8 g) was slowly added to the system. After finishing the addition, the system was heated to room temperature and stirred to react for 4 hours. Subsequently, hydrochloric acid aqueous solution (6 M, 10 mL) was added to the system to adjust a pH value to 1-2, the mixture was stirred for 5 minutes, and concentrated under reduced pressure to remove the tetrahydrofuran in the system. Subsequently, the concentrate was diluted with water and stirred for 10 minutes, and filtered. A filter cake was collected, and washed once with water and once with methyl tert-butyl ether respectively to obtain 1.4 g of magenta solids. ESI-MS: [M + H]⁺ = 406.1.

### Preparation of intermediate 3

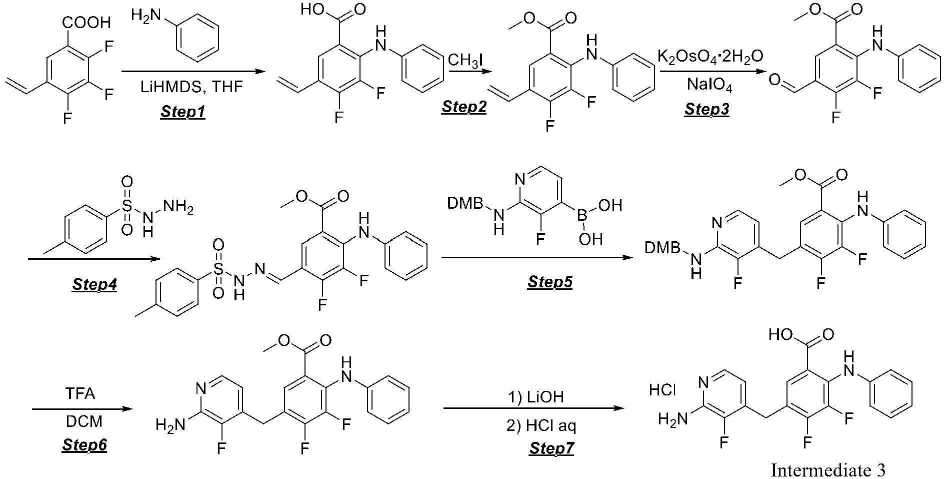

### Step 1: preparation of 3,4-difluoro-2-(phenylamino)-5-vinylbenzoic acid

2,3,4-trifluoro-5-vinylbenzoic acid (20.0 g), aniline (9.2 g) and tetrahydrofuran (400 mL) were added into a 2 L three-neck flask. Subsequently, the system was subjected to nitrogen purging, and cooled to -15°C to -20°C. Lithium bis(trimethylsilyl)amide (1 M, 297 mL) was slowly dropwise added to the system, and in this period, it was ensured that a temperature of the system was not higher than -15°C. After finishing the dropwise addition, the temperature of the system was slowly increased to room temperature, then hydrochloric acid (6 M) was added to adjust a pH value of the system to 1-2, and extracted with ethyl acetate. Organic phases were combined, washed with 1 L of saturated brine, dried and concentrated to obtain 24.5 g of yellow solids. ¹H NMR (400 MHz, DMSO-*d*₆): δ 13.69 (s, 1H), 9.13 (s, 1H), 7.97 (d, *J* = 7.2 Hz, 1H), 7.27 (t, *J* = 8.0 Hz, 2H), 7.00-6.96 (m, 3H), 6.78 (dd, *J*₁ = 17.6 Hz, *J*₂ = 11.2 Hz, 1H), 5.89 (d, *J =* 18.0 Hz, 1H), 5.44 (d, *J* = 11.6 Hz, 1H). ESI-MS: [M + H]⁺ = 276.1.

### Step 2: preparation of methyl 3,4-difluoro-2-(phenylamino)-5-vinylbenzoate

3,4-difluoro-2-(phenylamino)-5-vinylbenzoic acid (30.0 g) and N,N-dimethylformamide (300 mL) were added into a 1 L three-neck flask, and the system was stirred at room temperature until a clear solution was obtained. Potassium carbonate (22.6 g) and methyl iodide (20.1 g) were added to the system. Subsequently, the system reacted at room temperature for 16 hours. Suspended substances in the system were removed by filtration, and a filtrate was collected. The filtrate was slowly dropwise added into water to precipitate solids. After finishing the dropwise addition, the system was continuously stirred for 1 hour, and subjected to suction filtration. The filter cake was washed with ethanol to obtain 21.0 g of light yellow solids. ¹H NMR (400 MHz, DMSO-*d*₆): δ 8.77 (s, 1H), 7.91 (d, *J=* 6.8 Hz, 1H), 7.25 (t, *J =* 8.0 Hz, 2H), 6.97-6.96 (m, 3H), 6.78 (dd, *J*₁ = 17.6 Hz, *J*₂ = 11.2 Hz, 1H), 5.91 (d, *J =* 17.6 Hz, 1H), 5.46 (d, *J =* 11.6 Hz, 1H), 3.78 (s, 3H). ESI-MS: [M + H]⁺ = 290.1.

### Step 3: preparation of methyl 3,4-difluoro-2-(phenylamino)-5-formylbenzoate

Methyl 3,4-difluoro-2-(phenylamino)-5-vinylbenzoate (20.8 g) and tetrahydrofuran (312 mL) were added into a 1 L three-neck flask, and stirred in an ice bath until a clear solution was obtained. Potassium osmate dihydrate (1.3 g) was added to the system, and the mixture was continuously stirred in an ice bath to react. After 15 minutes, water (312 mL), sodium periodate (46.2 g) and 2,6-dimethylpyridine (7.7 g) were added to the system, and the mixture was heated to room temperature and stirred to react for 4 hours. The reaction system was poured into a saturated sodium sulfite aqueous solution, and extracted with ethyl acetate. Organic phases were combined, washed with saturated brine twice, and concentrated to obtain 20.0 g of yellow solids. ESI-MS: [M + H]⁺ = 292.1.

### Step 4: preparation of methyl (E)-3,4-difluoro-2-(phenylamino)-5-((2-p-toluenesulfonylhydrazinyl)methyl)benzoate

Methyl 3,4-difluoro-2-(phenylamino)-5-formylbenzoate (20.0 g), p-toluenesulfonyl hydrazide (12.8 g) and ethanol (600 mL) were added into a 2 L three-neck flask, and the system was heated to 50°C to react for 15 hours. The system was cooled to 0°C in an ice bath to precipitate a large number of solids, and subjected to suction filtration. The filter cake was washed with 50 mL of ethanol to obtain 26.0 g of off-white solids. ¹H NMR (400 MHz, DMSO-*d*₆): δ 11.71 (s, 1H), 8.98 (s, 1H), 8.00-7.97 (m, 2H), 7.75 (d, *J =* 8.0 Hz, 2H), 7.43 (d, *J =* 8.0 Hz, 2H), 7.26 (t, *J =* 8.0 Hz, 2H), 6.99-6.98 (m, 3H), 3.80 (s, 3H), 2.38 (s, 3H). ESI-MS: [M + H]⁺ = 460.1.

### Step 5: preparation of methyl 5-((2-((2,4-dimethoxybenzyl)amino)-3-fluoropyridin-4-yl)methyl)-3,4-difluoro-2-(phenylamino)be nzoate

(2-((2,4-dimethoxybenzyl)amino)-3-fluoropyridin-4-yl)boronic acid (6.9 g), methyl (*E*)-3,4-difluoro-2-(phenylamino)-5-((2-p-toluenesulfonylhydrazinyl)methyl)benzoate (8.0 g), potassium carbonate (3.6 g) and toluene (320 mL) were added into a 1 L single-neck flask, and the system was subjected to nitrogen purging. The system was heated to 100°C to react for 5 hours. After cooling to room temperature, saturated ammonium chloride aqueous solution was added to the system, and the mixture was extracted with ethyl acetate. Organic phases were combined, dried with anhydrous sodium sulfate, filtered and subjected to rotary evaporation to remove the solvent, so as to obtain 4.7 g of light yellow liquid. ESI-MS: [M + H]⁺ = 538.2.

### Step 6: preparation of methyl 5-((2-amino-3-fluoropyridin-4-yl)methyl)-3,4-difluoro-2-(phenylamino)benzoate

Methyl 5-((2-((2,4-dimethoxybenzyl)amino)-3-fluoropyridin-4-yl)methyl)-3,4-difluoro-2-(phenylamino)be nzoate (4.7 g) and dichloromethane (220 mL) were added into a 500 mL single-neck flask, and the mixture was stirred at room temperature until a clear solution was obtained. Subsequently, the system was placed in an ice bath, and trifluoroacetic acid (110 mL) was slowly added. After finishing the addition, the system was heated to room temperature and stirred to react for 1.5 hours. Saturated sodium carbonate aqueous solution was slowly added to adjust a pH value of the system to 8, and the mixture was extracted with dichloromethane. Organic phases were combined, dried with anhydrous sodium sulfate, filtered and subjected to rotary evaporation to remove the solvent. Subsequently, the crude product was purified by silica gel column chromatography to obtain 1.8 g of yellow solids.

### Step 7: preparation of 5-((2-amino-3-fluoropyridin-4-yl)methyl)-3,4-difluoro-2-(phenylamino)benzoic acid hydrochloride

Methyl 5-((2-amino-3-fluoropyridin-4-yl)methyl)-3,4-difluoro-2-(phenylamino)benzoate (1.8 g), tetrahydrofuran (40 mL) and water (40 mL) were sequentially added into a 500 mL single-neck flask, and the system was placed in an ice bath and stirred. Lithium hydroxide monohydrate (586 mg) was slowly added to the system. After finishing the addition, the system was heated to room temperature and stirred to react for 2 hours. Subsequently, hydrochloric acid aqueous solution (6 M, 20 mL) was added to the system to adjust a pH value to 1-2, stirred for 5 minutes, and concentrated under reduced pressure to remove the tetrahydrofuran in the system. Subsequently, water was added to the system to dilute the concentrate and stirred for 10 minutes, and filtered. A filter cake was collected, and washed with an acetone/n-hexane mixed solvent to obtain 1.4 g of light yellow solids.

### Preparation of intermediate 4

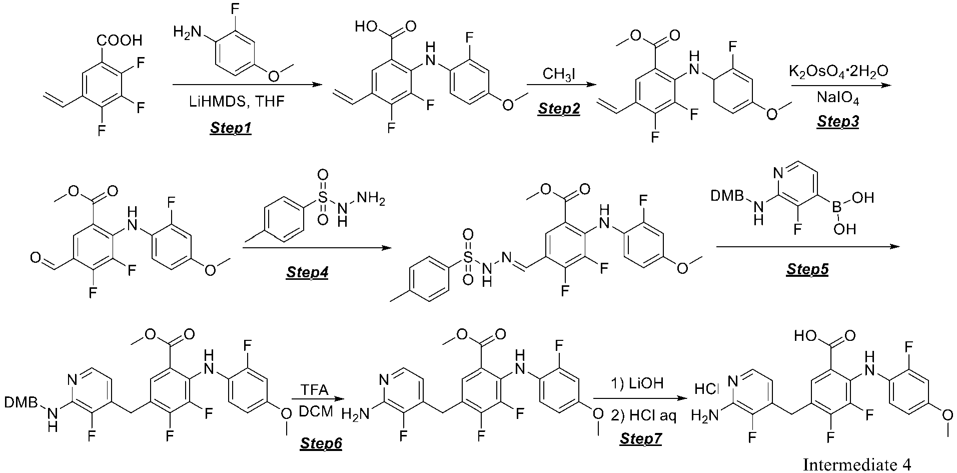

### Step 1: preparation of 3,4-difluoro-2-(2-fluoro-4-methoxyphenylamino)-5-vinylbenzoic acid

2,3,4-trifluoro-5-vinylbenzoic acid (10.0 g), 2-fluoro-4-methoxyaniline (7.0 g) and tetrahydrofuran (250 mL) were added into a 1 L three-neck flask. Subsequently, the system was subjected to nitrogen replacement, and cooled to -15°C to -20°C. Lithium bis(trimethylsilyl)amide (1 M, 150 mL) was slowly dropwise added to the system, and in this period, it was ensured that a temperature of the system was not higher than -15°C. After finishing the dropwise addition, the temperature of the system was slowly increased to room temperature, then sodium bisulfate aqueous solution (1.5 M) was added to adjust a pH value of the system to 1-2, and the mixture was extracted with methyl tert-butyl ether. Organic phases were combined, washed with 1 L of saturated brine, dried and concentrated to obtain 15.1 g of yellow solids. ESI-MS: [M - H]⁻ = 322.1.

### Step 2: preparation of methyl 3,4-difluoro-2-(2-fluoro-4-methoxyphenylamino)-5-vinylbenzoate

3,4-difluoro-2-(2-fluoro-4-methoxyphenylamino)-5-vinylbenzoic acid (16.2 g) and N,N-dimethylformamide (160 mL) were added into a 1 L three-neck flask, and the system was stirred at room temperature until a clear solution was obtained. Potassium carbonate (8.3 g) and methyl iodide (8.5 g) were added to the system. Subsequently, the system reacted at 35°C for 4 hours. Suspended substances in the system were removed by filtration, and a filtrate was collected. The filtrate was slowly dropwise added into water to precipitate solids. After finishing the dropwise addition, the system was continuously stirred for 1 hour, and subjected to suction filtration. The filter cake was washed with n-heptane to obtain 16.1 g of light yellow solids. ESI-MS: [M + H]⁺ = 338.1.

### Step 3: preparation of methyl 3,4-difluoro-2-((2-fluoro-4-methoxyphenyl)amino)-5-formylbenzoate

Methyl 3,4-difluoro-2-(2-fluoro-4-methoxyphenylamino)-5-vinylbenzoate (15.8 g) and tetrahydrofuran (314 mL) were added into a 500 mL three-neck flask, and stirred in an ice bath until a clear solution was obtained. Potassium osmate dihydrate (0.86 g) was added to the system, and the mixture was continuously stirred in an ice bath to react. After 15 minutes, water (312 mL), sodium periodate (30.0 g) and 2,6-dimethylpyridine (5.1 g) were added to the system, and the system was heated to room temperature and stirred to react for 4 hours. The reaction system was poured into a saturated sodium sulfite aqueous solution, and extracted with ethyl acetate. Organic phases were combined, washed with saturated brine, and concentrated. The concentrate was diluted with acetone. Water was slowly dropwise added to the system, the mixture was stirred, and subjected to suction filtration. The filter cake was dried to obtain 12.7 g of light yellow solids.

### Step 4: preparation of methyl (E)-3,4-difluoro-2-((2-fluoro-4-methoxyphenyl)amino)-5-((2-p-toluenesulfonylhydrazinyl)methyl)b enzoate

Methyl 3,4-difluoro-2-((2-fluoro-4-methoxyphenyl)amino)-5-formylbenzoate (4.0 g), p-toluenesulfonyl hydrazide (2.2 g) and ethanol (120 mL) were added into a 500 mL three-neck flask, and the system was heated to 60°C to react for 1 hour. The system was cooled to 0°C in an ice bath to precipitate a large number of solids, and subjected to suction filtration. The filter cake was washed with ethanol to obtain 5.1 g of light yellow solids. ESI-MS: [M + H]⁺ = 508.1.

### Step 5: preparation of methyl 5-((2-((2,4-dimethoxybenzyl)amino)-3-fluoropyridin-4-yl)methyl)-3,4-difluoro-2-((2-fluoro-4-meth oxyphenyl)amino)benzoate

(2-((2,4-dimethoxybenzyl)amino)-3-fluoropyridin-4-yl)boronic acid (3.6 g), methyl (*E*)-3,4-difluoro-2-((2-fluoro-4-methoxyphenyl)amino)-5-((2-p-toluenesulfonylhydrazinyl)methyl)b enzoate (5.0 g), potassium carbonate (1.63 g) and toluene (150 mL) were added into a 500 mL single-neck flask, and the system was subjected to nitrogen purging. The system was heated to 105°C to react for 3 hours. After cooling to room temperature, saturated ammonium chloride aqueous solution was added to the system, and the mixture was extracted with ethyl acetate. Organic phases were combined, dried with anhydrous sodium sulfate, filtered and subjected to rotary evaporation to remove the solvent. The crude product was purified by silica gel column chromatography to obtain 4.4 g of light yellow liquid. ESI-MS: [M + H]⁺ = 586.2.

### Step 6: preparation of methyl 5-((2-amino-3-fluoropyridin-4-yl)methyl)-3,4-difluoro-2-((2-fluoro-4-methoxyphenyl)amino)benzo ate

Methyl 5-((2-((2,4-dimethoxybenzyl)amino)-3-fluoropyridin-4-yl)methyl)-3,4-difluoro-2-((2-fluoro-4-meth oxyphenyl)amino)benzoate (4.4 g) and dichloromethane (27 mL) were added into a 500 mL single-neck flask, and stirred at room temperature until a clear solution was obtained. Subsequently, the system was placed in an ice bath, and trifluoroacetic acid (9.1 mL) was added. After finishing the addition, the system was heated to room temperature and stirred to react for 1.5 hours. Saturated sodium carbonate aqueous solution was added to the system to adjust a pH value of the system to 8, and the mixture was extracted with dichloromethane. Organic phases were combined, dried with anhydrous sodium sulfate, filtered and subjected to rotary evaporation to remove the solvent, so as to obtain 3.1 g of light yellow solids. ESI-MS: [M + H]⁺ = 436.1.

### Step 7: preparation of 5-((2-amino-3-fluoropyridin-4-yl)methyl)-3,4-difluoro-2-((2-fluoro-4-methoxyphenyl)amino)benzo ic acid hydrochloride

Methyl 5-((2-amino-3-fluoropyridin-4-yl)methyl)-3,4-difluoro-2-((2-fluoro-4-methoxyphenyl)amino)benzo ate (3.0 g), acetonitrile (18 mL) and ethanol (12 mL) were sequentially added into a 50 mL single-neck flask, and the system was placed in an ice bath and stirred. Aqueous solution (18 mL) of lithium hydroxide monohydrate (870 mg) was slowly dropwise added to the system. After finishing the addition, the system was heated to room temperature and stirred to react for 2.5 hours. Subsequently, sodium bisulfate aqueous solution (1.5 M) was added to the system to adjust a pH value to 1-2, the mixture was stirred for 5 minutes, and filtered. A filter cake was collected, and washed once with water and once with methyl tert-butyl ether respectively to obtain 2.7 g of off-white solids. ESI-MS: [M + H]⁺ = 422.1.

### Preparation of intermediate 5

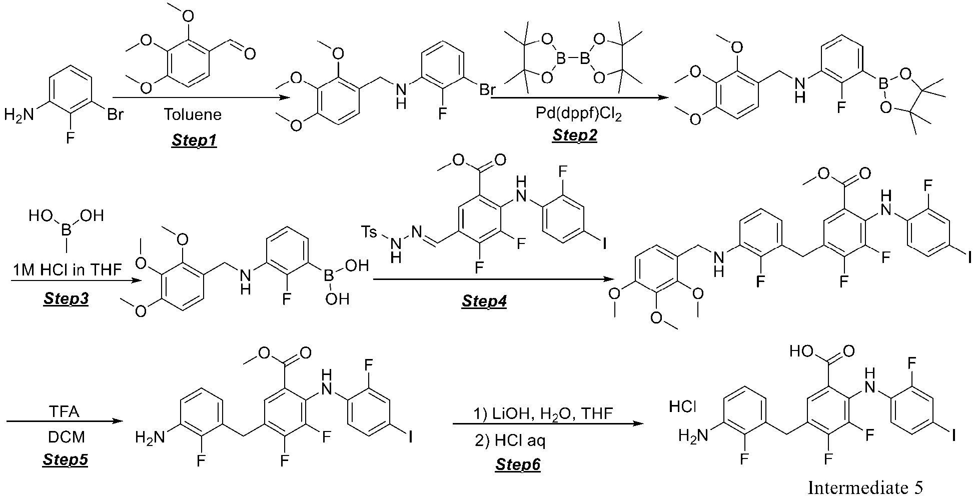

### Step 1: preparation of 3-bromo-2-fluoro-N-(2,3,4-trimethoxybenzyl)aniline

3-bromo-2-fluoroaniline (10.1 g), 2,3,4-trimethoxybenzaldehyde (10.0 g) and toluene (200 mL) were added into a 250 mL single-neck flask, and the system was heated to reflux and then stirred to react for 2 hours. After stopping heating, and then cooling to room temperature, the system was concentrated, and the concentrate was diluted with 150 mL of 1,2-dichloroethane. 3 mL of acetic acid and sodium triacetoxyborohydride (21.0 g) were added to the system, and the mixture was stirred at room temperature to react for 12 hours. The system was poured into water, and extracted with dichloromethane. Organic phases were combined, dried and concentrated to obtain 17.0 g of brown solids.

### Step 2: preparation of 2-fluoro-3-(4,4,5,5-tetramethyl-1,3,2-dioxaborolan-2-yl)-N-(2,3,4-trimethoxybenzyl)aniline

3-bromo-2-fluoro-N-(2,3,4-trimethoxybenzyl)aniline (5.2 g), bis(pinacolato)diboron (7.2 g), potassium pivalate (4.0 g), toluene (125 mL), dimethyl sulfoxide (2.2 g) and [1,1'-bis(diphenylphosphino)ferrocene]dichloropalladium (1.0 g) were added into a 250 mL single-neck flask, and the system was subjected to nitrogen purging. Subsequently, the system was heated to 55°C to react for 19 hours. After cooling to room temperature, the system was poured into water, and extracted with ethyl acetate. Organic phases were combined, dried with anhydrous sodium sulfate and concentrated. Subsequently, the concentrate was diluted with n-hexane, dried and filtered with diatomite, and the filtrate was concentrated to obtain 4.7 g of yellow solids.

### Step 3: preparation of (2-fluoro-3-((2,3,4-trimethoxybenzyl)amino)phenyl)boronic acid

2-fluoro-3-(4,4,5,5-tetramethyl-1,3,2-dioxaborolan-2-yl)-N-(2,3,4-trimethoxybenzyl)aniline (4.7 g) and tetrahydrofuran (100 mL) were added into a 250 mL single-neck flask, and stirred until a clear solution was obtained. 1 M hydrochloric acid aqueous solution (50 mL) was sloly added to the system, and the mixture was stirred at room temperature to react for 1 hour. Methylboric acid (2.4 g) was added to the system, and the mixture was stirred at room temperature to react for 24 hours. Subsequently, the system was extracted with petroleum ether. Organic phases were combined, dried and concentrated to obtain 1.1 g of light yellow solids. ESI-MS: [M + H]⁺ = 336.1.

### Step 4: preparation of methyl 3,4-difluoro-5-(2-fluoro-3-((2,3,4-trimethoxybenzyl)amino)benzyl)-2-((2-fluoro-4-iodophenyl)amin o)benzoate

(2-fluoro-3-((2,3,4-trimethoxybenzyl)amino)phenyl)boronic acid (1.1 g), methyl (*E*)-3,4-difluoro-2-((2-fluoro-4-iodophenyl)amino)-5-((2-p-toluenesulfonylhydrazinyl)methyl)benz oate (2.4 g), potassium carbonate (540 mg) and toluene (440 mL) were added into a 100 mL single-neck flask, and the system was subjected to nitrogen purging. The system was heated to 100°C to react for 5 hours. After cooling to room temperature, saturated ammonium chloride aqueous solution was added to the system, and the mixture was extracted with ethyl acetate. Organic phases were combined, dried with anhydrous sodium sulfate, filtered and subjected to rotary evaporation to remove the solvent. The crude product was purified by silica gel column chromatography to obtain 1.1 g of light yellow liquid. ESI-MS: [M + H]⁺ = 711.1.

### Step 5: preparation of methyl 5-(3-amino-2-fluorobenzyl)-3,4-difluoro-2-((2-fluoro-4-iodophenyl)amino)benzoate

Methyl 3,4-difluoro-5-(2-fluoro-3-((2,3,4-trimethoxybenzyl)amino)benzyl)-2-((2-fluoro-4-iodophenyl)amin o)benzoate (1.9 g) and dichloromethane (30 mL) were added into a 100 mL single-neck flask, and stirred at room temperature until a clear solution was obtained. Subsequently, the system was placed in an ice bath, and trifluoroacetic acid (30 mL) was slowly added. After finishing the addition, the system was heated to room temperature and stirred to react for 3.5 hours. P-toluenesulfonic acid (515 mg) was added to the system, and the mixture was heated to 40°C and stirred to react for 24 hours. The system was concentrated, and then the concentrate was diluted with a saturated sodium carbonate aqueous solution, adjusted to reach a pH value of 8, and extracted with ethyl acetate. Organic phases were combined, dried, filtered and concentrated. The concentrate was purified by silica gel column chromatography to obtain 670 mg of light yellow solids. ESI-MS: [M + H]⁺ = 531.0.

### Step 6: preparation of 5-(3-amino-2-fluorobenzyl)-3,4-difluoro-2-((2-fluoro-4-iodophenyl)amino)benzoic acid hydrochloride

Methyl 5-(3-amino-2-fluorobenzyl)-3,4-difluoro-2-((2-fluoro-4-iodophenyl)amino)benzoate (670 mg), tetrahydrofuran (20 mL) and water (10 mL) were sequentially added into a 100 mL single-neck flask, and the system was placed in an ice bath and stirred. Lithium hydroxide monohydrate (160 g) was slowly added to the system. After finishing the addition, the system was heated to room temperature and stirred to react for 13 hours. Subsequently, hydrochloric acid aqueous solution (2 M) was added to the system to adjust a pH value to 1-2, stirred for 5 minutes, and concentrated under reduced pressure to remove tetrahydrofuran in the system. Subsequently, water was added to the system to dilute the concentrate and stirred for 10 minutes, and filtered to obtain 630 mg of light yellow solids. ESI-MS: [M + H]⁺ = 517.0.

### Preparation of intermediate 6

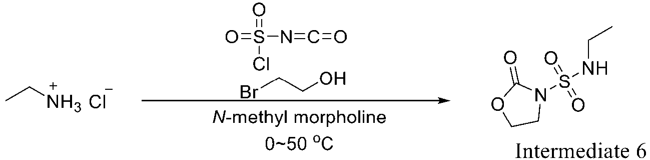

### Preparation of N-ethyl-2-oxooxazoline-3-sulfonamide

In an ice bath, 120 mL of acetonitrile and chlorosulfonyl isocyanate (3.2 g) were added into a 500 mL single-neck flask. Subsequently, 2-bromoethanol (2.8 g) was slowly added to the system. The system was continuously kept in the ice bath and stirred to react for 30 minutes, and then heated to room temperature and stirred to react for 1 hour. Subsequently, N-methyl morpholine (15.1 g) was slowly added to the system and the mixture was stirred to react for 10 minutes, and then ethylamine hydrochloride (1.8 g) was slowly added to the system. After finishing the addition, the system was heated to 50°C and stirred to react for 5 hours. Subsequently, the system was gradually cooled to room temperature, then continuously stirred to react for 15 hours, and subjected to suction filtration, and the filtrate was concentrated. The concentrate was diluted with ethyl acetate, and washed with saturated brine. Organic phases were dried with anhydrous sodium sulfate, filtered and concentrated to obtain 1.8 g of white solids. ¹H NMR (400 MHz, DMSO-*d*₆, δ): 8.34 (t, *J* = 5.2 Hz, 1H), 4.41-4.37 (m, 2H), 3.96-3.92 (m, 2H), 3.08-3.01 (m, 2H), 1.08 (t, *J =* 7.2 Hz, 3H).

### Preparation of intermediate 7

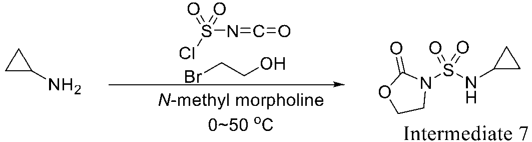

### Preparation of N-cyclopropyl-2-oxooxazoline-3-sulfonamide

In an ice bath, 500 mL of acetonitrile and chlorosulfonyl isocyanate (11.6 g) were added into a 1 L single-neck flask. Subsequently, 2-bromoethanol (10.3 g) was slowly added to the system. The system was continuously kept in the ice bath and stirred to react for 30 minutes, and then heated to room temperature and stirred to react for 1 hour. Subsequently, N-methyl morpholine (50.0 g) was slowly added to the system and the mixture was stirred to react for 10 minutes, and then cyclopropylamine (4.7 g) was slowly added to the system. After finishing the addition, the system was heated to 50°C and stirred to react for 5 hours. Subsequently, the system was gradually cooled to room temperature, then continuously stirred for 15 hours, and subjected to suction filtration, and the filtrate was concentrated to obtain light orange solids. Most of the solids were dissolved with ethyl acetate, and washed with saturated brine. Organic phases were separated, dried with anhydrous sodium sulfate, filtered and subjected to rotary evaporation to remove the solvent to obtain a crude product. The crude product was washed with methyl tert-butyl ether to obtain 4.4 g of off-white solids. ¹H NMR (400 MHz, DMSO-*d*₆, δ): 8.72 (s, 1H), 4.44-4.40 (m, 2H), 4.00-3.96 (m, 2H), 2.50-2.45 (m, 1H), 0.65-0.58 (m, 2H), 0.56-0.52 (m, 2H).

### Preparation of intermediate 8

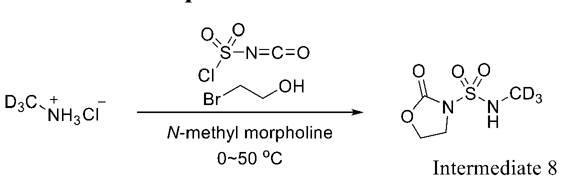

### Preparation of N-methyl-d₃-2-oxooxazoline-3-sulfonamide

In an ice bath, 720 mL of acetonitrile and chlorosulfonyl isocyanate (20.6 g) were added into a 2 L single-neck flask. Subsequently, 2-bromoethanol (18.2 g) was slowly added to the system. The system was continuously kept in the ice bath and stirred to react for 30 minutes, and then heated to room temperature and stirred to react for 1 hour. Subsequently, N-methyl morpholine (58.4 g) was slowly added to the system and the mixture was stirred to react for 10 minutes, and then deuterated methylamine hydrochloride (10.2 g) was slowly added to the system. After finishing the addition, the system was heated to 50°C and stirred to react for 5 hours. Subsequently, the system was gradually cooled to room temperature, then continuously stirred to react for 15 hours, and subjected to suction filtration, and the filtrate was concentrated. The concentrate was diluted with water, and extracted with ethyl acetate. Organic phases were combined, washed with saturated brine and dried with anhydrous sodium sulfate, filtered and subjected to rotary evaporation to remove the solvent, so as to obtain 11.9 g of light yellow solids. ¹H NMR (400 MHz, DMSO-*d*₆, δ): 8.17 (s, 1H), 4.42-4.38 (m, 2H), 3.96-3.93 (m, 2H).

### Preparation of compound

### Example 1

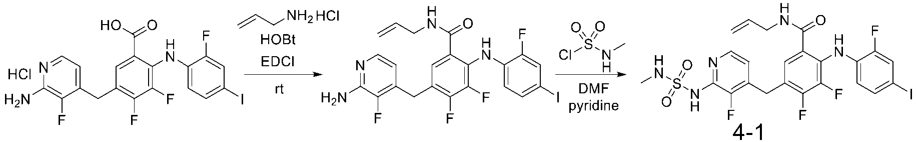

### Step 1: preparation of N-allyl-5-((2-amino-3-fluoropyridin-4-yl)methyl)-3,4-difluoro-2-((2-fluoro-4-iodophenyl)amino)be nzamide

5-((2-amino-3-fluoropyridin-4-yl)methyl)-3,4-difluoro-2-((2-fluoro-4-iodophenyl)amino)benz oic acid hydrochloride (1.1 g), 1-hydroxybenzotriazole (646 mg), 1-(3-dimethylaminopropyl)-3-ethylcarbodiimide hydrochloride (763 mg) and N,N-dimethylformamide (11 mL) were added into a 100 mL single-neck flask, and the system was stirred at room temperature to react for 2 hours. Subsequently, allylamine hydrochloride (560 mg) and N,N-diisopropylethylamine (1.0 g) were added to the system, and the mixture was continuously stirred at room temperature to react for 2 hours. The reaction system was added into a saturated sodium bicarbonate aqueous solution to precipitate solids, and the mixture was subjected to suction filtration. The filter cake was purified by silica gel column chromatography to obtain 300 mg of light yellow solids. ESI-MS: [M + H]⁺ = 557.1.

### Step 2: preparation of N-allyl-3,4-difluoro-5-((3-fluoro-2-((N-methylaminosulfonyl)amino)pyridin-4-yl)methyl)-2-((2-flu oro-4-iodophenyl)amino)benzamide

N-allyl-5-((2-amino-3-fluoropyridin-4-yl)methyl)-3,4-difluoro-2-((2-fluoro-4-iodophenyl)amin o)benzamide (300 mg), N,N-dimethylformamide (5.0 mL) and pyridine (422 mg) were added into a 50 mL single-neck flask, and the system was placed in an ice bath and stirred. After cooling to below 0°C, methylaminosulfonyl chloride (210 mg) was slowly dropwise added to the system. After 30 minutes, the reaction was stopped, and the system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 100 mg of off-white solids. ¹H NMR (400 MHz, DMSO-*d*₆): δ 10.40 (s, 1H), 9.05 (s, 1H), 8.87 (t, *J* = 5.6 Hz, 1H), 8.02 (d, *J =* 4.8 Hz, 1H), 7.59-7.57 (m, 2H), 7.36 (d, *J* = 8.4 Hz, 1H), 7.04-7.00 (m, 1H), 6.95-6.93 (m, 1H), 6.72-6.66 (m, 1H), 5.87-5.77 (m, 1H), 5.17-5.06 (m, 2H), 4.07 (s, 2H), 3.84 (t, *J =* 5.2 Hz, 2H), 2.53-2.51 (m, 3H). ESI-MS: [M + H]⁺ = 650.0.

### Example 2

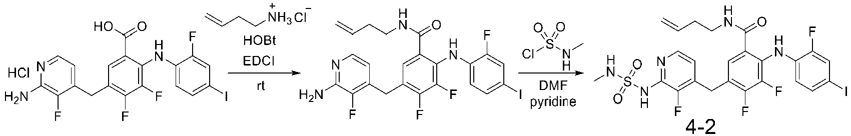

### Step 1 : preparation of 5-((2-amino-3-fluoropyridin-4-yl)methyl)-N-(but-3-en-1-yl)-3,4-difluoro-2-((2-fluoro-4-iodophenyl )amino)benzamide

5-((2-amino-3-fluoropyridin-4-yl)methyl)-3,4-difluoro-2-((2-fluoro-4-iodophenyl)amino)benz oic acid hydrochloride (300 mg), 1-hydroxybenzotriazole (149 mg), 1-(3-dimethylaminopropyl)-3-ethylcarbodiimide hydrochloride (414 mg) and N,N-dimethylformamide (5 mL) were added into a 100 mL single-neck flask, and the system was stirred at room temperature to react for 2 hours. Subsequently, but-3-en-1-amine hydrochloride (117 mg) and N,N-diisopropylethylamine (348 mg) were added to the system, and the mixture was continuously stirred at room temperature to react for 2 hours. The reaction system was added into a saturated sodium bicarbonate aqueous solution to precipitate solids, and subjected to suction filtration. The filter cake was purified by silica gel column chromatography to obtain 148 mg of off-white solids. ESI-MS: [M + H]⁺ = 571.2.

### Step 2: preparation of N-(but-3-en-1-yl)-3,4-difluoro-5-((3-fluoro-2-((N-methylaminosulfonyl)amino)pyridin-4-yl)methyl )-2-((2-fluoro-4-iodophenyl)amino)benzamide

5-((2-amino-3-fluoropyridin-4-yl)methyl)-N-(but-3-en-1-yl)-3,4-difluoro-2-((2-fluoro-4-iodop henyl)amino)benzamide (148 mg), N,N-dimethylformamide (5 mL) and pyridine (205 mg) were added into a 50 mL single-neck flask, and the system was placed in an ice bath and stirred. After cooling to below 0°C, methylaminosulfonyl chloride (326 mg) was slowly dropwise added to the system. After 30 minutes, the reaction was stopped, and the system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 70 mg of off-white solids. ¹H NMR (400 MHz, DMSO-*d*₆): δ 10.42 (s, 1H), 9.00 (s, 1H), 8.69 (t, *J =* 5.6 Hz, 1H), 8.02 (d, *J =* 5.2 Hz, 1H), 7.60-7.57 (m, 1H), 7.48 (d, *J =* 7.2 Hz, 1H), 7.36 (d, *J =* 8.4 Hz, 1H), 7.05-7.01 (m, 1H), 6.93 (t, *J =* 4.4 Hz, 1H), 7.00-6.64 (m, 1H), 5.81-5.71 (m, 1H), 5.04-4.94 (m, 2H), 4.07 (s, 2H), 3.25 (q, *J* = 6.4 Hz, 2H), 2.52-2.51 (m, 3H), 2.21 (q, *J* = 6.8 Hz, 2H). ESI-MS: [M + H]⁺ = 664.2.

### Example 3

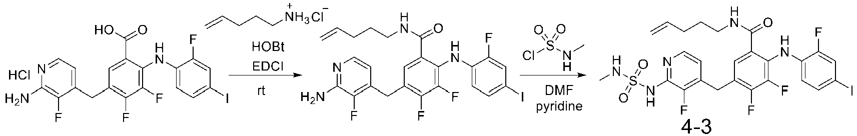

### Step 1: preparation of 5-((2-amino-3-fluoropyridin-4-yl)methyl)-3,4-difluoro-2-((2-fluoro-4-iodophenyl)amino)-N-(pent-4 -en-1-yl)benzamide

5-((2-amino-3-fluoropyridin-4-yl)methyl)-3,4-difluoro-2-((2-fluoro-4-iodophenyl)amino)benz oic acid hydrochloride (400 g), 1-hydroxybenzotriazole (105 mg), 1-(3-dimethylaminopropyl)-3-ethylcarbodiimide hydrochloride (177 mg) and N,N-dimethylformamide (15 mL) were added into a 100 mL single-neck flask, and the system was stirred at room temperature to react for 2 hours. Subsequently, 4-penten-1-amine hydrochloride (113 mg) and N,N-diisopropylethylamine (250 g) were added to the system, and the mixture was continuously stirred at room temperature to react for 2 hours. The reaction system was added into a saturated sodium bicarbonate aqueous solution to precipitate solids, and subjected to suction filtration to obtain 400 mg of light yellow solids. ESI-MS: [M + H]⁺ = 585.3.

### Step 2: preparation of 3,4-difluoro-5-((3-fluoro-2-((N-methylaminosulfonyl)amino)pyridin-4-yl)methyl)-2-((2-fluoro-4-io dophenyl)amino)-N-(pent-4-en-1-yl)benzamide

5-((2-amino-3-fluoropyridin-4-yl)methyl)-3,4-difluoro-2-((2-fluoro-4-iodophenyl)amino)-N-(p ent-4-en-1-yl)benzamide (400 mg), N,N-dimethylformamide (4 mL), dichloromethane (4 mL) and pyridine (270 mg) were added into a 50 mL single-neck flask, and the system was placed in an ice bath and stirred. After cooling to below 0°C, methylaminosulfonyl chloride (355 mg) was slowly dropwise added to the system. After finishing the addition, the reaction was continued for 30 minutes and then stopped. The system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 110 mg of off-white solids. ¹H NMR (400 MHz, DMSO-*d*₆): δ 10.41 (s, 1H), 9.02 (s, 1H), 8.67 (t, *J =* 5.2 Hz, 1H), 8.02 (d, *J* = 4.8 Hz, 1H), 7.58 (dd, *J*₁ = 10.8 Hz, *J*₂ = 1.6 Hz, 1H), 7.52 (d, *J* = 6.8 Hz, 1H), 7.36 (d, *J* = 8.8 Hz, 1H), 7.03 (q, *J* = 5.2 Hz, 1H), 6.93 (t, *J* = 4.8 Hz, 1H), 7.00-6.64 (m, 1H), 5.84-5.74 (m, 1H), 5.02-4.93 (m, 2H), 4.08 (s, 2H), 3.19 (q, *J =* 6.4 Hz, 2H), 2.52-2.51 (m, 3H), 2.01 (q, *J =* 7.2 Hz, 2H), 1.57-1.50 (m, 2H). ESI-MS: [M + H]⁺ = 678.3.

### Example 4

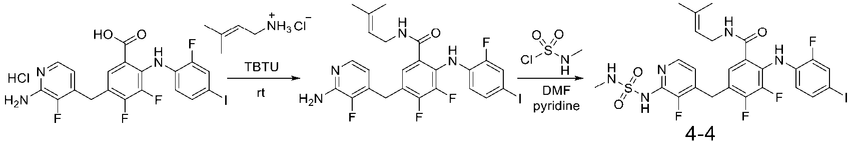

### Step 1: preparation of 5-((2-amino-3-fluoropyridin-4-yl)methyl)-3,4-difluoro-2-((2-fluoro-4-iodophenyl)amino)-N-(3-met hylbut-2-en-1-yl)benzamide

5-((2-amino-3-fluoropyridin-4-yl)methyl)-3,4-difluoro-2-((2-fluoro-4-iodophenyl)amino)benz oic acid hydrochloride (400 mg), 3-methylbut-2-en-1-amine (63 mg), N,N-diisopropylethylamine (373 mg) and N,N-dimethylformamide (8 mL) were added into a 100 mL single-neck flask, and the system was stirred at room temperature to react for 5 minutes. Subsequently, O-benzotriazole-N,N,N',N'-tetramethyluronium tetrafluoroborate (763 mg) was added to the system, and the mixture was continuously stirred at room temperature to react for 1.5 hours. The reaction system was added into a saturated sodium bicarbonate aqueous solution, and extracted with ethyl acetate. Organic phases were combined, dried with anhydrous sodium sulfate, filtered and subjected to rotary evaporation to remove the solvent. The crude product was purified by silica gel column chromatography to obtain 310 mg of off-white solids. ESI-MS: [M + H]⁺ = 585.3.

### Step 2: preparation of 3,4-difluoro-5-((3-fluoro-2-((N-methylaminosulfonyl)amino)pyridin-4-yl)methyl)-2-((2-fluoro-4-io dophenyl)amino)-N-(3-methylbut-2-en-1-yl)benzamide

5-((2-amino-3-fluoropyridin-4-yl)methyl)-3,4-difluoro-2-((2-fluoro-4-iodophenyl)amino)-N-(3 -methylbut-2-en-1-yl)benzamide (310 mg), N,N-dimethylformamide (6 mL) and pyridine (335 mg) were added into a 50 mL single-neck flask, and the system was placed in an ice bath and stirred. After cooling to below 0°C, methylaminosulfonyl chloride (272 mg) was slowly dropwise added to the system. After 30 minutes, the reaction was stopped, and the system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 200 mg of off-white solids. ¹H NMR (400 MHz, DMSO-*d*₆): δ 10.41 (s, 1H), 9.11 (s, 1H), 8.74 (t, *J* = 5.2 Hz, 1H), 8.20 (d, *J* = 4.8 Hz, 1H), 7.59 (dd, *J*₁ = 10.8 Hz, *J*₂ = 2.0 Hz, 1H), 7.54 (d, *J =* 6.8 Hz, 1H), 7.36 (d, *J =* 5.2 Hz, 1H), 7.03 (d, *J =* 4.8 Hz, 1H), 6.93 (s, 1H), 6.70-6.65 (m, 1H), 5.14 (t, *J =* 6.8 Hz, 1H), 4.07 (s, 2H), 3.79 (t, *J =* 6.0 Hz, 2H), 2.52-2.51 (m, 3H), 1.67 (s, 3H), 1.63 (s, 3H). ESI-MS: [M + H]⁺ = 678.3.

### Example 5

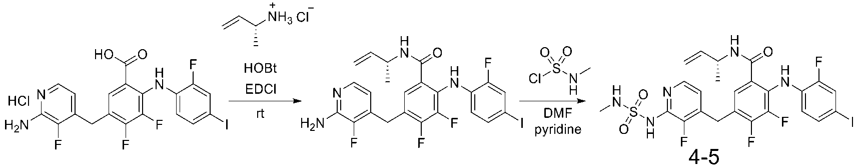

### Step 1: preparation of (R)-5-((2-amino-3-fluoropyridin-4-yl)methyl)-N-(but-3-en-2-yl)-3,4-difluoro-2-((2-fluoro-4-iodoph enyl)amino)benzamide

5-((2-amino-3-fluoropyridin-4-yl)methyl)-3,4-difluoro-2-((2-fluoro-4-iodophenyl)amino)benz oic acid hydrochloride (500 mg), 1-hydroxybenzotriazole (232 mg), 1-(3-dimethylaminopropyl)-3-ethylcarbodiimide hydrochloride (641 mg) and N,N-dimethylformamide (20 mL) were added into a 100 mL single-neck flask, and the system was stirred at room temperature to react for 2 hours. Subsequently, (R)-but-3-en-2-amine hydrochloride (195 mg) and N,N-diisopropylethylamine (350 mg) were added to the system, and the mixture was continuously stirred at room temperature to react for 2 hours. The reaction system was added into a saturated sodium bicarbonate aqueous solution to precipitate solids, and subjected to suction filtration. The filter cake was purified by silica gel column chromatography to obtain 340 mg of light yellow solids. ESI-MS: [M + H]⁺ = 571.2.

### Step 2: preparation of (R)-N-(but-3-en-2-yl)-3,4-difluoro-5-((3-fluoro-2-((N-methylaminosulfonyl)amino)pyridin-4-yl)me thyl)-2-((2-fluoro-4-iodophenyl)amino)benzamide

(*R*)-5-((2-amino-3-fluoropyridin-4-yl)methyl)-N-(but-3-en-2-yl)-3,4-difluoro-2-((2-fluoro-4-io dophenyl)amino)benzamide (330 mg), N,N-dimethylformamide (15 mL) and pyridine (458 mg) were added into a 50 mL single-neck flask, and the system was placed in an ice bath and stirred. After cooling to below 0°C, methylaminosulfonyl chloride (225 mg) was slowly dropwise added to the system. After finishing the addition, the reaction was stopped 30 minutes later, and the system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 320 mg of off-white solids. ¹H NMR (400 MHz, DMSO-*d*₆): δ 10.41 (s, 1H), 8.95 (s, 1H), 8.64 (d, *J =* 8.0 Hz, 1H), 8.02 (d, *J* = 5.2 Hz, 1H), 7.58 (d, *J* = 8.8 Hz, 2H), 7.36 (d, *J* = 8.8 Hz, 1H), 7.02 (q, *J* = 5.2 Hz, 1H), 6.93 (t, *J =* 4.8 Hz, 1H), 7.00-6.64 (m, 1H), 5.89-5.81 (m, 1H), 5.13-5.01 (m, 2H), 4.54-4.46 (m, 1H), 4.09 (s, 2H), 2.52-2.51 (m, 3H), 1.18 (d, *J =* 6.8 Hz, 3H). ESI-MS: [M + H]⁺ = 664.2.

### Example 6

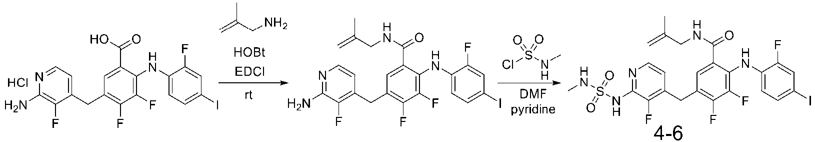

### Step 1: preparation of 5-((2-amino-3-fluoropyridin-4-yl)methyl)-3,4-difluoro-2-((2-fluoro-4-iodophenyl)amino)-N-(2-met hylallyl)benzamide

5-((2-amino-3-fluoropyridin-4-yl)methyl)-3,4-difluoro-2-((2-fluoro-4-iodophenyl)amino)benz oic acid hydrochloride (400 mg), 1-hydroxybenzotriazole (195 mg), 1-(3-dimethylaminopropyl)-3-ethylcarbodiimide hydrochloride (552 mg) and N,N-dimethylformamide (5 mL) were added into a 100 mL single-neck flask, and the system was stirred at room temperature to react for 2 hours. Subsequently, 2-methylprop-2-en-1-amine (102 mg) and N,N-diisopropylethylamine (464 mg) were added to the system, and continuously stirred at room temperature to react for 2 hours. The reaction system was added into a saturated sodium bicarbonate aqueous solution to precipitate solids, and subjected to suction filtration. The filter cake was purified by silica gel column chromatography to obtain 200 mg of white solids. ESI-MS: [M + H]⁺ = 571.1.

### Step 2: preparation of 3,4-difluoro-5-((3-fluoro-2-((N-methylaminosulfonyl)amino)pyridin-4-yl)methyl)-2-((2-fluoro-4-io dophenyl)amino)-N-(2-methylallyl)benzamide

5-((2-amino-3-fluoropyridin-4-yl)methyl)-3,4-difluoro-2-((2-fluoro-4-iodophenyl)amino)-N-(2 -methylallyl)benzamide (200 mg), N,N-dimethylformamide (3 mL) and pyridine (278 mg) were added into a 50 mL single-neck flask, and the system was placed in an ice bath and stirred. After cooling to below 0°C, methylaminosulfonyl chloride (208 mg) was slowly dropwise added to the system. After 30 minutes, the reaction was stopped, and the system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 170 mg of off-white solids. ¹H NMR (400 MHz, DMSO-*d*₆): δ 10.43 (s, 1H), 9.00 (s, 1H), 8.92 (t, *J =* 5.6 Hz, 1H), 8.02 (d, *J =* 4.8 Hz, 1H), 7.60-7.57 (m, 2H), 7.36 (d, *J =* 8.4 Hz, 1H), 7.06-7.02 (m, 1H), 6.95 (t, *J =* 4.8 Hz, 1H), 6.71-6.65 (m, 1H), 4.78 (s, 2H), 4.08 (s, 2H), 3.76 (d, *J =* 5.6 Hz, 2H), 2.51-2.50 (m, 3H), 1.67 (s, 3H). ESI-MS: [M + H]⁺ = 664.0.

### Example 7

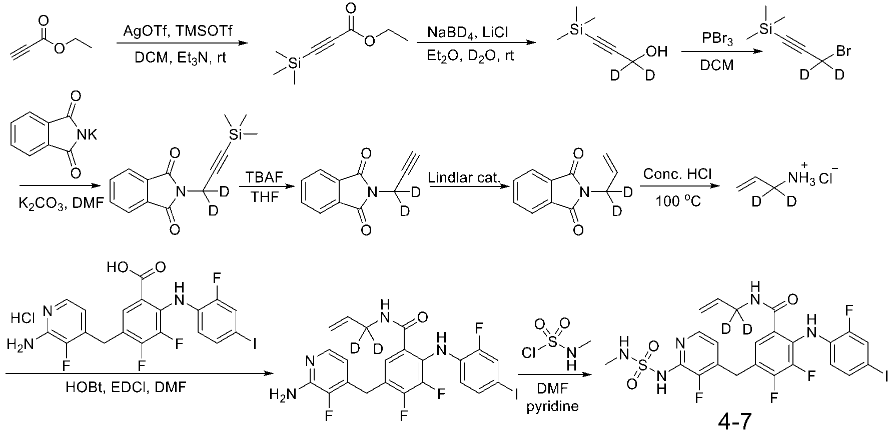

### Step 1: preparation of ethyl 3-(trimethylsilyl)propionate

1000 mL of dichloromethane, trimethylsilyl trifluoromethanesulfonate (271.8 g), triethylamine (123.7 g) and silver trifluoromethanesulfonate (10 g) were added into a 3 L reaction flask, dichloromethane (100 mL) solution of ethyl propiolate (100 g) was slowly dropwise added to the system. After finishing the dropwise addition, the system was stirred at room temperature for 30 minutes, and concentrated under reduced pressure to remove the dichloromethane. The concentrated residue was extracted twice with n-hexane. The n-hexane extracts were combined and washed twice with a saturated ammonium chloride solution, and the n-hexane phase was dried with anhydrous sodium sulfate, filtered and concentrated under reduced pressure to obtain a light yellow liquid.

### Step 2: preparation of 3-(trimethylsilyl)prop-2-yn-1,1-d₂-1-ol

Ethyl 3-(trimethylsilyl)propionate (50 g), ether (500 mL), sodium borodeuterid (17.3 g), lithium chloride (17.4 g) and heavy water (8.2 g) were added into a 1000 mL three-neck flask. After finishing the addition, the system was stirred at room temperature for 40 minutes, and then heated to 33°C to react for 8 hours. The reaction system was slowly added into 10% acetic acid aqueous solution, extracted with n-hexane, and subjected to liquid-liquid separation, and an aqueous layer was extracted with n-hexane. Organic phases were combined, washed with 1 N hydrochloric acid solution, washed to be neutral, dried with anhydrous sodium sulfate, and concentrated under reduced pressure to obtain a light yellow liquid. ¹H NMR (400 MHz, DMSO-*d*₆): δ 5.20 (brs, 1H), 0.13 (s, 9H).

### Step 3: preparation of (3-bromoprop-1-yn-1-yl-3,3-d₂)trimethylsilane

3-(trimethylsilyl)prop-2-yn-1,1-*d*₂-1-ol (6.0 g) and dichloromethane (75 mL) were added into a 100 mL single-neck flask, and the system was cooled to -10°C. Subsequently, phosphorus tribromide (30.0 g) was dropwise added to the system. After finishing the addition, the system was heated to room temperature to react for 1 hour. The system was poured into ice, saturated sodium bicarbonate aqueous solution was added to adjust pH to be neutral, and the mixture was extracted with dichloromethane. Organic phases were combined, dried and concentrated to obtain 5.1 g of colorless clear liquid.

### Step 4: preparation of 2-((3-(trimethylsilyl)prop-2-yn-1-yl-1,1-d₂)isoindoline-1,3-dione

(3-bromoprop-1-yn-1-yl-3,3-*d*₂)trimethylsilane (10.0 g), potassium phthalimide (10.5 g), potassium carbonate (8.6 g) and N,N-dimethylformamide (100 mL) were added into a 250 mL single-neck flask, and the system was stirred at room temperature to react for 16 hours. The system was poured into water, and extracted with ethyl acetate. Organic phases were combined, dried with anhydrous sodium sulfate, filtered and concentrated to obtain 12.0 g of off-white solids.

### Step 5: preparation of 2-(prop-2-yn-1-yl-1,1-d₂)isoindoline-1,3-dione

2-((3-(trimethylsilyl)prop-2-yn-1-yl-1,1-*d*₂)isoindoline-1,3-dione (3.4 g), tetrahydrofuran (15 mL) and tetra-n-butylammonium fluoride trihydrate (10.8 g) were added into a 250 mL single-neck flask, and stirred at room temperature to react for 10 minutes. The system was poured into water, and extracted with ethyl acetate. Organic phases were combined, dried with anhydrous sodium sulfate, and concentrated to obtain 900 mg of off-white solids.

### Step 6: preparation of 2-(allyl-1,1-d₂)isoindoline-1,3-dione

2-(prop-2-yn-1-yl-1,1-*d*₂)isoindoline-1,3-dione (1.8 g), methanol (45 mL) and a Lindlar catalyst (180 mg) were added into a 100 mL single-neck flask, and the system was subjected to hydrogen purging, stirred at room temperature to react for 10 minutes, and subjected to suction filtration. A filtrate was collected, and concentrated to obtain 1.0 g of off-white solids.

### Step 7: preparation of prop-2-en-1,1-d₂-1-amine hydrochloride

2-(allyl-1,1-*d*₂)isoindoline-1,3-dione (1.0 g) and concentrated hydrochloric acid (30 mL) were added into a 100 mL single-neck flask, stirred at 100°C to react for 19 hours, and subjected to suction filtration. A filtrate was collected and washed with diethyl ether. An aqueous phase was collected, concentrated and dried to obtain 325 mg of off-white solids.

### Step 8: preparation of N-(allyl-1,1-d₂)-5-((2-amino-3-fluoropyridin-4-yl)methyl)-3,4-difluoro-2-((2-fluoro-4-iodophenyl)a mino)benzamide

5-((2-amino-3-fluoropyridin-4-yl)methyl)-3,4-difluoro-2-((2-fluoro-4-iodophenyl)amino)benz oic acid hydrochloride (700 mg), 1-hydroxybenzotriazole (205 mg), 1-(3-dimethylaminopropyl)-3-ethylcarbodiimide hydrochloride (483 mg) and N,N-dimethylformamide (10 mL) were added into a 100 mL single-neck flask, and the system was stirred at room temperature to react for 2 hours. Subsequently, prop-2-en-1,1-*d*₂-1-amine hydrochloride (325 mg) and N,N-diisopropylethylamine (325 mg) were added to the system, and the mixture was continuously stirred at room temperature to react for 2 hours. The reaction system was added into saturated brine to precipitate solids, and subjected to suction filtration. The filter cake was purified by silica gel column chromatography to obtain 500 mg of yellow solids. ESI-MS: [M + H]⁺ = 559.1.

### Step 9: preparation of N-(allyl-1,1-d₂) -3,4-difluoro-5-((3-fluoro-2-((N-methylaminosulfonyl)amino)pyridin-4-yl)methyl)-2-((2-fluoro-4-i odophenyl)amino)benzamide

N-(allyl-1,1-*d*₂)-5-((2-amino-3-fluoropyridin-4-yl)methyl)-3,4-difluoro-2-((2-fluoro-4-iodophe nyl)amino)benzamide (250 mg), N,N-dimethylformamide (5 mL) and pyridine (351 mg) were added into a 50 mL single-neck flask, and the system was placed in an ice bath and stirred. After cooling to below 0°C, methylaminosulfonyl chloride (175 mg) was slowly dropwise added to the system. After finishing the addition, the reaction was continued for 30 minutes and then stopped. The system was poured into saturated brine and stirred at room temperature for 10 minutes, and extracted with ethyl acetate. Organic phases were combined, dried with anhydrous sodium sulfate, filtered and subjected to rotary evaporation to remove the solvent. Subsequently, the crude product was purified by silica gel column chromatography to obtain 180 mg of off-white solids. ¹H NMR (400 MHz, DMSO-*d*₆): δ 10.42 (s, 1H), 9.06 (s, 1H), 8.88 (s, 1H), 8.02 (d, *J* = 5.2 Hz, 1H), 7.60-7.57 (m, 2H), 7.36 (d, *J =* 8.8 Hz, 1H), 7.03 (q, *J =* 4.8 Hz, 1H), 6.95 (t, *J =* 4.8 Hz, 1H), 6.72-6.66 (m, 1H), 5.81 (dd, *J*₁ = 17.2 Hz, *J*₂ = 10.4 Hz, 1H), 5.18-5.06 (m, 2H), 4.07 (s, 2H), 2.52-2.51 (m, 3H). ESI-MS: [M + H]⁺ = 652.1.

### Example 8

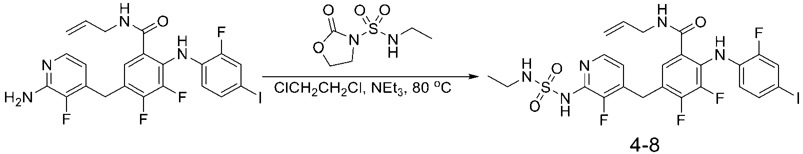

### Preparation of N-allyl-5-((2-((N-ethylaminosulfonyl)amino)-3-fluoropyridin-4-yl)methyl)-3,4-difluoro-2-((2-fluor o-4-iodophenyl)amino)benzamide

N-ethyl-2-oxooxazoline-3-sulfonamide (1.1 g), N-allyl-5-((2-amino-3-fluoropyridin-4-yl)methyl)-3,4-difluoro-2-((2-fluoro-4-iodophenyl)amino)be nzamide (350 mg), triethylamine (699 mg) and 1,2-dichloroethane (20 mL) were added into a 100 mL single-neck flask. The reaction system was heated to 80°C to react for 5.5 hours. After cooling to room temperature, the system was poured into a water/tetrahydrofuran mixed solvent, and extracted with ethyl acetate. Organic phases were combined, dried and concentrated, and purified by silica gel column chromatography. The crude product was washed with a hexane/acetone mixed solvent (4/1, v/v) to obtain 110 mg of off-white solids. ¹H NMR (400 MHz, DMSO-*d*₆): δ 10.40 (s, 1H), 9.05 (s, 1H), 8.89 (t, *J =* 5.6 Hz, 1H), 8.03 (d, *J* = 5.2 Hz, 1H), 7.60-7.56 (m, 2H), 7.36 (d, *J =* 8.4 Hz, 1H), 7.17 (t, *J* = 5.6 Hz, 1H), 6.93 (t, *J* = 4.8 Hz, 1H), 6.72-6.66 (m, 1H), 5.87-5.77 (m, 1H), 5.17-5.06 (m, 2H), 4.07 (s, 2H), 3.84 (t, *J =* 5.2 Hz, 2H), 2.96-2.90 (m, 2H), 1.02 (t, *J =* 7.2 Hz, 3H). ESI-MS: [M + H]⁺ = 664.2.

### Example 9

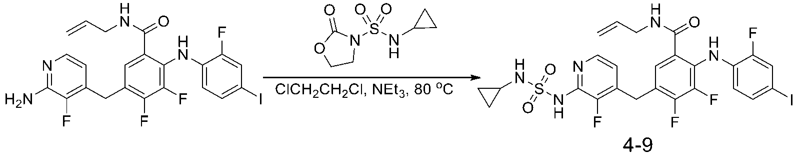

### Preparation of N-allyl-5-((2-((N-cyclopropylaminosulfonyl)amino)-3-fluoropyridin-4-yl)methyl)-3,4-difluoro-2-(( 2-fluoro-4-iodophenyl)amino)benzamide

N-cyclopropyl-2-oxooxazoline-3-sulfonamide (1.5 g), N-allyl-5-((2-amino-3-fluoropyridin-4-yl)methyl)-3,4-difluoro-2-((2-fluoro-4-iodophenyl)amino)be nzamide (400 mg), triethylamine (729 mg) and 1,2-dichloroethane (20 mL) were added into a 100 mL single-neck flask. The reaction system was heated to 80°C to react for 5.5 hours. After cooling to room temperature, the system was poured into a water/tetrahydrofuran mixed solvent, and extracted with ethyl acetate. Organic phases were combined, dried and concentrated, and purified by silica gel column chromatography. The crude product was washed with an n-hexane/acetone mixed solvent to obtain 150 mg of off-white solids. ¹H NMR (400 MHz, DMSO-*d*₆): δ 10.54 (s, 1H), 9.05 (s, 1H), 8.89 (t, *J* = 5.6 Hz, 1H), 8.03 (d, *J* = 5.2 Hz, 1H), 7.61-7.56 (m, 3H), 7.36 (d, *J=* 8.4 Hz, 1H), 6.95 (t, *J =* 4.8 Hz, 1H), 6.72-6.66 (m, 1H), 5.87-5.77 (m, 1H), 5.17-5.06 (m, 2H), 4.08 (s, 2H), 3.84 (t, *J =* 5.2 Hz, 2H), 2.32-2.30 (m, 1H), 0.56-0.47 (m, 4H). ESI-MS: [M + H]⁺ = 676.1.

### Example 10

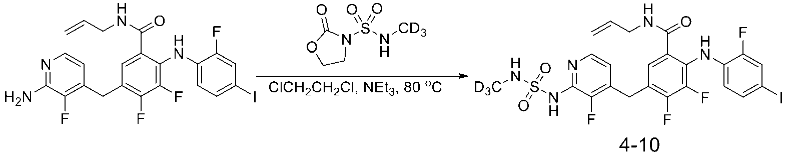

### Preparation of N-allyl-3,4-difluoro-5-((3-fluoro-2-((N-methyl-d₃-aminosulfonyl)amino)pyridin-4-yl)methyl)-2-((2-fluoro-4-iodophenyl)amino)benzamide

N-methyl-*d*₃-2-oxooxazoline-3-sulfonamide (1.0 g), N-allyl-5-((2-amino-3-fluoropyridin-4-yl)methyl)-3,4-difluoro-2-((2-fluoro-4-iodophenyl)amino)be nzamide (310 mg), triethylamine (621 mg) and 1,2-dichloroethane (30 mL) were added into a 100 mL single-neck flask. The reaction system was heated to 80°C to react for 5.5 hours. After cooling to room temperature, the system was poured into a water/tetrahydrofuran mixed solvent, and extracted with ethyl acetate. Organic phases were combined, dried and concentrated, and purified by silica gel column chromatography. The crude product was washed with an n-hexane/acetone mixed solvent to obtain 110 mg of off-white solids. ¹H NMR (400 MHz, DMSO-*d*₆): δ 10.41 (s, 1H), 9.05 (s, 1H), 8.89 (t, *J* = 5.6 Hz, 1H), 8.02 (d, *J* = 5.2 Hz, 1H), 7.60-7.57 (m, 2H), 7.36 (d, *J* = 8.4 Hz, 1H), 7.00 (s, 1H), 6.94 (t, *J =* 4.8 Hz, 1H), 6.72-6.66 (m, 1H), 5.87-5.77 (m, 1H), 5.17-5.06 (m, 2H), 4.07 (s, 2H), 3.84 (t, *J =* 5.2 Hz, 2H). ESI-MS: [M + H]⁺ = 653.2.

### Example 11

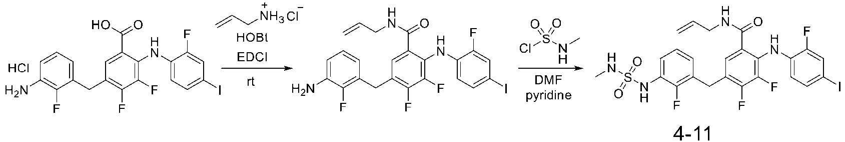

### Step 1: preparation of 5-((3-amino-2-fluorobenzyl)-3,4-difluoro-2-((2-fluoro-4-iodophenyl)amino)-N-allylbenzamide

5-(3-amino-2-fluorobenzyl)-3,4-difluoro-2-((2-fluoro-4-iodophenyl)amino)benzoic acid hydrochloride (310 mg), 1-hydroxybenzotriazole (170 mg), 1-(3-dimethylaminopropyl)-3-ethylcarbodiimide hydrochloride (322 mg) and N,N-dimethylformamide (6 mL) were added into a 100 mL single-neck flask, and the system was stirred at room temperature to react for 2 hours. Subsequently, allylamine hydrochloride (93 mg) and N,N-diisopropylethylamine (217 mg) were added to the system, and the mixture was continuously stirred at room temperature to react for 2 hours. The reaction system was added into a saturated sodium bicarbonate aqueous solution to precipitate solids, and subjected to suction filtration. The filter cake was purified by silica gel column chromatography to obtain 220 mg of off-white solids. ESI-MS: [M + H]⁺ = 556.0.

### Step 2: preparation of N-allyl-3,4-difluoro-5-(2-fluoro-3-((N-methylaminosulfonyl)amino)benzyl)-2-((2-fluoro-4-iodophe nyl)amino)benzamide

5-((3-amino-2-fluorobenzyl)-3,4-difluoro-2-((2-fluoro-4-iodophenyl)amino)-N-allylbenzamide (274 mg), N,N-dimethylformamide (10 mL) and pyridine (391 mg) were added into a 50 mL single-neck flask, and the system was placed in an ice bath and stirred. After cooling to below 0°C, methylaminosulfonyl chloride (192 mg) was slowly dropwise added to the system. After 30 minutes, the reaction was stopped, and the system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 180 mg of off-white solids. ¹H NMR (400 MHz, DMSO-*d*₆): δ 9.43 (s, 1H), 8.99 (s, 1H), 8.86 (t, *J* = 5.6 Hz, 1H), 7.58 (dd, *J*₁ = 10.8 Hz, *J*₂ = 2.0 Hz, 1H), 7.54 (d, *J* = 7.2 Hz, 1H), 7.37-7.31 (m, 2H), 7.25 (q, *J* = 4.8 Hz, 1H), 7.11 (t, *J* = 8.0 Hz, 1H), 7.02 (t, *J* = 6.8 Hz, 1H), 6.68-6.63 (m, 1H), 5.86-5.76 (m, 1H), 5.16-5.05 (m, 2H), 4.03 (s, 2H), 3.83 (t, *J=* 5.4 Hz, 2H), 2.52 (d, *J* = 4.8 Hz, 3H). ESI-MS: [M + Na]⁺ = 671.0.

### Example 12

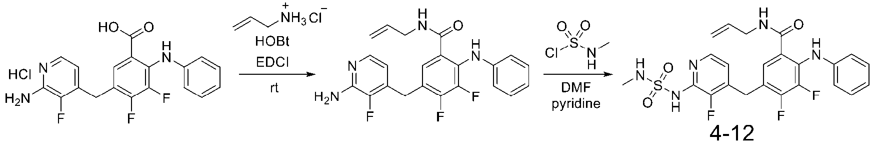

### Step 1: preparation of N-allyl-5-((2-amino-3-fluoropyridin-4-yl)methyl)-3,4-difluoro-2-(phenylamino)benzamide

5-((2-amino-3-fluoropyridin-4-yl)methyl)-3,4-difluoro-2-(phenylamino)benzoic acid hydrochloride (350 mg), 1-hydroxybenzotriazole (220 mg), 1-(3-dimethylaminopropyl)-3-ethylcarbodiimide hydrochloride (606 mg) and N,N-dimethylformamide (10 mL) were added into a 100 mL single-neck flask, and the system was stirred at room temperature to react for 2 hours. Subsequently, allylamine hydrochloride (160 mg) and N,N-diisopropylethylamine (331 mg) were added to the system, and continuously stirred at room temperature to react for 2 hours. The reaction system was added into a saturated sodium bicarbonate aqueous solution to precipitate solids, and subjected to suction filtration. The filter cake was purified by silica gel column chromatography to obtain 250 mg of light yellow solids.

### Step 2: preparation of N-allyl-3,4-difluoro-5-((3-fluoro-2-((N-methylaminosulfonyl)amino)pyridin-4-yl)methyl)-2-(pheny lamino)benzamide

N-allyl-5-((2-amino-3-fluoropyridin-4-yl)methyl)-3,4-difluoro-2-(phenylamino)benzamide (250 mg), N,N-dimethylformamide (10 mL) and pyridine (480 mg) were added into a 50 mL single-neck flask, and the system was placed in an ice bath and stirred. After cooling to below 0°C, methylaminosulfonyl chloride (236 mg) was slowly dropwise added to the system. After finishing the addition, the reaction was continued for 30 minutes and then stopped. The system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 165 mg of off-white solids. ¹H NMR (400 MHz, DMSO-*d*₆): δ 10.42 (s, 1H), 8.83 (s, 1H), 8.78 (t, *J =* 5.6 Hz, 1H), 8.02 (d, *J* = 4.8 Hz, 1H), 7.52 (d, *J =* 6.8 Hz, 1H), 7.22-7.18 (m, 2H), 7.03 (d, *J* = 4.8 Hz, 1H), 6.96-6.82 (m, 4H), 5.83-5.74 (m, 1H), 5.14-5.03 (m, 2H), 4.07 (s, 2H), 3.82-3.80 (m, 2H), 2.52-2.51 (m, 3H). ESI-MS: [M + H]⁺ = 506.2.

### Example 13

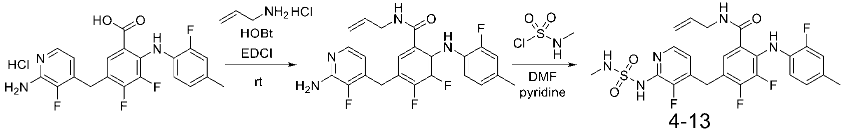

### Step 1 : preparation of N-allyl-5-((2-amino-3-fluoropyridin-4-yl)methyl)-3,4-difluoro-2-((2-fluoro-4-methylphenyl)amino) benzamide

5-((2-amino-3-fluoropyridin-4-yl)methyl)-3,4-difluoro-2-((2-fluoro-4-methylphenyl)amino)be nzoic acid hydrochloride (500 mg), 1-hydroxybenzotriazole (344 mg), 1-(3-dimethylaminopropyl)-3-ethylcarbodiimide hydrochloride (433 mg) and N,N-dimethylformamide (5 mL) were added into a 100 mL single-neck flask, and the system was stirred at room temperature to react for 2 hours. Subsequently, allylamine hydrochloride (317 mg) and N,N-diisopropylethylamine (875 mg) were added to the system, and the mixture was continuously stirred at room temperature to react for 2 hours. The reaction system was added into a saturated sodium bicarbonate aqueous solution to precipitate solids, and subjected to suction filtration. The filter cake was purified by silica gel column chromatography to obtain 350 mg of light yellow solids. ESI-MS: [M + H]⁺ = 445.3.

### Step 2: preparation of N-allyl-3,4-difluoro-5-((3-fluoro-2-((N-methylaminosulfonyl)amino)pyridin-4-yl)methyl)-2-((2-flu oro-4-methylphenyl)amino)benzamide

N-allyl-5-((2-amino-3-fluoropyridin-4-yl)methyl)-3,4-difluoro-2-((2-fluoro-4-methylphenyl)a mino)benzamide (350 mg), N,N-dimethylformamide (5 mL) and pyridine (617 mg) were added into a 50 mL single-neck flask, and the system was placed in an ice bath and stirred. After cooling to below 0°C, methylaminosulfonyl chloride (307 mg) was slowly dropwise added to the system. After 30 minutes, the reaction was stopped, and the system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 180 mg of off-white solids. ¹H NMR (400 MHz, DMSO-*d*₆): δ 10.41 (s, 1H), 9.08 (s, 1H), 8.90 (t, *J =* 5.6 Hz, 1H), 8.02 (d, *J =* 4.8 Hz, 1H), 7.58 (d, *J =* 6.8 Hz, 1H), 7.05-7.02 (m, 2H), 6.93 (t, *J =* 4.8 Hz, 1H), 6.88-6.79 (m, 2H), 5.88-5.80 (m, 1H), 5.18-5.07 (m, 2H), 4.05 (s, 2H), 3.85 (t, *J =* 5.2 Hz, 2H), 2.52-2.51 (m, 3H), 2.25 (s, 3H). ESI-MS: [M + H]⁺ = 538.3.

### Example 14

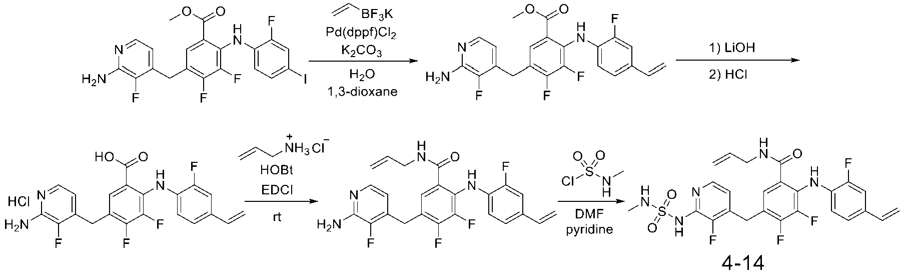

### Step 1: preparation of methyl 5-((2-amino-3-fluoropyridin-4-yl)methyl)-3,4-difluoro-2-((2-fluoro-4-vinylphenyl)amino)benzoate

Methyl 5-((2-amino-3-fluoropyridin-4-yl)methyl)-3,4-difluoro-2-((2-fluoro-4-iodophenyl)amino)benzoate (1.3 g), potassium vinyltrifluoroborate (636 mg), potassium carbonate (984 mg), 1,4-dioxane (12 mL), water (4 mL) and [1,1'-bis(diphenylphosphino)ferrocene]dichloropalladium (86 mg) were added into a 50 mL single-neck flask, and the system was subjected to nitrogen purging, and heated to 95°C and stirred to react for 2 hours. After cooling to room temperature, the system was poured into water for quenching, and extracted with ethyl acetate. Organic phases were combined, dried with anhydrous sodium sulfate, filtered and subjected to rotary evaporation to remove the solvent, so as to obtain 950 mg of brownish-yellow solids. ESI-MS: [M + H]⁺ = 432.2.

### Step 2: preparation of 5-((2-amino-3-fluoropyridin-4-yl)methyl)-3,4-difluoro-2-((2-fluoro-4-vinylphenyl)amino)benzoic acid hydrochloride

Methyl 5-((2-amino-3-fluoropyridin-4-yl)methyl)-3,4-difluoro-2-((2-fluoro-4-vinylphenyl)amino)benzoate (950 mg), tetrahydrofuran (20 mL) and water (10 mL) were added into a 50 mL single-neck flask, and the system was stirred at room temperature until a clear solution was obtained. Subsequently, the system was placed in an ice bath, and lithium hydroxide monohydrate (360 mg) was slowly added. After finishing the addition, the system was heated to room temperature and stirred to react for 3 hours. Subsequently, hydrochloric acid (6 M, 5 mL) was slowly dropwise added to the system to adjust a pH value of the system to 1-2, and stirred for 5 minutes. The tetrahydrofuran in the system was removed under reduced pressure. Subsequently, water was added to the system to dilute the concentrate and the mixture was stirred for 10 minutes, and filtered. A filter cake was collected, and washed once with water and once with methyl tert-butyl ether respectively to obtain 700 mg of brown solids. The product in this step was directly used in the next step without further characterization and purification.

### Step 3: preparation of N-allyl-5-((2-amino-3-fluoropyridin-4-yl)methyl)-3,4-difluoro-2-((2-fluoro-4-vinylphenyl)amino)b enzamide

5-((2-amino-3-fluoropyridin-4-yl)methyl)-3,4-difluoro-2-((2-fluoro-4-vinylphenyl)amino)benz oic acid hydrochloride (300 mg), 1-hydroxybenzotriazole (107 mg), 1-(3-dimethylaminopropyl)-3-ethylcarbodiimide hydrochloride (253 mg) and N,N-dimethylformamide (5 mL) were added into a 100 mL single-neck flask, and the system was stirred at room temperature to react for 2 hours. Subsequently, allylamine hydrochloride (126 mg) and N,N-diisopropylethylamine (426 mg) were added to the system, and the mixture was continuously stirred at room temperature to react for 2 hours. The reaction system was added into a saturated sodium bicarbonate aqueous solution to precipitate solids, and subjected to suction filtration. The filter cake was purified by silica gel column chromatography to obtain 150 mg of light yellow solids. ESI-MS: [M + H]⁺ =457.3.

### Step 4: preparation of N-allyl-3,4-difluoro-5-((3-fluoro-2-((N-methylaminosulfonyl)amino)pyridin-4-yl)methyl)-2-((2-flu oro-4-vinylphenyl)amino)benzamide

N-allyl-5-((2-amino-3-fluoropyridin-4-yl)methyl)-3,4-difluoro-2-((2-fluoro-4-vinylphenyl)ami no)benzamide (146 mg), N,N-dimethylformamide (5 mL) and pyridine (250 mg) were added into a 50 mL single-neck flask, and the system was placed in an ice bath and stirred. After cooling to below 0°C, methylaminosulfonyl chloride (240 mg) was slowly dropwise added to the system. After 30 minutes, the reaction was stopped, and the system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 70 mg of off-white solids. ¹H NMR (400 MHz, DMSO-*d*₆): δ 10.42 (s, 1H), 9.13 (s, 1H), 8.91 (t, *J =* 5.6 Hz, 1H), 8.03 (d, *J =* 4.8 Hz, 1H), 7.59 (d, *J =* 6.8 Hz, 1H), 7.39 (dd, *J*₁ = 12.8 Hz, *J₂* = 1.2 Hz, 1H), 7.13 (d, *J =* 8.0 Hz, 1H), 7.03 (q, *J =* 5.2 Hz, 1H), 6.95 (t, *J* = 4.8 Hz, 1H), 6.87-6.82 (m, 1H), 6.64 (dd, *J*₁ = 17.6 Hz, *J*₂ = 10.8 Hz, 1H), 5.88-5.79 (m, 1H), 5.74 (d, *J =* 17.6 Hz, 1H), 5.19-5.13 (m, 2H), 5.07 (dd, *J*₁ = 10.4 Hz, *J*₂ = 5.6 Hz, 1H), 4.08 (s, 2H), 3.85 (t, *J =* 5.2 Hz, 2H), 2.53-2.51 (m, 3H). ESI-MS: [M + H]⁺ = 550.3.

### Example 15

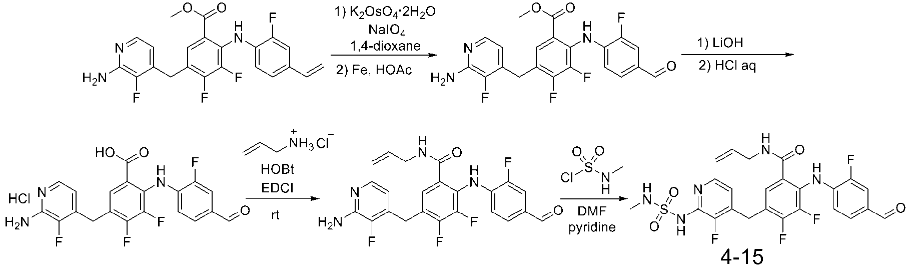

### Step 1: preparation of methyl 5-((2-amino-3-fluoropyridin-4-yl)methyl)-3,4-difluoro-2-((2-fluoro-4-formylphenyl)amino)benzoat e

Methyl 5-((2-amino-3-fluoropyridin-4-yl)methyl)-3,4-difluoro-2-((2-fluoro-4-vinylphenyl)amino)benzoate (1.1 g), potassium osmate dihydrate (91 mg), sodium periodate (2.7 g), 1,4-dioxane (30 mL) and water (12 mL) were added into a 100 mL single-neck flask, and the system was stirred at room temperature to react for 2 hours. The reaction system was poured into water, and extracted with ethyl acetate. Organic phases were combined, dried with anhydrous sodium sulfate, and filtered and subjected to rotary evaporation to remove the solvent, so as to obtain 950 mg of solids. The solids were diluted with acetic acid (20 mL). Subsequently, iron powder (9.0 g) was added to the system, and the mixture was stirred at room temperature to react for 1 hour. 50 mL of ethyl acetate was added to the system, and the mixture was subjected to suction filtration to remove insoluble substances. A filtrate was collected and concentrated. The concentrate was diluted with water, then sodium bicarbonate aqueous solution was added to adjust a pH value of the system to 7-8, and extracted with ethyl acetate. Organic phases were combined, dried with anhydrous sodium sulfate, filtered and subjected to rotary evaporation to remove the solvent, so as to obtain a crude product. The crude product was purified by silica gel column chromatography to obtain 450 mg of yellow solids. ESI-MS: [M + H]⁺ = 434.1.

### Step 2: preparation of 5-((2-amino-3-fluoropyridin-4-yl)methyl)-3,4-difluoro-2-((2-fluoro-4-formylphenyl)amino)benzoic acid hydrochloride

Methyl 5-((2-amino-3-fluoropyridin-4-yl)methyl)-3,4-difluoro-2-((2-fluoro-4-formylphenyl)amino)benzoat e (450 mg), tetrahydrofuran (10 mL) and water (5 mL) were added into a 50 mL single-neck flask, and the system was stirred at room temperature until a clear solution was obtained. Subsequently, the system was placed in an ice bath, and lithium hydroxide monohydrate (131 mg) was slowly added to the system. After finishing the addition, the system was heated to room temperature and stirred to react for 2 hours. Subsequently, hydrochloric acid (6 M, 3 mL) was slowly dropwise added to adjust a pH value to 1-2, the mixture was stirred for 5 minutes. The tetrahydrofuran in the system was removed under reduced pressure. Subsequently, the concentrate was diluted with water and stirred for 10 minutes, and filtered. A filter cake was collected, and washed once with water and methyl tert-butyl ether respectively to obtain 390 mg of light yellow solids. The product in this step was directly used in the next step of reaction without further purification and characterization.

### Step 3: preparation of N-allyl-5-((2-amino-3-fluoropyridin-4-yl)methyl)-3,4-difluoro-2-((2-fluoro-4-formylphenyl)amino) benzamide

5-((2-amino-3-fluoropyridin-4-yl)methyl)-3,4-difluoro-2-((2-fluoro-4-formylphenyl)amino)be nzoic acid hydrochloride (440 mg), 1-hydroxybenzotriazole (248 mg), 1-(3-dimethylaminopropyl)-3-ethylcarbodiimide hydrochloride (686 mg) and N,N-dimethylformamide (15 mL) were added into a 100 mL single-neck flask, and the system was stirred at room temperature to react for 2 hours. Subsequently, allylamine hydrochloride (90 mg) and N,N-diisopropylethylamine (374 g) were added to the system, and the mixture was continuously stirred at room temperature to react for 2 hours. The reaction system was added into a saturated sodium bicarbonate aqueous solution to precipitate solids, and subjected to suction filtration. The filter cake was purified by silica gel column chromatography to obtain 290 mg of off-white solids. ESI-MS: [M + H]⁺ = 459.2.

### Step 4: preparation of N-allyl-3,4-difluoro-5-((3-fluoro-2-((N-methylaminosulfonyl)amino)pyridin-4-yl)methyl)-2-((2-flu oro-4-formylphenyl)amino)benzamide

N-allyl-5-((2-amino-3-fluoropyridin-4-yl)methyl)-3,4-difluoro-2-((2-fluoro-4-formylphenyl)a mino)benzamide (280 mg), N,N-dimethylformamide (10 mL) and pyridine (483 mg) were added into a 50 mL single-neck flask, and the system was placed in an ice bath and stirred. After cooling to below 0°C, methylaminosulfonyl chloride (238 mg) was added slowly dropwise to the system. After 30 minutes, the reaction was stopped, and the system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 270 mg of white solids. ¹H NMR (400 MHz, DMSO-*d*₆): δ 10.44 (s, 1H), 9.79 (d, *J =* 1.6 Hz, 1H), 9.22 (s, 1H), 8.85 (t, *J =* 5.6 Hz, 1H), 8.04 (d, *J =* 5.2 Hz, 1H), 7.67 (dd, *J*₁ = 12.0 Hz, *J*₂ = 1.6 Hz, 1H), 7.61 (dd, *J*₁ = 8.4 Hz, *J*₂ *=* 1.2 Hz, 1H), 7.57 (d, *J =* 6.8 Hz, 1H), 7.06-7.03 (m, 1H), 6.98 (t, *J =* 4.8 Hz, 1H), 6.95-6.89 (m, 1H), 5.84-5.75 (m, 1H), 5.16-5.03 (m, 2H), 4.13 (s, 2H), 3.82 (t, *J =* 5.2 Hz, 2H), 2.53-2.51 (m, 3H). ESI-MS: [M + H]⁺ = 552.1.

### Example 16

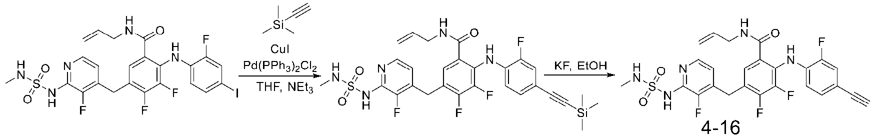

### Step 1: preparation of N-allyl-3,4-difluoro-5-((3-fluoro-2-((N-methylaminosulfonyl)amino)pyridin-4-yl)methyl)-2-((2-flu oro-4-((trimethylsilyl)ethynyl)phenyl)amino)benzamide

N-allyl-3,4-difluoro-5-((3-fluoro-2-((N-methylaminosulfonyl)amino)pyridin-4-yl)methyl)-2-(( 2-fluoro-4-iodophenyl)amino)benzamide (140 mg), trimethylsilylacetylene (85 mg), cuprous iodide (1 mg), bis(triphenylphosphine)palladium dichloride (3 mg), tetrahydrofuran (20 mL) and triethylamine (0.5 mL) were added into a 100 mL single-neck flask. Subsequently, the system was subjected to nitrogen purging, stirred at room temperature to react for 16 hours, filtered, concentrated, and purified by silica gel column chromatography to obtain 120 mg of yellow solids. ESI-MS: [M + H]⁺ = 620.4.

### Step 2: preparation of N-allyl-2-((4-ethynyl-2-fluorophenyl)amino)-3,4-difluoro-5-((3-fluoro-2-((N-methylaminosulfonyl) amino)pyridin-4-yl)methyl)benzamide

N-allyl-3,4-difluoro-5-((3-fluoro-2-((N-methylaminosulfonyl)amino)pyridin-4-yl)methyl)-2-(( 2-fluoro-4-((trimethylsilyl)ethynyl)phenyl)amino)benzamide (120 mg), ethanol (5 mL) and potassium fluoride (50 mg) were added into a 50 mL single-neck flask, and the system was stirred at room temperature to react for 21 hours. Water was added to the system and the mixture was stirred at room temperature for 10 minutes, and extracted with ethyl acetate. Organic phases were combined, dried with anhydrous sodium sulfate, filtered and subjected to rotary evaporation to remove the solvent, and purified by silica gel column chromatography to obtain 50 mg of light yellow solids. ¹H NMR (400 MHz, DMSO-*d*₆): δ 10.42 (s, 1H), 9.12 (s, 1H), 8.89 (t, *J =* 5.6 Hz, 1H), 8.01 (d, *J =* 3.6 Hz, 1H), 7.58 (d, *J =* 6.8 Hz, 1H), 7.34 (d, *J =* 12.0 Hz, 1H), 7.15 (d, *J =* 8.4 Hz, 1H), 7.02 (brs, 1H), 6.94 (brs, 1H), 6.85-6.79 (m, 1H), 5.87-5.77 (m, 1H), 5.17-5.06 (m, 2H), 4.13 (s, 1H), 4.09 (s, 2H), 3.84 (t, *J =* 5.2 Hz, 2H), 2.52-2.51 (m, 3H). ESI-MS: [M + H]⁺ = 548.3.

### Example 17

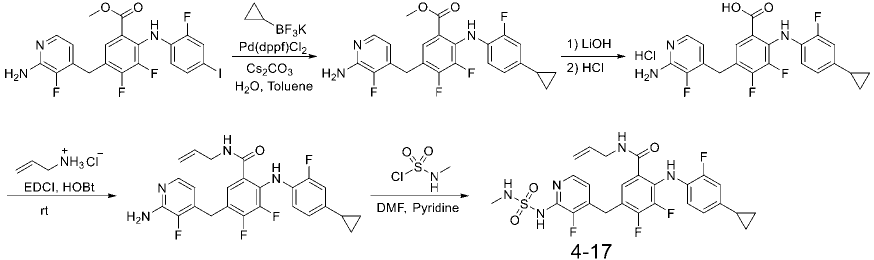

### Step 1: preparation of methyl 5-((2-amino-3-fluoropyridin-4-yl)methyl)-2-((4-cyclopropyl-2-fluorophenyl)amino)-3,4-difluorobe nzoate

Methyl 5-((2-amino-3-fluoropyridin-4-yl)methyl)-3,4-difluoro-2-((2-fluoro-4-iodophenyl)amino)benzoate (1.9 g), potassium cyclopropyltrifluoroborate (799 mg), cesium carbonate (3.5 g), toluene (38 mL), water (4 mL) and [1,1'-bis(diphenylphosphino)ferrocene]dichloropalladium (294 mg) were added into a 100 mL single-neck flask, and subjected to nitrogen purging. The system was heated to 100°C and stirred to react for 23 hours. The reaction system was cooled to room temperature, ethyl acetate was added, and the mixture was washed with saturated brine. Organic phases were collected, dried with anhydrous sodium sulfate, filtered and subjected to rotary evaporation to remove the solvent. The concentrate was purified by silica gel column chromatography to obtain 1.0 g of yellow solids. ESI-MS: [M + H]⁺ = 446.2.

### Step 2: preparation of 5-((2-amino-3-fluoropyridin-4-yl)methyl)-2-((4-cyclopropyl-2-fluorophenyl)amino)-3,4-difluorobe nzoic acid hydrochloride

Methyl 5-((2-amino-3-fluoropyridin-4-yl)methyl)-2-((4-cyclopropyl-2-fluorophenyl)amino)-3,4-difluorobe nzoate (1.0 g), tetrahydrofuran (14 mL) and water (7 mL) were added into a 50 mL single-neck flask, and the system was stirred at room temperature until a clear solution was obtained. Subsequently, the system was placed in an ice bath, and lithium hydroxide monohydrate (283 mg) was slowly added to the system. After finishing the addition, the system was heated to room temperature and stirred to react for 19 hours. Hydrochloric acid (6 M, 6 mL) was slowly dropwise added to the system to adjust a pH value to 1-2 and the mixture was stirred for 5 minutes, and concentrated under reduced pressure to remove the tetrahydrofuran in the system. Subsequently, the concentrate was diluted with water and stirred for 10 minutes, and filtered. A filter cake was collected, and washed with water and methyl tert-butyl ether respectively to obtain 1.0 g of off-white solids. ESI-MS: [M + H]⁺ = 432.2.

### Step 3: preparation of N-allyl-5-((2-amino-3-fluoropyridin-4-yl)methyl)-2-((4-cyclopropyl-2-fluorophenyl)amino)-3,4-difl uorobenzamide

5-((2-amino-3-fluoropyridin-4-yl)methyl)-2-((4-cyclopropyl-2-fluorophenyl)amino)-3,4-difluo robenzoic acid hydrochloride (320 mg), 1-hydroxybenzotriazole (110 mg), 1-(3-dimethylaminopropyl)-3-ethylcarbodiimide hydrochloride (261 mg) and N,N-dimethylformamide (5 mL) were added into a 100 mL single-neck flask, and the system was stirred at room temperature to react for 2 hours. Subsequently, allylamine hydrochloride (127 mg) and N,N-diisopropylethylamine (439 mg) were added to the system, and the mixture was continuously stirred at room temperature to react for 2 hours. The reaction system was added into a saturated sodium bicarbonate aqueous solution to precipitate solids, and subjected to suction filtration. The filter cake was purified by silica gel column chromatography to obtain 250 mg of off-white solids. ESI-MS: [M + H]⁺ = 471.2.

### Step 4: preparation of N-allyl-2-((4-cyclopropyl-2-fluorophenyl)amino)-3,4-difluoro-5-((3-fluoro-2-((N-methylaminosulf onyl)amino)pyridin-4-yl)methyl)benzamide

N-allyl-5-((2-amino-3-fluoropyridin-4-yl)methyl)-2-((4-cyclopropyl-2-fluorophenyl)amino)-3, 4-difluorobenzamide (250 mg), N,N-dimethylformamide (10 mL) and pyridine (414 mg) were added into a 50 mL single-neck flask, and the system was placed in an ice bath and stirred. After cooling to 0°C or a lower temperature, methylaminosulfonyl chloride (206 mg) was slowly dropwise added to the system. After 30 minutes, the reaction was stopped, and the system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 200 mg of off-white solids. ¹H NMR (400 MHz, DMSO-*d*₆): δ 10.41 (s, 1H), 9.07 (s, 1H), 8.90 (t, *J =* 5.6 Hz, 1H), 8.02 (d, *J* = 4.8 Hz, 1H), 7.58 (d, *J =* 6.8 Hz, 1H), 7.03 (q, *J =* 5.2 Hz, 1H), 6.94-6.89 (m, 2H), 6.81-6.80 (m, 2H), 5.89-5.79 (m, 1H), 5.18-5.07 (m, 2H), 4.05 (s, 2H), 3.85 (t, *J =* 5.6 Hz, 2H), 2.52-2.51 (m, 3H), 1.91-1.84 (m, 1H), 0.93-0.88 (m, 2H), 0.64-0.60 (m, 2H). ESI-MS: [M + Na]⁺ = 586.2.

### Example 18

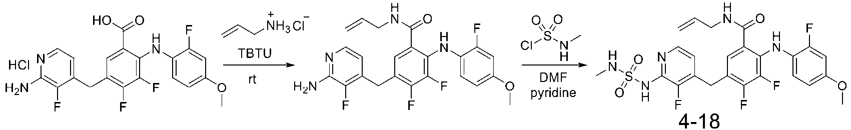

### Step 1: preparation of N-allyl-5-((2-amino-3-fluoropyridin-4-yl)methyl)-3,4-difluoro-2-((2-fluoro-4-methoxyphenyl)amin o)benzamide

5-((2-amino-3-fluoropyridin-4-yl)methyl)-3,4-difluoro-2-((2-fluoro-4-methoxyphenyl)amino)b enzoic acid hydrochloride (300 mg), allylamine hydrochloride (67 mg), N,N-diisopropylethylamine (366 mg), N,N-dimethylformamide (6 mL) were added into a 100 mL single-neck flask. Subsequently, the system was subjected to nitrogen purging, O-benzotriazole-N,N,N',N'-tetramethylurea tetrafluoroboric acid (228 mg in total) was added in batches, and the mixture was stirred at room temperature to react for 3 hours. Saturated brine was added to the reaction solution, and the system was extracted with ethyl acetate. Organic phases were combined, washed with a citric acid aqueous solution and saturated brine respectively, dried and concentrated to obtain a crude product. The crude product was purified by silica gel column chromatography to obtain 230 mg of yellow solids. ESI-MS: [M + H]⁺ = 461.2.

### Step 2: preparation of N-allyl-3,4-difluoro-5-((3-fluoro-2-((N-methylaminosulfonyl)amino)pyridin-4-yl)methyl)-2-((2-flu oro-4-methoxyphenyl)amino)benzamide

N-allyl-5-((2-amino-3-fluoropyridin-4-yl)methyl)-3,4-difluoro-2-((2-fluoro-4-methoxyphenyl) amino)benzamide (200 mg), N,N-dimethylformamide (6 mL) and pyridine (275 mg) were added into a 50 mL single-neck flask, and the system was cooled to -16°C to -20°C in a nitrogen atmosphere, and methylaminosulfonyl chloride (334 mg) was slowly dropwise added to react for 2 hours. Water was added to the system and the mixture was stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 190 mg of light yellow solids. ¹H NMR (400 MHz, DMSO-*d*₆): δ 10.41 (s, 1H), 9.10 (s, 1H), 8.89 (t, *J =* 5.6 Hz, 1H), 8.01 (d, *J =* 4.8 Hz, 1H), 7.57 (d, *J* = 6.8 Hz, 1H), 7.03 (brs, 1H), 7.0-6.86 (m, 3H), 6.68-6.66 (m, 1H), 5.90-5.81 (m, 1H), 5.20-5.07 (m 2H), 4.03 (s, 2H), 3.87-3.84 (m, 2H), 3.73 (s, 3H), 2.52-2.51 (m, 3H). ESI-MS: [M + H]⁺ = 554.2.

### Example 19

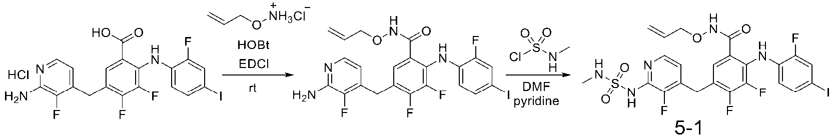

### Step 1: preparation of N-(allyloxy)-5-((2-amino-3-fluoropyridin-4-yl)methyl)-3,4-difluoro-2-((2-fluoro-4-iodophenyl)ami no)benzamide

5-((2-amino-3-fluoropyridin-4-yl)methyl)-3,4-difluoro-2-((2-fluoro-4-iodophenyl)amino)benz oic acid hydrochloride (400 mg), 1-hydroxybenzotriazole (195 mg), 1-(3-dimethylaminopropyl)-3-ethylcarbodiimide hydrochloride (552 mg) and N,N-dimethylformamide (5 mL) were added into a 100 mL single-neck flask, and the system was stirred at room temperature to react for 2 hours. Subsequently, allylhydroxylamine hydrochloride (158 mg) and N,N-diisopropylethylamine (464 mg) were added to the system, and the mixture was continuously stirred at room temperature to react for 2 hours. The reaction system was added into a saturated sodium bicarbonate aqueous solution to precipitate solids, and subjected to suction filtration. The filter cake was purified by silica gel column chromatography to obtain 200 mg of off-white solids. ESI-MS: [M + H]⁺ = 573.1.

### Step 2: preparation of N-(allyloxy)-3,4-difluoro-5-((3-fluoro-2-((N-methylaminosulfonyl)amino)pyridin-4-yl)methyl)-2-(( 2-fluoro-4-iodophenyl)amino)benzamide

*N*-(allyloxy)-5-((2-amino-3-fluoropyridin-4-yl)methyl)-3,4-difluoro-2-((2-fluoro-4-iodophenyl )amino)benzamide (200 mg), N,N-dimethylformamide (3 mL) and pyridine (278 mg) were added into a 50 mL single-neck flask, and the system was placed in an ice bath and stirred. After cooling to 0°C or a lower temperature, methylaminosulfonyl chloride (208 mg) was slowly dropwise added. After 30 minutes, the reaction was stopped, and the system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 100 mg of off-white solids. ¹H NMR (400 MHz, DMSO-*d*₆): δ 11.76 (s, 1H), 10.41 (s, 1H), 8.49 (s, 1H), 8.02 (d, *J* = 2.8 Hz, 1H), 7.57 (d, *J* = 10.8 Hz, 1H), 7.36-7.34 (m, 2H), 7.03-7.02 (m, 1H), 6.94 (s, 1H), 6.68-6.62 (m, 1H), 5.95-5.87 (m, 1H), 5.30-5.19 (m, 2H), 4.30 (s, 2H), 4.07 (s, 2H), 2.51-2.50 (m, 3H). ESI-MS: [M + H]⁺ = 666.0.

### Example 20

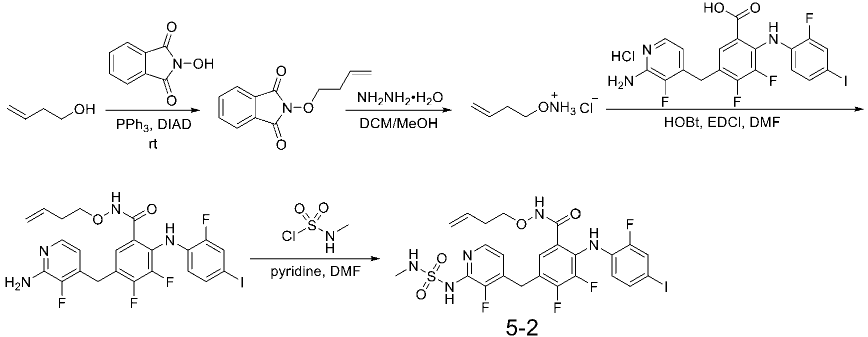

### Step 1: preparation of 2-(but-3-en-1-yloxy)isoindoline-1,3-dione

But-3-en-1-ol (5.0 g), triphenylphosphine (23.7 g), N-hydroxyphthalimide (12.5 g) and tetrahydrofuran (250 mL) were added into a 500 mL three-neck flask, and the system was subjected to nitrogen purging. The system was cooled to 0°C or a lower temperature, and tetrahydrofuran solution (18.2 g, 100 mL tetrahydrofuran) of diisopropyl azodicarboxylate was dropwise added. After finishing the addition, the system was taken out of the cooling bath, and stirred at room temperature to react for 16 hours. The reaction system was concentrated, then added into saturated brine, and extracted with ethyl acetate. Organic phases were combined, dried and concentrated. The concentrate was purified by silica gel column chromatography to obtain 14.0 g of light yellow oily substance.

### Step 2: preparation of O-(but-3-en-1-yl)hydroxylamine hydrochloride

2-(but-3-en-1-yloxy)isoindoline-1,3-dione (7.0 g) and dichloromethane/methanol (2/1, v/v, 168 mL in total) were added into a 250 mL single-neck flask, and the system was subjected to nitrogen purging. Hydrazine hydrate (1.8 g) was slowly dropsie added to the system. After finishing the addition, the system was continuously stirred to react for 3 hours, and subjected to suction filtration. The filter cake was washed with dichloromethane. Organic phases were combined, and concentrated. The concentrate was diluted with methyl tert-butyl ether, and subjected to suction filtration. Ethyl acetate solution of hydrogen chloride was added to the filtrate to adjust a pH value of the filtrate to 1, and concentrated to obtain 1.7 g of off-white solids.

### Step 3: preparation of 5-((2-amino-3-fluoropyridin-4-yl)methyl)-N-(but-3-en-1-yloxy)-3,4-difluoro-2-((2-fluoro-4-iodoph enyl)amino)benzamide

5-((2-amino-3-fluoropyridin-4-yl)methyl)-3,4-difluoro-2-((2-fluoro-4-iodophenyl)amino)benz oic acid hydrochloride (400 mg), 1-hydroxybenzotriazole (147 mg), 1-(3-dimethylaminopropyl)-3-ethylcarbodiimide hydrochloride (416 mg) and N,N-dimethylformamide (10 mL) were added into a 100 mL single-neck flask, and the system was stirred at room temperature to react for 2 hours. Subsequently, O-(but-3-en-1-yl)hydroxylamine hydrochloride (178 mg) and N,N-diisopropylethylamine (280 mg) were added to the system, and the mixture was continuously stirred at room temperature to react for 2 hours. The reaction system was added into saturated brine to precipitate solids, and subjected to suction filtration. The filter cake was purified by silica gel column chromatography to obtain 70 mg of light yellow solids. ESI-MS: [M + H]⁺ = 587.1.

### Step 4: preparation of N-(but-3-en-1-yloxy)-3,4-difluoro-5-((3-fluoro-2-((N-methylaminosulfonyl)amino)pyridin-4-yl)met hyl)-2-((2-fluoro-4-iodophenyl)amino)benzamide

5-((2-amino-3-fluoropyridin-4-yl)methyl)-N-(but-3-en-1-yloxy)-3,4-difluoro-2-((2-fluoro-4-io dophenyl)amino)benzamide (75 mg), N,N-dimethylformamide (8 mL) and pyridine (101 mg) were added into a 50 mL single-neck flask, and the system was placed in an ice bath and stirred. After cooling to 0°C or a lower temperature, methylaminosulfonyl chloride (50 mg) was slowly dropwise added to the system. After 30 minutes, the reaction was stopped, and the system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 50 mg of off-white solids. ¹H NMR (400 MHz, DMSO-*d*₆): δ 11.76 (s, 1H), 10.42 (s, 1H), 8.52 (s, 1H), 8.02 (d, *J* = 4.8 Hz, 1H), 7.57 (dd, *J*₁ = 10.8 Hz, *J*₂ = 1.6 Hz, 1H), 7.38-7.34 (m, 2H), 7.05-7.01 (m, 1H), 6.95 (t, *J =* 4.8 Hz, 1H), 6.68-6.63 (m, 1H), 5.86-5.76 (m, 1H), 5.14-5.02 (m, 2H), 4.08 (s, 2H), 3.81 (t, *J =* 6.8 Hz, 2H), 2.52-2.51 (m, 3H), 2.33-2.28 (m, 2H). ESI-MS: [M + H]⁺ = 680.1.

### Example 21

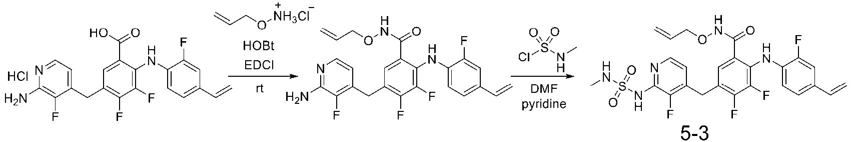

### Step 1: preparation of N-(allyloxy) -5-((2-amino-3-fluoropyridin-4-yl)methyl)-3,4-difluoro-2-((2-fluoro-4-vinylphenyl)amino)benzami de

5-((2-amino-3-fluoropyridin-4-yl)methyl)-3,4-difluoro-2-((2-fluoro-4-vinylphenyl)amino)benz oic acid hydrochloride (300 mg), 1-hydroxybenzotriazole (134 mg), 1-(3-dimethylaminopropyl)-3-ethylcarbodiimide hydrochloride (380 mg) and N,N-dimethylformamide (10 mL) were added into a 50 mL single-neck flask, and the system was stirred at room temperature to react for 1 hour. Subsequently, allylhydroxylamine hydrochloride (145 mg) and N,N-diisopropylethylamine (256 mg) were added to the system, and the mixture was continuously stirred at room temperature to react for 2 hours. The reaction system was added into saturated brine to precipitate solids, and subjected to suction filtration. The filter cake was purified by silica gel column chromatography to obtain 254 mg of light yellow solids.

### Step 2: preparation of N-(allyloxy)-3,4-difluoro-5-((3-fluoro-2-((N-methylaminosulfonyl)amino)pyridin-4-yl)methyl)-2-(( 2-fluoro-4-vinylphenyl)amino)benzamide

N-(allyloxy)-5-((2-amino-3-fluoropyridin-4-yl)methyl)-3,4-difluoro-2-((2-fluoro-4-vinylpheny l)amino)benzamide (254 mg), N,N-dimethylformamide (10 mL) and pyridine (425 mg) were added into a 50 mL single-neck flask, and the system was placed in an ice bath and stirred. After cooling to 0°C or a lower temperature, methylaminosulfonyl chloride (210 mg) was slowly dropwise added. After 30 minutes, the reaction was stopped. The system was poured into saturated brine and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 101 mg of off-white solids. ¹H NMR (400 MHz, DMSO-*d*₆): δ 11.81 (s, 1H), 10.42 (s, 1H), 8.57 (s, 1H), 8.03 (d, *J =* 4.0 Hz, 1H), 7.39-7.34 (m, 2H), 7.12 (dd, *J*₁ = 8.4 Hz, *J*₂ = 1.2 Hz, 1H), 7.03 (d, *J* = 4.0 Hz, 1H), 6.95 (s, 1H), 6.85-6.79 (m, 1H), 6.64 (dd, *J*₁ = 17.6 Hz, *J*₂ = 11.2 Hz, 1H), 5.98-5.88 (m, 1H), 5.74 (d, *J =* 17.6 Hz, 1H), 5.28 (d, *J =* 17.2 Hz, 1H), 5.22-5.16 (m, 2H), 4.31 (d, *J =* 4.8 Hz, 2H), 4.07 (s, 2H), 2.53-2.51 (m, 3H). ESI-MS: [M + H]⁺ = 566.2.

### Example 22

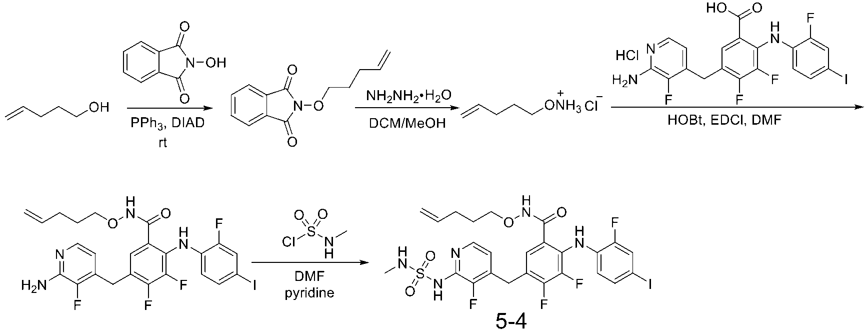

### Step 1: preparation of 2-(pent-4-en-1-yloxy)isoindoline-1,3-dione

Pent-4-en-1-ol (5.0 g), triphenylphosphine (19.8 g), N-hydroxyphthalimide (10.4 g) and tetrahydrofuran (250 mL) were added into a 1 L three-neck flask, and the system was subjected to nitrogen purging. The system was cooled to 0°C or a lower temperature, and tetrahydrofuran solution (15.3 g, 150 mL tetrahydrofuran) of diisopropyl azodicarboxylate was dropwise added. After finishing the addition, the system was taken out of the cooling bath, and stirred at room temperature to react for 16 hours. The reaction system was concentrated, then added into saturated brine, and extracted with ethyl acetate. Organic phases were combined, dried and concentrated. The concentrate was purified by silica gel column chromatography to obtain 10.0 g of light yellow oily substance.

### Step 2: preparation of O-(pent-4-en-1-yl)hydroxylamine hydrochloride

2-(pent-4-en-1-yloxy)isoindoline-1,3-dione (5.0 g) and dichloromethane/methanol (2/1, v/v, 120 mL in total) were added into a 250 mL single-neck flask, and the system was subjected to nitrogen purging and stirred until a clear solution was obtained. The system was slowly dropwise added with hydrazine hydrate (1.2 g). After finishing the addition, the system was continuously stirred to react for 3 hours, and subjected to suction filtration. The filter cake was washed with dichloromethane. Organic phases were combined, and concentrated. The concentrate was diluted with methyl tert-butyl ether, and subjected to suction filtration. Ethyl acetate solution of hydrogen chloride was added to the filtrate to adjust a pH value of the filtrate to 1, and the mixture was concentrated to obtain 1.2 g of off-white solids.

### Step 3: preparation of 5-((2-amino-3-fluoropyridin-4-yl)methyl)-3,4-difluoro-2-((2-fluoro-4-iodophenyl)amino)-N-(pent-4 -en-1-yloxy)benzamide

5-((2-amino-3-fluoropyridin-4-yl)methyl)-3,4-difluoro-2-((2-fluoro-4-iodophenyl)amino)benz oic acid hydrochloride (500 mg), 1-hydroxybenzotriazole (180 mg), 1-(3-dimethylaminopropyl)-3-ethylcarbodiimide hydrochloride (520 mg) and N,N-dimethylformamide (10 mL) were added into a 100 mL single-neck flask, and the system was stirred at room temperature to react for 2 hours. Subsequently, *O*-(pent-4-en-1-yl)hydroxylamine hydrochloride (250 mg) and N,N-diisopropylethylamine (350 mg) were added to the system, and the mixture was continuously stirred at room temperature to react for 2 hours. The reaction system was added into a saturated sodium bicarbonate aqueous solution to precipitate solids, and subjected to suction filtration. The filter cake was purified by silica gel column chromatography to obtain 120 mg of light yellow oily substance. ESI-MS: [M + H]⁺ = 601.1.

### Step 4: preparation of 3,4-difluoro-5-((3-fluoro-2-((N-methylaminosulfonyl)amino)pyridin-4-yl)methyl)-2-((2-fluoro-4-io dophenyl)amino)-N-(pent-4-en-1-yloxy)benzamide

5-((2-amino-3-fluoropyridin-4-yl)methyl)-3,4-difluoro-2-((2-fluoro-4-iodophenyl)amino)-N-(p ent-4-en-1-yloxy)benzamide (120 mg), N,N-dimethylformamide (3 mL) and pyridine (160 mg) were added into a 50 mL single-neck flask, and the system was placed in an ice bath and stirred. After cooling to 0°C or a lower temperature, methylaminosulfonyl chloride (120 mg) was slowly dropwise added to the system. After finishing the addition, the reaction was continued for 30 minutes and then stopped. The system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. Subsequently, the crude product was purified by silica gel column chromatography to obtain 20 mg of light yellow solids. ¹H NMR (400 MHz, DMSO-*d*₆): δ 11.73 (s, 1H), 10.42 (s, 1H), 8.51 (s, 1H), 8.02 (d, *J =* 4.8 Hz, 1H), 7.57 (dd, *J*₁ = 10.8 Hz, *J*₂ = 1.6 Hz, 1H), 7.37-7.33 (m, 2H), 7.03 (d, *J* = 5.6 Hz, 1H), 6.95-6.93 (m, 1H), 6.67-6.62 (m, 1H), 5.85-5.75 (m, 1H), 5.04-4.95 (m, 2H), 4.07 (b, 2H), 3.75 (t, *J* = 6.4 Hz, 2H), 2.52-2.51 (m, 3H), 2.09 (q, *J =* 7.2 Hz, 2H), 1.64-1.58 (m, 2H). ESI-MS: [M + H]⁺ = 694.1.

### Example 23

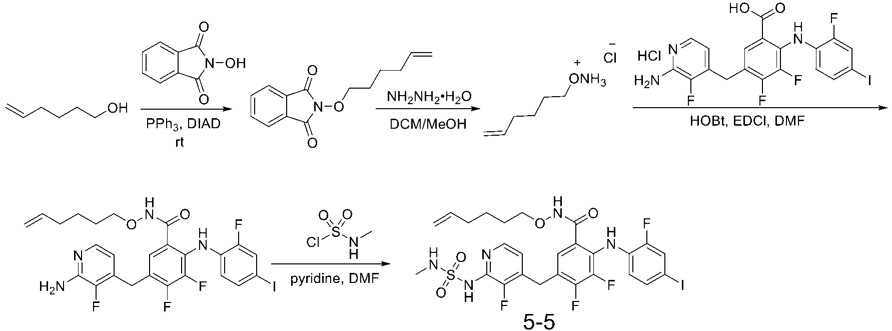

### Step 1: preparation of 2-(hex-5-en-1-yloxy)isoindoline-1,3-dione

Hex-5-en-1-ol (5.0 g), triphenylphosphine (17.0 g), N-hydroxyphthalimide (9.0 g) and tetrahydrofuran (250 mL) were added into a 500 mL three-neck flask, and the system was subjected to nitrogen purging. The system was cooled to 0°C or a lower temperature, and tetrahydrofuran solution (13.1 g, 100 mL tetrahydrofuran) of diisopropyl azodicarboxylate was dropwise added. After finishing the addition, the system was taken out of the cooling bath, and stirred at room temperature to react for 16 hours. The reaction system was concentrated, then added into saturated brine, and extracted with ethyl acetate. Organic phases were combined, dried and concentrated. The concentrate was purified by silica gel column chromatography to obtain 9.2 g of light yellow oily substance.

### Step 2: preparation of O-(hex-5-en-1-yl)hydroxylamine hydrochloride

2-(hex-5-en-1-yloxy)isoindoline-1,3-dione (9.2 g) and dichloromethane/methanol (2/1, v/v, 213 mL in total) were added into a 250 mL single-neck flask, and the system was subjected to nitrogen purging and stirred until a clear solution was obtained. Hydrazine hydrate (2.6 g) was slowly dropwise added to the system. After finishing the addition, the system was continuously stirred to react for 3 hours, and subjected to suction filtration. The filter cake was washed with dichloromethane. Organic phases were combined, and concentrated. The concentrate was diluted with methyl tert-butyl ether, and subjected to suction filtration. Ethyl acetate solution of hydrogen chloride was added to the filtrate to adjust a pH value of the filtrate to 1, and the mixture was concentrated to obtain 3.7 g of off-white solids.

### Step 3: preparation of 5-((2-amino-3-fluoropyridin-4-yl)methyl)-3,4-difluoro-2-((2-fluoro-4-iodophenyl)amino)-N-(hex-5-en-1-yloxy)benzamide

5-((2-amino-3-fluoropyridin-4-yl)methyl)-3,4-difluoro-2-((2-fluoro-4-iodophenyl)amino)benz oic acid hydrochloride (400 mg), 1-hydroxybenzotriazole (126 mg), 1-(3-dimethylaminopropyl)-3-ethylcarbodiimide hydrochloride (296 mg) and N,N-dimethylformamide (10 mL) were added into a 100 mL single-neck flask, and the system was stirred at room temperature to react for 2 hours. Subsequently, O-(hex-5-en-1-yl)hydroxylamine hydrochloride (129 mg) and N,N-diisopropylethylamine (274 mg) were added to the system, and the mixture was continuously stirred at room temperature to react for 2 hours. The reaction system was added into saturated brine to precipitate solids, and subjected to suction filtration. The filter cake was purified by silica gel column chromatography to obtain 200 mg of light yellow solids. ESI-MS: [M + H]⁺ = 615.1.

### Step 4: preparation of 3,4-difluoro-5-((3-fluoro-2-((N-methylaminosulfonyl)amino)pyridin-4-yl)methyl)-2-((2-fluoro-4-io dophenyl)amino)-N-(hex-5-en-1-yloxy)benzamide

5-((2-amino-3-fluoropyridin-4-yl)methyl)-3,4-difluoro-2-((2-fluoro-4-iodophenyl)amino)-N-(h ex-5-en-1-yloxy)benzamide (200 mg), N,N-dimethylformamide (5 mL) and pyridine (257 mg) were added into a 50 mL single-neck flask, and the system was placed in an ice bath and stirred. After cooling to 0°C or a lower temperature, methylaminosulfonyl chloride (175 mg) was slowly dropwise added to the system. After 30 minutes, the reaction was stopped, and the system was poured into a saturated sodium bicarbonate aqueous solution and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 140 mg of off-white solids. ¹H NMR (400 MHz, DMSO-*d*₆): δ 11.70 (s, 1H), 10.41 (s, 1H), 8.50 (s, 1H), 8.02 (d, *J =* 4.8 Hz, 1H), 7.57 (dd, *J*₁ = 10.8 Hz, *J*₂ = 1.6 Hz, 1H), 7.37-7.33 (m, 2H), 7.05-7.01 (m, 1H), 6.94 (t, *J =* 4.8 Hz, 1H), 6.67-6.63 (m, 1H), 5.83-5.73 (m, 1H), 5.02-4.93 (m, 2H), 4.07 (s, 2H), 3.75-3.72 (m, 2H), 2.52-2.50 (m, 3H), 2.05-2.00 (m, 2H), 1.54-1.50 (m, 2H), 1.44-1.35 (m, 2H). ESI-MS: [M + H]⁺ = 708.1.

### Example 24

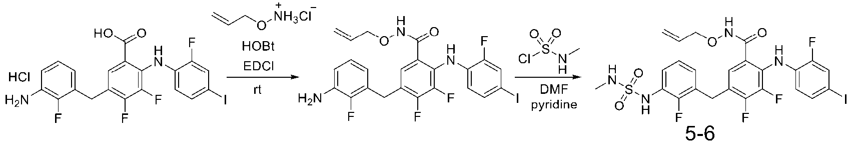

### Step 1 : preparation of N-(allyloxy)-5-(3-amino-2-fluorophenyl)-3,4-difluoro-2-((2-fluoro-4-iodophenyl)amino)benzamide

5-(3-amino-2-fluorobenzyl)-3,4-difluoro-2-((2-fluoro-4-iodophenyl)amino)benzoic acid hydrochloride (400 mg), 1-hydroxybenzotriazole (104 mg), 1-(3-dimethylaminopropyl)-3-ethylcarbodiimide hydrochloride (294 mg) and N,N-dimethylformamide (12 mL) were added into a 100 mL single-neck flask, and the system was stirred at room temperature to react for 2 hours. Subsequently, allylhydroxylamine hydrochloride (93 mg) and N,N-diisopropylethylamine (298 mg) were added to the system, and the mixture was continuously stirred at room temperature to react for 2 hours. The reaction system was added into saturated brine to precipitate solids, and subjected to suction filtration. The filter cake was purified by silica gel column chromatography to obtain 128 mg of light yellow oily substance.

### Step 2: preparation of N-(allyloxy)-3,4-difluoro-5-(2-fluoro-3-((N-methylaminosulfonyl)amino)benzyl)-2-((2-fluoro-4-iod ophenyl)amino)benzamide

N-(allyloxy)-5-(3-amino-2-fluorophenyl)-3,4-difluoro-2-((2-fluoro-4-iodophenyl)amino)benza mide (120 mg), N,N-dimethylformamide (3 mL) and pyridine (265 mg) were added into a 50 mL single-neck flask, and the system was placed in an ice bath and stirred. After cooling to 0°C or a lower temperature, methylaminosulfonyl chloride (272 mg) was slowly dropwise added to the system. After 30 minutes, the reaction was stopped. The system was poured into saturated brine and stirred at room temperature for 10 minutes, and extracted with ethyl acetate. Organic phases were combined, dried with anhydrous sodium sulfate, filtered and subjected to rotary evaporation to remove the solvent. The crude product was purified by silica gel column chromatography to obtain 90 mg of light yellow solids. ¹H NMR (400 MHz, DMSO-*d*₆): δ 11.74 (s, 1H), 9.43 (s, 1H), 8.43 (s, 1H), 7.57 (dd, *J*₁ = 10.8 Hz, *J₂* = 1.6 Hz, 1H), 7.36-7.25 (m, 4H), 7.11 (t, *J =* 8.0 Hz, 1H), 7.02 (t, *J* = 6.8 Hz, 1H), 6.65-6.59 (m, 1H), 5.96-5.86 (m, 1H), 5.29-5.18 (m, 2H), 4.28 (d, *J =* 6.0 Hz, 2H), 4.03 (s, 2H), 2.53-2.51 (m, 3H). ESI-MS: [M + H]⁺ = 665.1.

### Example 25

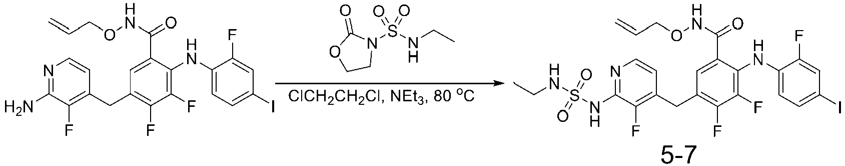

### Preparation of N-(allyloxy)-5-((2-((N-ethylaminosulfonyl)amino)-3-fluoropyridin-4-yl)methyl)-3,4-difluoro-2-((2-fluoro-4-iodophenyl)amino)benzamide

N-ethyl-2-oxooxazoline-3-sulfonamide (848 mg), *N*-(allyloxy)-5-((2-amino-3-fluoropyridin-4-yl)methyl)-3,4-difluoro-2-((2-fluoro-4-iodophenyl)ami no)benzamide (250 mg), triethylamine (487 mg) and 1,2-dichloroethane (20 mL) were added into a 100 mL single-neck flask. The reaction system was heated to 80°C to react for 16.5 hours. After cooling to room temperature, the system was poured into 100 mL of water/tetrahydrofuran mixed solvent (90/10, v/v), an aqueous phase was extracted with ethyl acetate, and organic phases were combined, dried and concentrated to obtain a crude product. The crude product was purified by silica gel column chromatography to obtain 8 mg of yellow solids. ¹H NMR (400 MHz, DMSO-*d*₆): δ 11.78 (s, 1H), 10.41 (s, 1H), 8.49 (s, 1H), 8.03 (d, *J =* 5.2 Hz, 1H), 7.57 (d, *J =* 10.8 Hz, 1H), 7.36-7.32 (m, 2H), 7.18 (t, *J =* 5.6 Hz, 1H), 6.94 (t, *J =* 4.8 Hz, 1H), 6.67-6.62 (m, 1H), 5.97-5.87 (m, 1H), 5.30-5.19 (m, 2H), 4.29 (d, *J =* 6.0 Hz, 2H), 4.07 (s, 2H), 2.97-2.90 (m, 2H), 1.02 (t, *J =* 7.2 Hz, 3H). ESI-MS: [M + H]⁺ = 680.1.

### Example 26

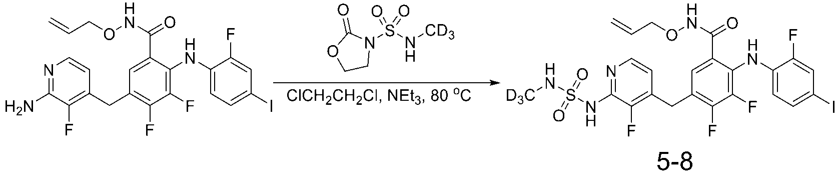

### Preparation of N-(allyloxy)-3,4-difluoro-5-((3-fluoro-2-((N-methyl-d₃-aminosulfonyl)amino)pyridin-4-yl)methyl)-2-((2-fluoro-4-iodophenyl)amino)benzamide

N-methyl-d₃-2-oxooxazoline-3-sulfonamide (800 mg), N-(allyloxy)-5-((2-amino-3-fluoropyridin-4-yl)methyl)-3,4-difluoro-2-((2-fluoro-4-iodophenyl)ami no)benzamide (250 mg), triethylamine (486 mg) and 1,2-dichloroethane (20 mL) were added into a 50 mL single-neck flask. The reaction system was heated to 80°C to react for 5 hours. After cooling to room temperature, the system was poured into a water/tetrahydrofuran mixed solvent (90/10, v/v), an aqueous phase was extracted with ethyl acetate, and organic phases were combined, dried and concentrated to obtain a crude product. The crude product was purified by silica gel column chromatography to obtain 35 mg of yellow solids. ¹H NMR (400 MHz, DMSO-*d*₆): δ 11.78 (s, 1H), 10.42 (s, 1H), 8.50 (s, 1H), 8.02 (d, *J =* 4.8 Hz, 1H), 7.58 (d, *J =* 9.6 Hz, 1H), 7.36-7.32 (m, 2H), 7.01 (s, 1H), 6.94 (s, 1H), 6.68-6.63 (m, 1H), 5.97-5.89 (m, 1H), 5.30-5.19 (m, 2H), 4.29 (d, *J =* 5.6 Hz, 2H), 4.07 (s, 2H). ESI-MS: [M + H]⁺ = 669.1.

### Example 27

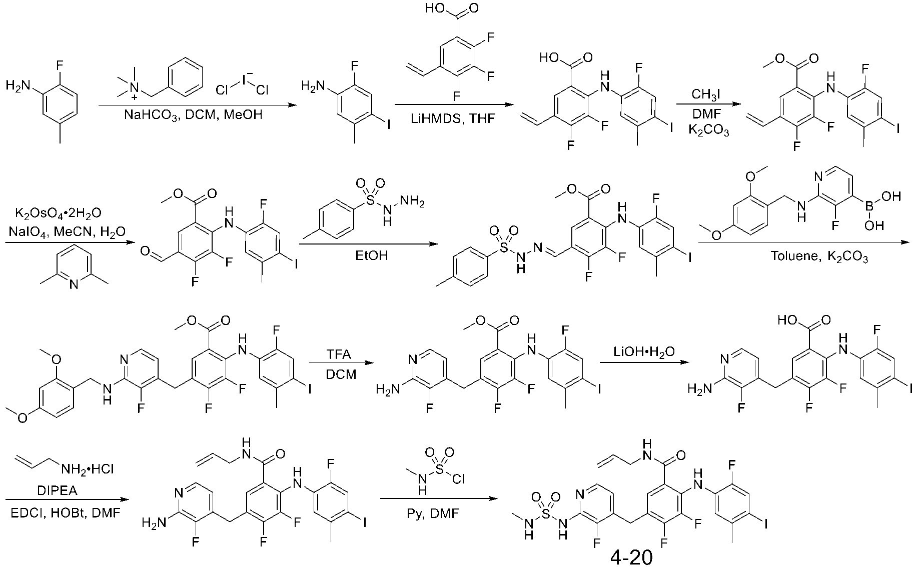

### Step 1: preparation of 2-fluoro-4-iodo-5-methylaniline

2-fluoro-5-methylaniline (10.0 g), sodium bicarbonate (13.4 g), methanol (50 mL) and dichloromethane (50 mL) were added into a 250 mL three-neck flask, and cooled to -5°C and stirred until a clear solution was obtained. Dichloromethane solution (50 mL) of benzyltrimethyldichloroiodate ammonium (27.8 g) was slowly dropwise added to the system. After finishing the dropwise addition, the system was continuously stirred at -5°C to react for 30 minutes, and then gradually heated to room temperature and stirred to react for 2 hours. The reaction system was poured into water, and extracted with dichloromethane. Organic phases were combined, washed with saturated brine, dried and concentrated to obtain 18.4 g of brown liquid.

### Step 2: preparation of 3,4-difluoro-2-((2-fluoro-4-iodo-5-methylphenyl)amino)-5-vinylbenzoic acid

2,3,4-trifluoro-5-vinylbenzoic acid (3.2 g), 2-fluoro-4-iodo-5-methylaniline (4.0 g) and tetrahydrofuran (64 mL) were added into a 250 mL three-neck flask, subjected to nitrogen purging, cooled to -20°C, and lithium bis(trimethylsilyl)amide (1 M, 48 mL) was dropwise added. After finishing the dropwise addition, the system was heated to room temperature and stirred to react for 1 hour. Sodium bisulfate aqueous solution was dropwise added to the system to adjust a pH value of the system to 1-2, and the mixture was extracted with ethyl acetate. Organic phases were combined, washed with saturated brine, dried and concentrated. The concentrate was triturated with an ethyl acetate/n-hexane (v/v = 1/3) mixed solvent to obtain 5.0 g of brown solids.

### Step 3: preparation of methyl 3,4-difluoro-2-((2-fluoro-4-iodo-5-methylphenyl)amino)-5-vinylbenzoate

3,4-difluoro-2-((2-fluoro-4-iodo-5-methylphenyl)amino)-5-vinylbenzoic acid (4.7 g), potassium carbonate (1.8 g) and N,N-dimethylformamide (47 mL) were sequentially added into a 100 mL three-neck flask, stirred at room temperature and methyl iodide (1.9 g) was dropwise added. After finishing the dropwise addition, the system was heated to 35°C and stirred to react for 2 hours. The system was poured into water, and subjected to suction filtration. A filter cake was collected, washed with water and dried to obtain 4.6 g of brownish red solids. ESI-MS: [M + Na]⁺ = 470.0.

### Step 4: preparation of methyl 3,4-difluoro-2-((2-fluoro-4-iodo-5-methylphenyl)amino)-5-formylbenzoate

Methyl 3,4-difluoro-2-((2-fluoro-4-iodo-5-methylphenyl)amino)-5-vinylbenzoate (4.1 g) and acetonitrile (80 mL) were sequentially added into a 250 mL three-neck flask, cooled to 0°C, potassium osmate dihydrate (169 mg) was added and the mixture was stirred to react for 15 minutes. Subsequently, water (80 mL), sodium periodate (5.9 g) and 2,6-dimethylpyridine (986 mg) were added to the system, and the mixture was heated to room temperature and stirred to react for 6 hours. The system was poured into a saturated sodium sulfite aqueous solution, and subjected to suction filtration. A filter cake was collected and washed with water to obtain 4.1 g of brownish grey solids. ESI-MS: [M - H]⁻ = 448.0.

### Step 5: preparation of methyl (E)-3,4-difluoro-2-((2-fluoro-4-iodo-5-methylphenyl)amino)-5-((2-(4-methylphenyl)sulfonylhydraz inylidene)methyl)benzoate

Methyl 3,4-difluoro-2-((2-fluoro-4-iodo-5-methylphenyl)amino)-5-formylbenzoate (4.2 g), p-toluenesulfonyl hydrazide (1.7 g) and anhydrous ethanol (130 mL) were added into a 250 mL three-neck flask, and heated to 60°C to react for 2 hours. Subsequently, the system was cooled to -10°C and stirred for 1 hour, and subjected to suction filtration. A filter cake was collected, washed with cold ethanol and dried to obtain 4.7 g of light brown solids. ESI-MS: [M + Na]⁺ = 640.0.

### Step 6: preparation of methyl 2-((2-fluoro-4-iodo-5-methylphenyl)amino)-5-((2-((2,4-dimethoxybenzyl)amino)-3-fluoropyridin-4 -yl)methyl)-3,4-difluorobenzoate

Methyl (*E*)-3,4-difluoro-2-((2-fluoro-4-iodo-5-methylphenyl)amino)-5-((2-(4-methylphenyl)sulfonylhydraz inylidene)methyl)benzoate (4.7 g), (2-((2,4-dimethoxybenzyl)amino)-3-fluoropyridin-4-yl)boronic acid (3.0 g), anhydrous potassium carbonate (1.4 g) and toluene (140 mL) were added into a 250 mL three-neck flask, subjected to nitrogen purging, heated to 105°C and stirred to react for 6 hours. The reaction system was poured into water, and extracted with ethyl acetate. Organic phases were combined, dried and concentrated to obtain 5.2 g of brown oily substance. The product in this step was directly used in the next step of reaction without further purification. ESI-MS: [M + H]⁺ = 696.1.

### Step 7: preparation of methyl 5-((2-amino-3-fluoropyridin-4-yl)methyl)-2-((2-fluoro-4-iodo-5-methylphenyl)amino)-3,4-difluoro benzoate

Methyl 2-((2-fluoro-4-iodo-5-methylphenyl)amino)-5-((2-((2,4-dimethoxybenzyl)amino)-3-fluoropyridin-4 -yl)methyl)-3,4-difluorobenzoate (6.0 g) and dichloromethane (120 mL) were added into a 250 mL three-neck flask, cooled to 10°C, and dropwise added with trifluoroacetic acid (90 mL). After finishing the dropwise addition, the temperature of the system was returned to room temperature and stirred to react for 1 hour. Saturated sodium bicarbonate aqueous solution was added to the system to adjust pH of the system to neutral, and the mixture was extracted with ethyl acetate. Organic phases were combined, dried and concentrated to obtain 3.0 g of brown solids. ESI-MS: [M + H]⁺ = 546.0.

### Step 8: preparation of 5-((2-amino-3-fluoropyridin-4-yl)methyl)-3,4-difluoro-2-((2-fluoro-4-iodo-5-methylphenyl)amino) benzoic acid

Methyl 5-((2-amino-3-fluoropyridin-4-yl)methyl)-2-((2-fluoro-4-iodo-5-methylphenyl)amino)-3,4-difluoro benzoate (3.0 g), acetonitrile (18 mL) and ethanol (18 mL) were added into a 100 mL single-neck flask, and stirred until a clear solution was obtained. Lithium hydroxide monohydrate (413 mg) aqueous solution (18 mL) was dropwise added to the system. After finishing the dropwise addition, the system was stirred at room temperature to react for 3 hours, sodium bisulfate aqueous solution was added to adjust a pH value of the system to 1-2, and the mixture was subjected to suction filtration. The filtrate was extracted with ethyl acetate. Organic phases were combined, dried and concentrated to obtain 1.3 g of brown solids. ESI-MS: [M - H]⁻ = 530.0.

### Step 9: preparation of N-allyl-5-((2-amino-3-fluoropyridin-4-yl)methyl)-3,4-difluoro-2-((2-fluoro -4-iodo-5-methylphenyl)amino)benzamide

5-((2-amino-3-fluoropyridin-4-yl)methyl)-3,4-difluoro-2-((2-fluoro-4-iodo-5-methylphenyl)am ino)benzoic acid (1.2 g) and N,N-dimethylformamide (24 mL) were added into a 100 mL single-neck flask, and cooled to 0°C. Subsequently, 1-hydroxybenzotriazole (305 mg) and 1-(3-dimethylaminopropyl)-3-ethylcarbodiimide hydrochloride (863 mg) were added to the system, the mixture was stirred to react for 5 minutes, and then heated to room temperature to react for 2 hours. Allylamine hydrochloride (233 mg) and N,N-diisopropylethylamine (583 mg) were added to the reaction flask, and the system was continuously stirred at room temperature to react for 2 hours. The reaction system was slowly added into a saturated sodium bicarbonate solution, and extracted with ethyl acetate. Organic phases were combined, dried and concentrated. The concentrate was purified by silica gel column chromatography to obtain 500 mg of yellow solids. ESI-MS: [M + H]⁺ = 571.1.

### Step 10: preparation of N-allyl-3,4-difluoro-5-((3-fluoro-2-((N-methylaminosulfonyl)amino)pyridin-4-yl)methyl)-2-((2-flu oro-4-iodo-5-methylphenyl)amino)benzamide

N-allyl-5-((2-amino-3-fluoropyridin-4-yl)methyl)-3,4-difluoro-2-((2-fluoro -4-iodo-5-methylphenyl)amino)benzamide (480 mg), N,N-dimethylformamide (6 mL) and pyridine (666 mg) were added into a 25 mL single-neck flask, cooled to -20°C, and methylaminosulfonyl chloride (545 mg) was slowly dropwise added. After finishing the dropwise addition, the system reacted for 30 minutes. The reaction system was poured into water, and extracted with ethyl acetate. Organic phases were combined, dried and concentrated. The concentrate was slurried with methyl tert-butyl ether to obtain 150 mg of off-white solids. ¹H NMR (400 MHz, DMSO-*d*₆): δ 10.41 (s, 1H), 9.00 (s, 1H), 8.87 (t, *J =* 5.6, 1H), 8.02 (d, *J* = 4.8, 1H), 7.62 (d, *J =* 10. 8, 1H), 7.56 (d, *J =* 7.2, 1H), 7.02 (q, *J =* 4.8, 1H), 6.95 (t, *J =* 4.8, 1H), 6.89-6.86 (m, 1H), 5.86-5.77 (m, 1H), 5.17-5.06 (m, 2H), 4.07 (s, 2H), 3.83 (t, *J =* 5.2, 2H), 2.52-2.51 (m, 3H), 2.25 (s, 3H). ESI-MS: [M + H]⁺ = 664.1.

### Example 28

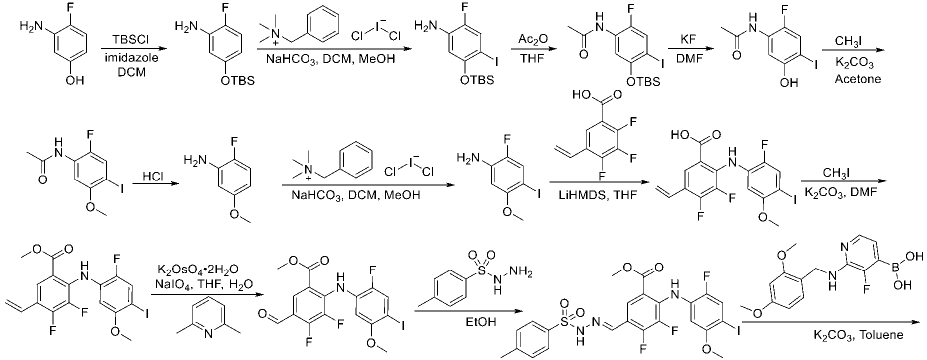

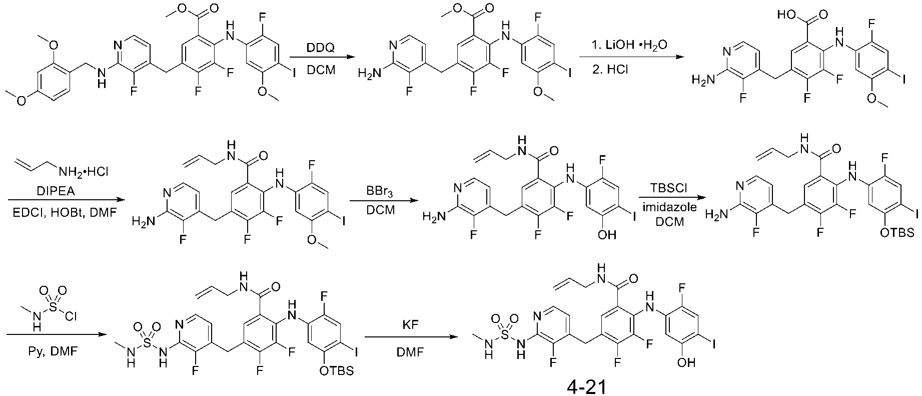

### Step 1: preparation of 5-((tert-butyldimethylsilyl)oxy)-2-fluoroaniline

3-amino-4-fluorophenol (5.0 g), imidazole (4.8 g), tert-butyldimethylchlorosilane (8.9 g) and dichloromethane (150 mL) were added into a 250 mL three-neck flask. After finishing the addition, the system reacted at room temperature for 24 hours. The system was poured into water, organic phases were separated, and an aqueous phase was extracted with dichloromethane. The organic phases were combined, dried and concentrated. The concentrate was purified by silica gel column chromatography to obtain 9.5 g of colorless transparent oily substance. ESI-MS: [M + H]⁺ = 242.1.

### Step 2: preparation of 5-((tert-butyldimethylsilyl)oxy)-2-fluoro-4-iodoaniline

5-((tert-butyldimethylsilyl)oxy)-2-fluoroaniline (9.8 g), sodium bicarbonate (12.2 g), methanol (100 mL) and dichloromethane (100 mL) were added into a 250 mL three-neck flask, stirred and cooled to -5°C. Dichloromethane solution (100 mL) of benzyltrimethyldichloroiodate ammonium (10.6 g) was slowly dropwise added to the system. After finishing the dropwise addition, the system was continuously stirred at -5°C to react for 30 minutes, and then gradually heated to room temperature and stirred to react for 2 hours. The reaction system was poured into water, and extracted with dichloromethane. Organic phases were combined, washed with saturated brine, dried and concentrated. The concentrate was purified by silica gel column chromatography to obtain 9.8 g of orange oily liquid. ESI-MS: [M + H]⁺ = 368.0.

### Step 3: preparation of N-(5-((tert-butyldimethylsilyl)oxy)-2-fluoro-4-iodophenyl)acetamide

5-((tert-butyldimethylsilyl)oxy)-2-fluoro-4-iodoaniline (7.0 g), acetic anhydride (3.0 g) and tetrahydrofuran (100 mL) were added into a 250 mL three-neck flask, and heated to 50°C to react for 3 hours. The reaction system was added into a saturated sodium bicarbonate aqueous solution, and extracted with ethyl acetate. Organic phases were combined, washed with saturated brine, dried and concentrated. The concentrate was purified by silica gel column chromatography to obtain 9.8 g of brown oily liquid.

### Step 4: preparation of N-(2-fluoro-5-hydroxy-4-iodophenyl)acetamide

N-(5-((tert-butyldimethylsilyl)oxy)-2-fluoro-4-iodophenyl)acetamide (21.7 g), potassium fluoride (3.7 g) and N,N-dimethylformamide (200 mL) were added into a 250 mL three-neck flask. After finishing the addition, the system reacted at room temperature for 2 hours. The reaction system was poured into ice water, and extracted with ethyl acetate. Organic phases were combined, washed with saturated brine, dried and concentrated. The concentrate was purified by silica gel column chromatography to obtain 10.0 g of brown solids. ESI-MS: [M + H]⁺ = 296.0.

### Step 5: preparation of N-(2-fluoro-4-iodo-5-methoxyphenyl)acetamide

N-(2-fluoro-5-hydroxy-4-iodophenyl)acetamide (10.0 g), potassium carbonate (7.0 g), methyl iodide (5.8 g) and acetone (100 mL) were added into a 250 mL three-neck flask. After finishing the addition, the system reacted at room temperature for 17 hours. The reaction system was concentrated, and the concentrate was diluted with methyl tert-butyl ether, and then washed with water. Organic phases were dried and concentrated to obtain 10.0 g of brown solids. ESI-MS: [M + H]⁺ = 310.0.

### Step 6: preparation of 2-fluoro-5-methoxyaniline

N-(2-fluoro-4-iodo-5-methoxyphenyl)acetamide (10.0 g), water (100 mL) and concentrated hydrochloric acid (100 mL) were added into a 250 mL three-neck flask, and heated to 90°C and stirred to react for 4 hours. Water was added to the system, and the mixture was extracted with methyl tert-butyl ether. Organic phases were combined, washed with a saturated sodium bicarbonate aqueous solution to be neutral, dried and concentrated. The concentrate was purified by silica gel column chromatography to obtain 3.0 g of brown solids. ESI-MS: [M + MeCN + H]⁺ = 183.1.

### Step 7: preparation of 2-fluoro-4-iodo-5-methoxyaniline

2-fluoro-5-methoxyaniline (2.0 g), sodium bicarbonate (8.9 g), methanol (30 mL) and dichloromethane (30 mL) were added into a 250 mL three-neck flask, and cooled to -5°C and stirred until a clear solution was obtained. Dichloromethane solution (30 mL) of benzyltrimethyldichloroiodate ammonium (7.7 g) was slowly dropwise added to the system. After finishing the dropwise addition, the system was continuously stirred at -5°C to react for 30 minutes, and then gradually heated to room temperature and stirred to react for 2 hours. The reaction system was poured into water, and extracted with dichloromethane. Organic phases were combined, washed with saturated brine, dried and concentrated to obtain 5.0 g of brown oily liquid. The product in this step was directly used in the next step of reaction without further purification.

### Step 8: preparation of 3,4-difluoro-2((2-fluoro-4-iodo-5-methoxyphenyl)amino)-5-vinylbenzoic acid

2-fluoro-4-iodo-5-methoxyaniline (5.0 g), 2,3,4-trifluoro-5-vinylbenzoic acid (3.8 g) and tetrahydrofuran (76 mL) were added into a 250 mL three-neck flask, and stirred at room temperature for 5 minutes. The system was cooled to -20°C in a cooling bath, and lithium bis(trimethylsilyl)amide (1 M, 56.1 mL) was dropwise added. After finishing the dropwise addition, the system was continuously stirred at -20°C to react for 20 minutes, and then gradually heated to room temperature and stirred to react for 2 hours. The system was poured into water, dilute hydrochloric acid was added to adjust a pH value of the system to 2-3, and extracted with ethyl acetate. Organic phases were combined, dried and concentrated. The concentrate was slurried with a petroleum ether/ethyl acetate (v/v = 10/1) mixed solvent to obtain 5.0 g of yellowish-brown solids. ESI-MS: [M - H]⁻ = 448.0.

### Step 9: preparation of methyl 3,4-difluoro-2-((2-fluoro-4-iodo-5-methoxyphenyl)amino)-5-vinylbenzoate

3,4-difluoro-2((2-fluoro-4-iodo-5-methoxyphenyl)amino)-5-vinylbenzoic acid (5.0 g), potassium carbonate (2.3 g), methyl iodide (1.9 g) and N,N-dimethylformamide (50 mL) were added into a 250 mL three-neck flask, and stirred at 35°C to react for 4 hours. The system was poured into water, and extracted with ethyl acetate. Organic phases were combined, dried and concentrated to obtain 5.0 g of brown solids. The product in this step was directly used in the next step of reaction without further purification.

### Step 10: preparation of methyl 3,4-difluoro-2-((2-fluoro-4-iodo-5-methoxyphenyl)amino)-5-formylbenzoate

Methyl 3,4-difluoro-2-((2-fluoro-4-iodo-5-methoxyphenyl)amino)-5-vinylbenzoate (5.0 g) and tetrahydrofuran (75 mL) were added into a 250 mL three-neck flask, and stirred in an ice bath until a clear solution was obtained. Potassium osmate dihydrate (199 mg) was added to the system, and the mixture was continuously stirred in an ice bath to react for 15 minutes. Water (75 mL), sodium periodate (6.9 g) and 2,6-dimethylpyridine (1.2 g) were added to the system, and the mixture was heated to room temperature and stirred to react for 1 hour. The reaction system was poured into a saturated sodium sulfite aqueous solution, and extracted with ethyl acetate. Organic phases were combined, washed with saturated brine, and concentrated to obtain 5.0 g of grey solids. The product in this step was directly used in the next step of reaction without further purification.

### Step 11: preparation of methyl (E)-3,4-difluoro-2-((2-fluoro-4-iodo-5-methoxyphenyl)amino)-5-((2-(4-methylphenyl)sulfonylhydr azinylidene)methyl)benzoate

Methyl 3,4-difluoro-2-((2-fluoro-4-iodo-5-methoxyphenyl)amino)-5-formylbenzoate (5.0 g), p-toluenesulfonyl hydrazide (2.0 g) and ethanol (150 mL) were added into a 250 mL single-neck flask, and heated to 50°C to react for 14 hours. The reaction solution was concentrated, and the concentrate was purified by silica gel column chromatography to obtain 6.8 g of off-white solids. ESI-MS: [M + H]⁺ = 634.0.

### Step 12: preparation of methyl 5-((2-((2,4-dimethoxybenzyl)amino)-3-fluoropyridin-4-yl)methyl)-3,4-difluoro-2-((2-fluoro-4-iodo-5-methoxyphenyl)amino)benzoate

Methyl (*E*)-3,4-difluoro-2-((2-fluoro-4-iodo-5-methoxyphenyl)amino)-5-((2-(4-methylphenyl)sulfonylhydr azinylidene)methyl)benzoate (6.8 g), (2-((2,4-dimethoxybenzyl)amino)-3-fluoropyridin-4-yl)boronic acid (4.9 g), potassium carbonate (3.0 g) and toluene (210 mL) were added into a 250 mL single-neck flask, and heated to 100°C and stirred to react for 3 hours. The reaction system was poured into water, and extracted with ethyl acetate. Organic phases were combined, washed with saturated brine, dried and concentrated. The concentrate was purified by silica gel column chromatography to obtain 3.0 g of white solids. ESI-MS: [M + H]⁺ = 712.1.

### Step 13: preparation of methyl 5-((2-amino-3-fluoropyridin-4-yl)methyl)-3,4-difluoro-2-((2-fluoro-4-iodo-5-methoxyphenyl)amino )benzoate

Methyl 5-((2-((2,4-dimethoxybenzyl)amino)-3-fluoropyridin-4-yl)methyl)-3,4-difluoro-2-((2-fluoro-4-iodo-5-methoxyphenyl)amino)benzoate (2.0 g), 2,3-dichloro-5,6-dicyano-p-benzoquinone (2.5 g), dichloromethane (60 mL) and water (3 mL) were added into a 100 mL single-neck flask, and stirred at room temperature to react for 2 hours. The reaction system was poured into a sodium bicarbonate aqueous solution, and extracted with dichloromethane. Organic phases were combined, washed with saturated brine, dried and concentrated. The concentrate was purified by silica gel column chromatography to obtain 700 mg of off-white solids. ESI-MS: [M + H]⁺ = 562.0.

### Step 14: preparation of 5-((2-amino-3-fluoropyridin-4-yl)methyl)-3,4-difluoro-2-((2-fluoro-4-iodo-5-methoxyphenyl)amino )benzoic acid

Methyl 5-((2-amino-3-fluoropyridin-4-yl)methyl)-3,4-difluoro-2-((2-fluoro-4-iodo-5-methoxyphenyl)amino )benzoate (700 mg), tetrahydrofuran (20 mL) and water (10 mL) were sequentially added into a 100 mL single-neck flask, and placed in an ice bath and stirred. Lithium hydroxide monohydrate (169 mg) was slowly added to the system. After finishing the addition, the system was heated to room temperature and stirred to react for 2.5 hours, hydrochloric acid was added to adjust a pH value to 2-3 and stirred for 5 minutes, and concentrated under reduced pressure to remove the tetrahydrofuran in the system. Subsequently, the concentrate was diluted with 50 mL of water and stirred for 10 minutes, and filtered. A filter cake was collected, washed with water and methyl tert-butyl ether respectively, and dried to obtain 680 mg of yellow solids.

### Step 15: preparation of N-allyl-5-((2-amino-3-fluoropyridin-4-yl)methyl)-3,4-difluoro-2-((2-fluoro -4-iodo-5-methoxyphenyl)amino)benzamide

5-((2-amino-3-fluoropyridin-4-yl)methyl)-3,4-difluoro-2-((2-fluoro-4-iodo-5-methoxyphenyl)a mino)benzoic acid (700 mg) and N,N-dimethylformamide (10 mL) were added into a 100 mL single-neck flask, and cooled to 0°C in an ice bath. 1-hydroxybenzotriazole (208 mg) and 1-(3-dimethylaminopropyl)-3-ethylcarbodiimide hydrochloride (491 mg) were added to the system, the mixture was stirred to react for 5 minutes, and then heated to room temperature to react for 2 hours. Allylamine hydrochloride (240 mg) and N,N-diisopropylethylamine (330 mg) were added to the system, and the mixture was continuously stirred at room temperature to react for 2 hours. The reaction system was slowly added into a saturated sodium bicarbonate solution, and extracted with ethyl acetate. Organic phases were combined, dried and concentrated. The concentrate was purified by silica gel column chromatography to obtain 700 mg of white solids. ESI-MS: [M + H]⁺ = 587.1.

### Step 16: preparation of N-allyl-5-((2-amino-3-fluoropyridin-4-yl)methyl)-3,4-difluoro-2-((2-fluoro-5-hydroxy-4-iodopheny l)amino)benzamide

N-allyl-5-((2-amino-3-fluoropyridin-4-yl)methyl)-3,4-difluoro-2-((2-fluoro -4-iodo-5-methoxyphenyl)amino)benzamide (500 mg) and dichloromethane (110 mL) were added into a 500 mL single-neck flask, and cooled to -15°C. Dichloromethane solution of boron tribromide (1 M, 10 mL) was dropwise added to the system. After finishing the dropwise addition, the system was stirred at -15°C to react for 3 hours. The reaction system was poured into a saturated sodium bicarbonate solution, and extracted with ethyl acetate. Organic phases were combined, dried and concentrated to obtain 450 mg of yellow solids. ESI-MS: [M - H]⁻ = 571.0.

### Step 17: preparation of N-allyl-5-((2-amino-3-fluoropyridin-4-yl)methyl)-2-((5-((tert-butyldimethylsilyl)oxy)-2-fluoro-4-io dophenyl)amino)-3,4-difluorobenzamide

N-allyl-5-((2-amino-3-fluoropyridin-4-yl)methyl)-3,4-difluoro-2-((2-fluoro-5-hydroxy-4-iodop henyl)amino)benzamide (400 mg), imidazole (86 mg), tert-butyl dimethylchlorosilane (158 mg) and dichloromethane (50 mL) were added into a 250 mL three-neck flask, and stirred at room temperature to react for 5.5 hours. The reaction solution was concentrated, and the concentrate was purified by silica gel column chromatography to obtain 400 mg of colorless transparent oily substance. ESI-MS: [M + H]⁺ = 687.1.

### Step 18: preparation of N-allyl-2-((5-((tert-butyldimethylsilyl)oxy)-2-fluoro-4-iodophenyl)amino)-3,4-difluoro-5-((3-fluoro -2-((N-methylaminosulfonyl)amino)pyridin-4-yl)methyl)benzamide

N-allyl-5-((2-amino-3-fluoropyridin-4-yl)methyl)-2-((5-[(tert-butyldimethylsilyl)oxy)-2-fluoro -4-iodophenyl)amino)-3,4-difluorobenzamide (350 mg), N,N-dimethylformamide (7 mL) and pyridine (356 mg) were added into a 50 mL single-neck flask, and cooled to -20°C. Methylaminosulfonyl chloride (175 mg) was slowly dropwise added to the system. After finishing the dropwise addition, the system was continuously stirred at -20°C to react for 30 minutes. The system was poured into water, and extracted with ethyl acetate. Organic phases were combined, dried and concentrated to obtain 350 mg of yellow oily substance.

### Step 19: preparation of N-allyl-3,4-difluoro-5-((3-fluoro-2-((N-methylaminosulfonyl)amino)pyridin-4-yl)methyl)-2-((2-flu oro-5-hydroxy-4-iodophenyl)amino)benzamide

N-allyl-2-((5-((tert-butyldimethylsilyl)oxy)-2-fluoro-4-iodophenyl)amino)-3,4-difluoro-5-((3-f luoro-2-((*N*-methylaminosulfonyl)amino)pyridin-4-yl)methyl)benzamide (350 mg), N,N-dimethylformamide (10 mL) and potassium fluoride (31 mg) were added into a 50 mL single-neck flask, and stirred at room temperature to react for 30 minutes. The system was poured into water, and extracted with ethyl acetate. Organic phases were combined, dried and concentrated. The concentrate was purified by silica gel column chromatography to obtain a crude product, and the crude product was further purified by preparative HPLC to obtain 100 mg of off-white solids. ¹H NMR (400 MHz, DMSO): δ 10.44 (s, 1H), 9.98 (s, 1H), 8.91-8.89 (m, 2H), 8.03 (d, *J =* 4.4 Hz, 1H), 7.61 (d, *J =* 6.4 Hz, 1H), 7.46 (d, *J =* 10.4 Hz, 1H), 7.03 (d, *J =* 3.2 Hz, 1H), 6.95 (s, 1H), 6.35-6.32 (m, 1H), 5.88-5.79 (m, 1H), 5.18-5.06 (m, 2H), 4.09 (s, 2H), 3.85 (t, *J* = 5.6, 2H), 2.51-2.50 (m, 3H). ESI-MS: [M + H]⁺ = 666.0.

### Example 29

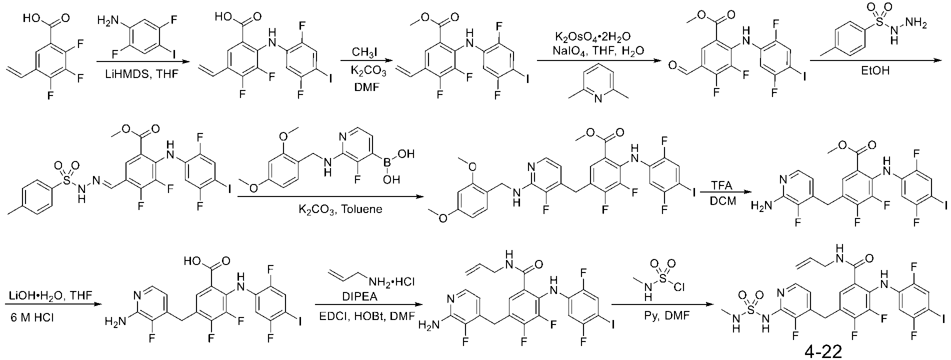

### Step 1: preparation of 2-((2,5-difluoro-4-iodophenyl)amino)-3,4-difluoro-5-vinylbenzoic acid

2,3,4-trifluoro-5-vinylbenzoic acid (3.2 g), 2,5-difluoro-4-iodoaniline (4.0 g) and tetrahydrofuran (78 mL) were added into a 250 mL three-neck flask, subjected to nitrogen purging, cooled to -20°C and stirred to react, and lithium bis(trimethylsilyl)amide (1 M, 47.1 mL) was dropwise added. After finishing the dropwise addition, the system was heated to room temperature and stirred to react for 1 hour, a sodium bisulfate aqueous solution was added to adjust a pH value to 1-2, and the mixture was extracted with methyl tert-butyl ether. Organic phases were combined, washed with a saturated sodium chloride aqueous solution, dried and concentrated. The concentrate was slurried with a methyl tert-butyl ether/n-heptane mixed solvent, and dried to obtain 3.9 g of brownish-yellow solids.

### Step 2: preparation of methyl 2-((2,5-difluoro-4-iodophenyl)amino)-3,4-difluoro-5-vinylbenzoate

2-((2,5-difluoro-4-iodophenyl)amino)-3,4-difluoro-5-vinylbenzoic acid (3.9 g), potassium carbonate (2.0 g) and N,N-dimethylformamide (46 mL) were added into a 250 mL three-neck flask, stirred at room temperature, and methyl iodide (1.5 g) was dropwise added. After finishing the dropwise addition, the system was heated to 35°C to react for 2 hours. The system was poured into water, and subjected to suction filtration. A filter cake was collected to obtain 3.8 g of brown solids.

### Step 3: preparation of methyl 2-((2,5-difluoro-4-iodophenyl)amino)-3,4-difluoro-5-formylbenzoate

Methyl 2-((2,5-difluoro-4-iodophenyl)amino)-3,4-difluoro-5-vinylbenzoate (3.8 g), tetrahydrofuran (76 mL) and potassium osmate dihydrate (155 mg) were added into a 250 mL three-neck flask, and cooled to 0°C and stirred to react for 15 minutes. Water (76 mL), sodium periodate (5.4 g) and 2,6-dimethylpyridine (911 mg) were added to the system, and then the mixture was heated to room temperature and stirred to react for 2.5 hours. The system was poured into a saturated sodium sulfite aqueous solution, and subjected to suction filtration. A filter cake was collected to obtain 3.3 g of brown solids.

### Step 4: preparation of methyl (E)-2-((2,5-difluoro-4-iodophenyl)amino)-3,4-difluoro-5-((2-(4-methylphenyl)sulfonylhydrazinylid ene)methyl)benzoate

Methyl 2-((2,5-difluoro-4-iodophenyl)amino)-3,4-difluoro-5-formylbenzoate (3.3 g), p-toluenesulfonyl hydrazide (1.3 g) and anhydrous ethanol (100 mL) were added into a 250 mL three-neck flask, heated to 60°C to react for 2 hours, then cooled to -10°C and stirred for 30 minutes, and subjected to suction filtration. A filter cake was collected and washed with cold ethanol to obtain 3.4 g of light brown solids. ESI-MS: [M + H]⁺ = 622.0.

### Step 5: preparation of methyl 2-((2,5-difluoro-4-iodophenyl)amino)-5-((2-((2,4-dimethoxybenzyl)amino)-3-fluoropyridin-4-yl)m ethyl)-3,4-difluorobenzoate

Methyl (*E*)-2-((2,5-difluoro-4-iodophenyl)amino)-3,4-difluoro-5-((2-(4-methylphenyl)sulfonylhydrazinylid ene)methyl)benzoate (3.4 g), (2-((2,4-dimethoxybenzyl)amino)-3-fluoropyridin-4-yl)boronic acid (2.2 g), potassium carbonate (1.0 g) and toluene (102 mL) were added into a 250 mL three-neck flask, and heated to 100°C and stirred to react for 6 hours. The system was poured into water, and extracted with ethyl acetate. Organic phases were combined, dried and concentrated to obtain 3.8 g of brownish-yellow oily matter. The product in this step was directly used in the next step of reaction without further purification. ESI-MS: [M + H]⁺ = 700.1.

### Step 6: preparation of methyl 5-((2-amino-3-fluoropyridin-4-yl)methyl)-2-((2,5-difluoro-4-iodophenyl)amino)-3,4-difluorobenzo ate

Methyl 2-((2,5-difluoro-4-iodophenyl)amino)-5-((2-((2,4-dimethoxybenzyl)amino)-3-fluoropyridin-4-yl)m ethyl)-3,4-difluorobenzoate (5.0 g) and dichloromethane (100 mL) were added into a 250 mL three-neck flask, cooled to 10°C, and trifluoroacetic acid (75 mL) was dropwise added. After finishing the dropwise addition, the temperature of the system was returned to room temperature and the system was stirred to react for 1 hour, saturated sodium bicarbonate aqueous solution was added to the system to adjust pH of the system to neutral, and the mixture was extracted with ethyl acetate. Organic phases were combined, dried and concentrated to obtain 1.8 g of brown solids. ESI-MS: [M + H]⁺ = 550.0.

### Step 7: preparation of 5-((2-amino-3-fluoropyridin-4-yl)methyl)-2-((2,5-difluoro-4-iodophenyl)amino)-3,4-difluorobenzoi c acid

Methyl 5-((2-amino-3-fluoropyridin-4-yl)methyl)-2-((2,5-difluoro-4-iodophenyl)amino)-3,4-difluorobenzo ate (1.8 g) and tetrahydrofuran (36 mL) were added into a 100 mL single-neck flask, stirred at room temperature, and lithium hydroxide monohydrate (413 mg) aqueous solution (18 mL) was dropwise added. After finishing the dropwise addition, the system was continuously stirred at room temperature to react for 3 hours, hydrochloric acid was added to adjust a pH value of the system to 1-2, and the mixture was subjected to suction filtration. The filtrate was extracted with ethyl acetate. Organic phases were combined, dried and concentrated to obtain 860 mg of brown solids. ESI-MS: [M - H]⁻ = 534.0.

### Step 8: preparation of N-allyl-5-((2-amino-3-fluoropyridin-4-yl)methyl)-2-((2,5-difluoro-4-iodophenyl)amino)-3,4-difluor obenzamide

5-((2-amino-3-fluoropyridin-4-yl)methyl)-2-((2,5-difluoro-4-iodophenyl)amino)-3,4-difluorob enzoic acid (860 mg) and N,N-dimethylformamide (17 mL) were added into a 100 mL single-neck flask, cooled to 0°C, 1-hydroxybenzotriazole (217 mg) and 1-(3-dimethylaminopropyl)-3-ethylcarbodiimide hydrochloride (613 mg) were added, the mixture was stirred to react for 5 minutes, and then heated to room temperature to react for 2 hours. Allylamine hydrochloride (164 mg) and N,N-diisopropylethylamine (415 mg) were added to the system, and the mixture was continuously stirred at room temperature to react for 2 hours. The reaction system was slowly added into a saturated sodium bicarbonate solution, and extracted with ethyl acetate. Organic phases were combined, dried and concentrated. The concentrate was purified by silica gel column chromatography to obtain 580 mg of yellow solids. ESI-MS: [M + H]⁺ = 575.0.

### Step 9: preparation of N-allyl-2-((2,5-difluoro-4-iodophenyl)amino)-3,4-difluoro-5-((3-fluoro-2-((N-methylaminosulfonyl )amino)pyridin-4-yl)methyl)benzamide

N-allyl-5-((2-amino-3-fluoropyridin-4-yl)methyl)-2-((2,5-difluoro-4-iodophenyl)amino)-3,4-di fluorobenzamide (580 mg), N,N-dimethylformamide (7 mL) and pyridine (799 mg) were added into a 25 mL single-neck flask, cooled to -20°C, and methylaminosulfonyl chloride (654 mg) was slowly dropwise added. After finishing the dropwise addition, the system was stirred for 30 minutes. The system was poured into water, and extracted with ethyl acetate. Organic phases were combined, dried and concentrated. The concentrate was slurried with methyl tert-butyl ether to obtain 410 mg of off-white solids. ¹HNMR (400 MHz, DMSO-*d*₆): δ 10.41 (s, 1H), 9.09 (s, 1H), 8.86 (t, *J =* 5.6, 1H), 8.02 (d, *J*=4.8, 1H), 7.69-7.65 (m, 1H), 7.54 (d, *J* = 7.2, 1H), 7.02 (q, *J =* 5.2, 1H), 6.95 (t, *J =* 5.2, 1H), 6.85-6.80 (m, 1H), 5.85-5.75 (m, 1H), 5.16-5.05 (m, 2H), 4.08 (s, 2H), 3.82 (t, *J* = 5.2, 2H), 2.52-2.51 (m, 3H). ESI-MS: [M + H]⁺ = 668.0.

### Example 30

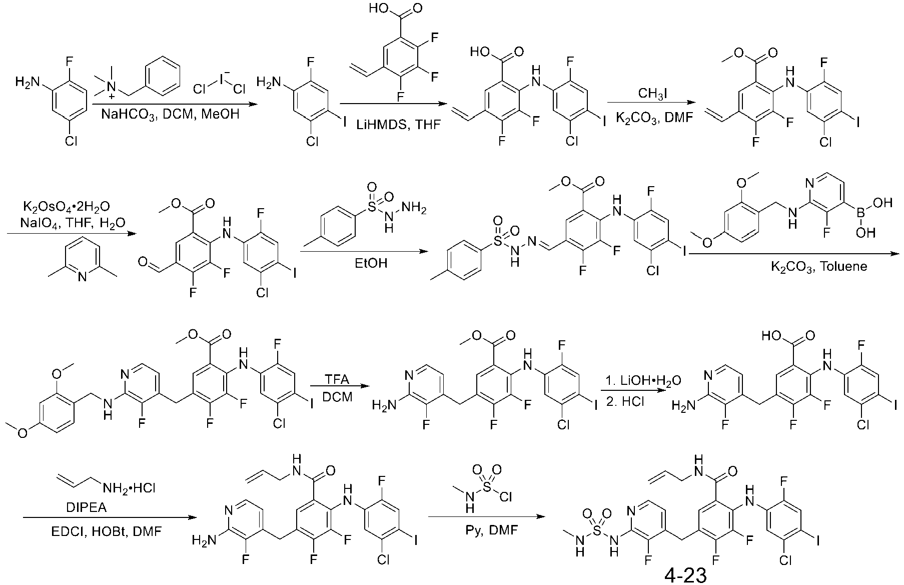

### Step 1: preparation of 5-chloro-2-fluoro-4-iodoaniline

5-chloro-2-fluoroaniline (7.0 g), sodium bicarbonate (8.1 g), methanol (35 mL) and dichloromethane (35 mL) were added into a 250 mL three-neck flask, stirred and cooled to -5°C. dichloromethane solution (100 mL) of benzyltrimethyldichloroiodate ammonium (16.7 g) was slowly dropwise added to the system. After finishing the dropwise addition, the system was continuously stirred at -5°C to react for 30 minutes, and then gradually heated to room temperature and stirred to react for 2 hours. The reaction system was poured into water, and extracted with ethyl acetate. Organic phases were combined, washed with saturated brine, dried and concentrated. The concentrate was purified by silica gel column chromatography to obtain 5.5 g of brown solids.

### Step 2: preparation of 2-((5-chloro-2-fluoro-4-iodophenyl)amino)-3,4-difluoro-5-vinylbenzoic acid

5-chloro-2-fluoro-4-iodoaniline (5.5 g), 2,3,4-trifluoro-5-vinylbenzoic acid (4.1 g) and tetrahydrofuran (40 mL) were added into a 250 mL three-neck flask, subjected to nitrogen purging, cooled to -20°C, and lithium bis(trimethylsilyl)amide (1 M, 62 mL) was slowly dropwise added. After finishing the dropwise addition, the system was continuously stirred at -20°C to react for 10 minutes, and then gradually heated to room temperature to react for 30 minutes. The reaction system was poured into water, 1 M hydrochloric acid was added to adjust a pH value to 3-4, and the mixture was extracted with ethyl acetate. Organic phases were combined, washed with saturated brine, dried and concentrated. The concentrate was slurried with methanol to obtain 5.0 g of brown solids. ESI-MS: [M - H]⁻ = 451.9.

### Step 3: preparation of methyl 2-((5-chloro-2-fluoro-4-iodophenyl)amino)-3,4-difluoro-5-vinylbenzoate

2-((5-chloro-2-fluoro-4-iodophenyl)amino)-3,4-difluoro-5-vinylbenzoic acid (5.0 g), potassium carbonate (3.1 g), N,N-dimethylformamide (40 mL) and methyl iodide (1.9 g) were added into a 100 mL single-neck flask, and stirred at room temperature to react for 5 hours. The reaction system was poured into a saturated ammonium chloride aqueous solution, and extracted with ethyl acetate. Organic phases were combined, washed with saturated brine, dried and concentrated. The concentrate was purified by silica gel column chromatography to obtain 3.9 g of light red product.

### Step 4: preparation of methyl 2-((5-chloro-2-fluoro-4-iodophenyl)amino)-3,4-difluoro-5-formylbenzoate

Methyl 2-((5-chloro-2-fluoro-4-iodophenyl)amino)-3,4-difluoro-5-vinylbenzoate (3.9 g), potassium osmate dihydrate (153 mg) and tetrahydrofuran (60 mL) were added into a 250 mL single-neck flask, and stirred at room temperature to react for 5 minutes. Water (60 mL), sodium periodate (5.3 g) and 2,6-dimethylpyridine (890 mg) were added to the system, and the mixture was continuously stirred at room temperature to react for 4 hours. The reaction system was poured into a saturated sodium sulfite aqueous solution, and extracted with ethyl acetate. Organic phases were combined, washed with saturated brine, dried and concentrated. The concentrate was purified by silica gel column chromatography to obtain 1.8 g of light yellow solids.

### Step 5: preparation of methyl (E)-2-((5-chloro-2-fluoro-4-iodophenyl)amino)-3,4-difluoro-5-((2-(4-methylphenyl)sulfonylhydrazi nylidene)methyl)benzoate

Methyl 2-((5-chloro-2-fluoro-4-iodophenyl)amino)-3,4-difluoro-5-formylbenzoate (1.8 g), p-toluenesulfonyl hydrazide (715 mg) and ethanol (40 mL) were added into a 100 mL single-neck flask, and heated to 50°C to react for 3 hours. The reaction solution was concentrated, and then slurried with n-hexane to obtain 2.3 g of off-white solids.

### Step 6: preparation of methyl 2-((5-chloro-2-fluoro-4-iodophenyl)amino)-5-((2-((2,4-dimethoxybenzyl)amino)-3-fluoropyridin-4-yl)methyl)-3,4-difluorobenzoate

Methyl (*E*)-2-((5-chloro-2-fluoro-4-iodophenyl)amino)-3,4-difluoro-5-((2-(4-methylphenyl)sulfonylhydrazi nylidene)methyl)benzoate (2.3 g), (2-((2,4-dimethoxybenzyl)amino)-3-fluoropyridin-4-yl)boronic acid (1.4 g), potassium carbonate (748 mg) and toluene (80 mL) were added into a 250 mL single-neck flask, and heated to 100°C and stirred to react for 12 hours. The reaction system was poured into water, and extracted with ethyl acetate. Organic phases were combined, washed with saturated brine, dried and concentrated. The concentrate was purified by silica gel column chromatography to obtain 1.1 g of light yellow solids. ESI-MS: [M + H]⁺ = 716.1.

### Step 7: preparation of methyl 5-((2-amino-3-fluoropyridin-4-yl)methyl)-2-((5-chloro-2-fluoro-4-iodophenyl)amino)-3,4-difluorob enzoate

Methyl 2-((5-chloro-2-fluoro-4-iodophenyl)amino)-5-((2-((2,4-dimethoxybenzyl)amino)-3-fluoropyridin-4-yl)methyl)-3,4-difluorobenzoate (1.1 g) and dichloromethane (30 mL) were added into a 50 mL single-neck flask, and stirred at room temperature until a clear solution was obtained. The system was placed in an ice bath, and trifluoroacetic acid (5 mL) was slowly dropwise added. After finishing the addition, the system was heated to room temperature and stirred to react for 1.5 hours. The system was slowly added into a saturated sodium carbonate aqueous solution to adjust a pH value to be 8, and extracted with dichloromethane. Organic phases were combined, dried, filtered and concentrated to obtain 0.8 g of light yellow solids. ESI-MS: [M + H]⁺ = 566.0.

### Step 8: preparation of 5-((2-amino-3-fluoropyridin-4-yl)methyl)-2-((5-chloro-2-fluoro-4-iodophenyl)amino)-3,4-difluorob enzoic acid

Methyl 5-((2-amino-3-fluoropyridin-4-yl)methyl)-2-((5-chloro-2-fluoro-4-iodophenyl)amino)-3,4-difluorob enzoate (1.1 g), tetrahydrofuran (20 mL) and water (20 mL) were sequentially added into a 100 mL single-neck flask, and placed in an ice bath and stirred. Lithium hydroxide monohydrate (250 mg) was slowly added to the system. After finishing the addition, the system was heated to room temperature and stirred to react for 2.5 hours. Hydrochloric acid (6 M) was added to the system to adjust a pH value to 1-2, stirred for 5 minutes, and concentrated under reduced pressure to remove the tetrahydrofuran in the system. Subsequently, the concentrate was diluted with water and stirred for 10 minutes, and filtered. A filter cake was collected, and washed with water and methyl tert-butyl ether sequentially to obtain 900 mg of light yellow solids.

### Step 9: preparation of N-allyl-5-((2-amino-3-fluoropyridin-4-yl)methyl)-2-((5-chloro-2-fluoro-4-iodophenyl)amino)-3,4-d ifluorobenzamide

5-((2-amino-3-fluoropyridin-4-yl)methyl)-2-((5-chloro-2-fluoro-4-iodophenyl)amino)-3,4-difl uorobenzoic acid (900 mg) and N,N-dimethylformamide (20 mL) were added into a 50 mL single-neck flask, cooled to 0°C in an ice bath, 1-hydroxybenzotriazole (240 mg) and 1-(3-dimethylaminopropyl)-3-ethylcarbodiimide hydrochloride (627 mg) were added, the mixture was stirred for 5 minutes, and then heated to room temperature to react for 2 hours. Allylamine hydrochloride (257 mg) and N,N-diisopropylethylamine (631 mg) were added to the system, and the mixture was continuously stirred at room temperature to react for 2 hours. The reaction system was slowly added into a saturated sodium bicarbonate solution, and extracted with ethyl acetate. Organic phases were combined, dried and concentrated. The concentrate was purified by silica gel column chromatography to obtain 580 mg of light yellow solids. ESI-MS: [M + H]⁺ = 591.0.

### Step 10: preparation of N-allyl-2-((5-chloro-2-fluoro-4-iodophenyl)amino)-3,4-difluoro-5-((3-fluoro-2-((N-methylaminosul fonyl)amino)pyridin-4-yl)methyl)benzamide

N-allyl-5-((2-amino-3-fluoropyridin-4-yl)methyl)-2-((5-chloro-2-fluoro-4-iodophenyl)amino)-3,4-difluorobenzamide (580 mg), N,N-dimethylformamide (10 mL) and pyridine (776 mg) were added into a 25 mL single-neck flask, and cooled to -20°C. Methylaminosulfonyl chloride (382 mg) was slowly dropwise added to the system. After finishing the dropwise addition, the system was stirred for 30 minutes. The system was poured into water and stirred at room temperature for 10 minutes, and subjected to suction filtration. The filter cake was dissolved with ethyl acetate, and washed with saturated brine. Organic phases were dried and concentrated. The concentrate was purified by silica gel column chromatography, and then further slurried with an acetone/n-hexane mixed solvent (v/v = 1/6, 105 mL) to obtain 320 mg of off-white solids. ¹H NMR (400 MHz, DMSO-*d*₆): δ 10.41 (s, 1H), 9.06 (s, 1H), 8.85 (t, *J =* 5.2 Hz, 1H), 8.02 (d, *J* = 4.8 Hz, 1H), 7.77 (d, *J* = 10.4 Hz, 1H), 7.53 (d, *J =* 6.8 Hz, 1H), 7.06-7.00 (m, 2H), 6.95 (t, *J =* 4.8 Hz, 1H), 5.83-5.77 (m, 1H), 5.16-5.05 (m, 2H), 4.09 (s, 2H), 3.82 (t, *J =* 5.2 Hz, 2H), 2.52-2.51 (m, 3H). ESI-MS: [M + Na]⁺ = 706.1.

### Preparation of control compound

Control compounds Ref A-1 and Ref A-7 were synthesized according to the reference CN114929669A, which were respectively compounds A-1 and A-7, with structures as follows:

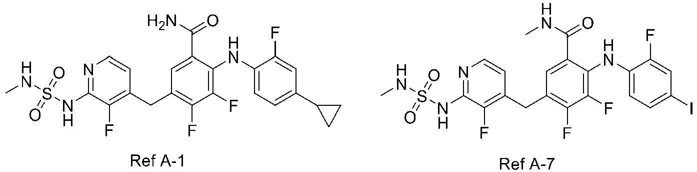

In-vivo therapeutic effect and safety of drugs were results of comprehensive effects of multiple factors, such as inhibitory activity of compounds, various physical and chemical properties, and absorption, metabolism, distribution and excretion. The compounds of the present invention showed superior effectiveness and safety, thereby having a good therapeutic prospect.

### Biological Test

### 1. Proliferation inhibitory activity test against colorectal cancer cells HCT116

Cell proliferation activity was detected by a CellTiter-3D-Glo reagent method. A testing process was as follows: HCT116 cells in logarithmic growth period were digested, diluted to a suitable cell density with McCoy's 5A complete medium containing 10% FBS and 1% PS, inoculated into a low-adhesion 96-well U-bottom plate, and cultured in 5% CO₂; and then a series of to-be-tested compounds with concentration gradient was added, and the system was incubated in a 5% CO₂ incubator. After finishing the incubation, the cells were lysed and detected by using the CellTiter-3D-Glo reagent. Chemiluminescence values were measured by a microplate reader, and data were fitted by Graphpad Prism software, so as to calculate IC₅₀ values of tested substances. Test results can be seen in the following table:
wherein, "A" represents inhibitory activity IC₅₀ < 20 nM, "B" represents inhibitory activity 20 nM ≤ IC₅₀ < 50 nM, and "C" represents inhibitory activity IC₅₀ ≥ 50 nM.

| Compound | IC₅₀ (nM) |
|---|---|
| 4-1 | A |
| 4-5 | A |
| 4-8 | A |
| 4-10 | A |
| 4-14 | A |

It could be seen from the results in the above table that the compounds of the present invention showed significant cell proliferation inhibitory activity against the colorectal cancer cells HCT116.

### 2. 3D model proliferation inhibitory activity test against colorectal cancer cells SW480

Cell proliferation activity was detected by a CellTiter-3D-Glo reagent method. A specific method was as follows: SW480 cells in logarithmic growth period were digested, diluted to a suitable cell density with DMEM complete medium containing 10% FBS and 1% PS, inoculated into a low-adhesion 96-well U-bottom plate, and cultured in 5% CO₂; and then a series of to-be-tested compounds with concentration gradient was added, and incubated in a 5% CO₂ incubator. After finishing the incubation, the cells were lysed and detected by using the CellTiter-3D-Glo reagent. Chemiluminescence values were measured by a microplate reader, and data were fitted by Graphpad Prism software, so as to calculate IC₅₀ values of tested substances. Test results can be seen in the following table:

| Serial number of compound | IC₅₀ (nM) |
|---|---|
| 4-1 | 0.89 |
| 4-10 | 1.05 |
| 4-14 | 2.67 |
| 5-1 | 0.17 |
| 5-2 | 0.63 |
| Ref A-1 | 4.46 |

The test results indicated that the compounds of the present invention showed significant cell proliferation inhibitory activity against the colorectal cancer cells SW480.

### 3. 3D model proliferation inhibitory activity test against lung cancer cells A427

Cell proliferation activity was detected by a CellTiter-3D-Glo reagent method. A specific method was as follows: A427 cells in logarithmic growth period were digested, diluted to a suitable cell density with MEM complete medium containing 10% FBS and 1% PS, inoculated into a low-adhesion 96-well U-bottom plate, and cultured in 5% CO₂; and then a series of to-be-tested compounds with concentration gradient was added, and incubated in a 5% CO₂ incubator. After finishing the incubation, the cells were lysed and detected by using the CellTiter-3D-Glo reagent. Chemiluminescence values were measured by a microplate reader, and data were fitted by Graphpad Prism software, so as to calculate IC₅₀ values of tested substances. Test results can be seen in the following table:

| Serial number of compound | IC₅₀ (nM) |
|---|---|
| 4-1 | 1.16 |
| 4-5 | 2.13 |
| 4-7 | 1.09 |
| 4-8 | 2.88 |
| 4-10 | 0.89 |
| 5-1 | 0.53 |
| Ref A-7 | 9.01 |

The test results indicated that the compounds of the present invention showed significant cell proliferation inhibitory activity against the lung cancer cells A427.

### 4. Pharmacodynamic experiment of subcutaneous xenograft tumor model of pancreatic cancer cells MIA PaCa-2

Animal species and number: BALB/c nude mice, 6 mice in each group.

Experimental samples: compound 4-1 and compound 5-1.

Experimental groups: blank solvent control group, compound 4-1 (0.2 mg/kg, QD × 15 days) and compound 5-1 (0.2 mg/kg, QD × 15 days).

Establishment of animal model: MIA PaCa-2 tumor cells in logarithmic growth period were cultured and collected in vitro, and inoculated subcutaneously in right backs of the Balb/c Nude mice, and when tumors grew to about 180-250 mm³, the tumor-bearing mice were randomly divided into groups. Subsequently, each group of animals was administered with drugs, and the first day of drug administration was defined as the 1^{st} day of the experiment.

Drug administration route and frequency: oral gavage, once per day, for 15 consecutive days.

General state observation: the mice were observed once per day, and observation contents included, but were not limited to, locally administered parts, physical signs, general behaviors, mental states, death and other situations, and other abnormal manifestations of the animals.

Tumor volume calculation: V = 0.5 a × b², wherein a was a long diameter of tumor and b was a short diameter of tumor. Tumor growth inhibition rates TGI (%) were used to evaluate anti-tumor effects of the compounds. TGI (%) = [1 - (mean tumor volume of treatment group at the end of drug administration - mean tumor volume of treatment group at the begining of drug administration) / (mean tumor volume of control group at the end of drug administration - mean tumor volume of control group at the begining of drug administration)] × 100%.

"+" represents tumor inhibition rate < 60%; "++" represents tumor inhibition rate 60%-90%; and +++" represents tumor inhibition rate > 90%.

| Compound | Tumor inhibition rate |
|---|---|
| 4-1 | +++ |
| 5-1 | +++ |

The experimental results showed that the compounds 4-1 and 5-1 had significant inhibitory effects on the growth of subcutaneously transplanted tumors of pancreatic cancer MIA PaCa-2 cells in the nude mice; and during the drug administration, diets, weights and activities of the animals in each drug administration group were normal, thereby showing good safety.

### 5. Pharmacodynamic experiment of subcutaneous xenograft tumor model of colorectal cancer cells SW480

Animal species and number: BALB/c nude mice, 6 mice in each group.

Experimental samples: compound 4-1 and compound Ref A-1.

Experimental groups: blank solvent control group, compound 4-1 (0.2 mg/kg, QD × 18 days) and compound Ref A-1 (0.2 mg/kg, QD × 18 days).

Establishment of animal model: SW480 tumor cells in logarithmic growth period were cultured and collected in vitro, and inoculated subcutaneously in right backs of the Balb/c Nude mice, and when tumors grew to about 180-250 mm³, the tumor-bearing mice were randomly divided into groups. Subsequently, each group of animals were administered with drugs, and the first day of drug administration was defined as the 1^{st} day of the experiment.

Drug administration route and frequency: oral gavage, once per day, for 18 consecutive days.

General state observation: the mice were observed once per day, and observation contents included, but were not limited to, locally administered parts, physical signs, general behaviors, mental states, death and other situations, and other abnormal manifestations of the animals.

Tumor volume calculation: V = 0.5 a × b², wherein a was a long diameter of tumor and b was a short diameter of tumor. Tumor growth inhibition rates TGI (%) were used to evaluate anti-tumor effects of the compounds. TGI (%) = [1 - (mean tumor volume of treatment group at the end of drug administration - mean tumor volume of treatment group at the begining of drug administration) / (mean tumor volume of control group at the end of drug administration - mean tumor volume of control group at the begining of drug administration)] × 100%.

"+" represents tumor inhibition rate < 60%; "++" represents tumor inhibition rate 60%-80%; "+++" represents tumor inhibition rate 80%-100%; and "++++" represents tumor inhibition rate > 100%.

| Compound | Tumor inhibition rate |
|---|---|
| 4-1 | ++++ |
| Ref A-1 | ++ |

The experimental results indicated that the compound 4-1 showed a significant inhibitory effect on the growth of subcutaneously transplanted tumors of colorectal cancer SW480 cells in the nude mice, thereby being significantly better than the control compound Ref A-1.

### 6. In-vivo pharmacokinetic study

Pharmacokinetic study on compound 4-1 in SD rats was conducted. 12 SD rats (half male and half female) were selected and randomly divided into 2 groups (3 rats/sex/group) according to their body weights, and the groups were recorded as a group 1 and a group 2, which were administered with 0.2 mg/kg compound 4-1 by intravenous and oral gavage route respectively. All the experimental animals were administered with a single dose on Day 0, and a drug administration moment was recorded as 0 hour. About 0.25 mL of whole blood (EDTA-K2) was respectively collected from all the experimental animals in group 1 before the drug administration, at 5 minutes, 15 minutes, 30 minutes, 1 hour, 2 hours, 4 hours, 6 hours, 8 hours and 24 hours after finishing the drug administration to prepare plasma; and about 0.25 mL of whole blood (EDTA-K2) was respectively collected from all the experimental animals in group 2 before the drug administration, at 15 minutes, 30 minutes, 1 hour, 2 hours, 4 hours, 6 hours, 8 hours and 24 hours after finishing the drug administration to prepare plasma. The compounds in the plasma samples were quantitatively determined by a LC-MS/MS method, and pharmacokinetic parameters were analyzed and calculated by non-compartmental analysis.

The experimental results indicated that the compound 4-1 showed higher absolute bioavailability (F% > 65%) and greater absorption and clearance rates, thereby indicating higher safety.

## Claims

1. A compound represented by formula (I), or a stereoisomer, a tautomer or a pharmaceutically acceptable salt thereof, 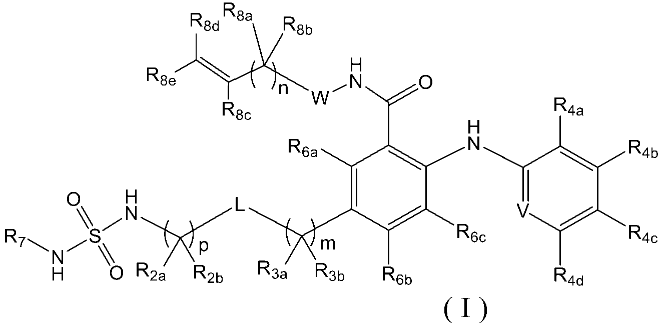
wherein, W is selected from a bond, oxygen, or sulfur;
V is selected from nitrogen or CR₄ₑ;
L is selected from -NR₁C(O)-, -C(O)NR₁-, C₃₋₆ cycloalkyl, 5-10 membered heterocyclyl, C₆₋₁₀ aryl, or 5-10 membered heteroaryl;
R₁ is selected from hydrogen, halogen, C₁₋₆ alkyl, C₁₋₆ deuterated alkyl, C₁₋₆ haloalkyl, C₁₋₆ alkoxy, C₂₋₆ alkenyl, C₂₋₆ alkynyl, or C₃₋₆ cycloalkyl;
each of R₂ₐ, R_{2b}, R₃ₐ, R_{3b}, R₄ₐ, R_{4b}, R_{4c}, R_{4d}, R₄ₑ, R₆ₐ, R_{6b} and R_{6c} is independently selected from hydrogen, deuterium, halogen, aldehyde group, cyano, amino, hydroxyl, C₁₋₆ alkyl, C₁₋₆ deuterated alkyl, C₁₋₆ haloalkyl, C₁₋₆ alkoxy, C₂₋₆ alkenyl, C₂₋₆ alkynyl, or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl, or C₂₋₆ alkynyl, wherein the alkyl, alkoxy, alkenyl, alkynyl ,and cycloalkyl are optionally substituted with one or more substituents selected from deuterium, halogen, amino, C₁₋₆ alkyl, C₁₋₆ alkoxy, C₂₋₆ alkenyl, C₂₋₆ alkynyl and C₃₋₆ cycloalkyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl, C₁₋₆ haloalkyl or C₃₋₆ cycloalkyl;
m is 0, 1, 2, or 3;
n is 1, 2, 3, or 4; and
p is 0, 1, 2, or 3.

2. A compound represented by formula (II), or a stereoisomer, a tautomer or a pharmaceutically acceptable salt thereof, 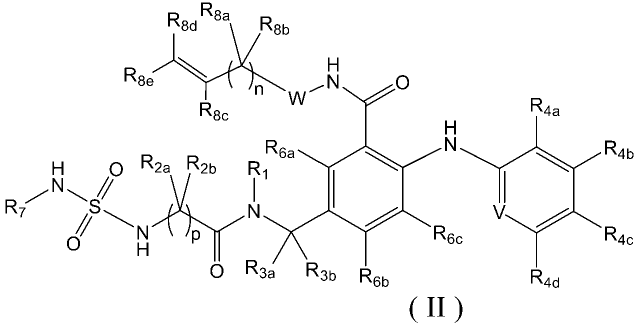
wherein, W is selected from a bond, oxygen, or sulfur;
V is selected from nitrogen or CR₄ₑ;
R₁ is selected from hydrogen, halogen, C₁₋₆ alkyl, C₁₋₆ deuterated alkyl, C₁₋₆ haloalkyl, C₁₋₆ alkoxy, C₂₋₆ alkenyl, C₂₋₆ alkynyl, or C₃₋₆ cycloalkyl;
each of R₂ₐ, R_{2b}, R₃ₐ, R_{3b}, R₄ₐ, R_{4b}, R_{4c}, R_{4d}, R₄ₑ, R₆ₐ, R_{6b} and R_{6c} is independently selected from hydrogen, deuterium, halogen, aldehyde group, cyano, amino, hydroxyl, C₁₋₆ alkyl, C₁₋₆ deuterated alkyl, C₁₋₆ haloalkyl, C₁₋₆ alkoxy, C₂₋₆ alkenyl, C₂₋₆ alkynyl, or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl, or C₂₋₆ alkynyl, wherein the alkyl, alkoxy, alkenyl, alkynyl, and cycloalkyl are optionally substituted with one or more substituents selected from deuterium, halogen, amino, C₁₋₆ alkyl, C₁₋₆ alkoxy, C₂₋₆ alkenyl, C₂₋₆ alkynyl, and C₃₋₆ cycloalkyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl, C₁₋₆ haloalkyl, or C₃₋₆ cycloalkyl;
n is 1, 2, 3, or 4;and
p is 0, 1, 2, or 3.

3. A compound as follows, or a stereoisomer, a tautomeror a pharmaceutically acceptable salt thereof,

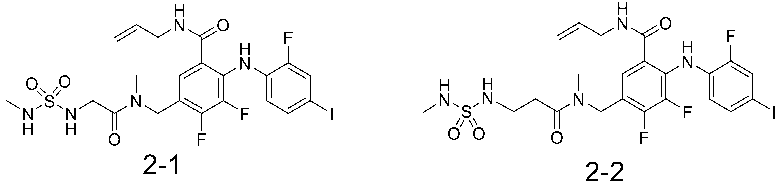

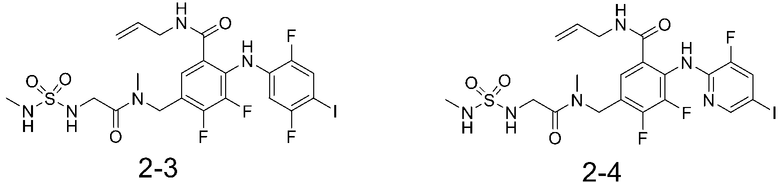

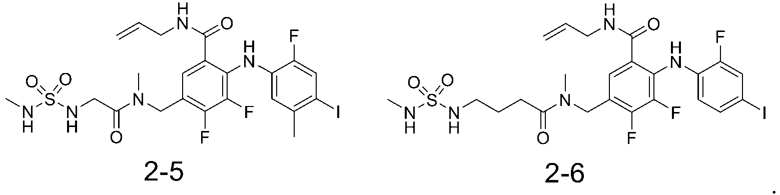

4. A compound represented by formula (III), or a stereoisomer, a tautomer or a pharmaceutically acceptable salt thereof, 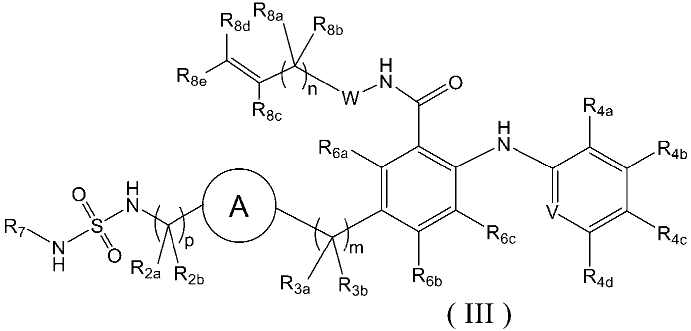
wherein, W is selected from a bond, oxygen, or sulfur;
V is selected from nitrogen or CR₄ₑ;
ring A is selected from C₆₋₁₀ aryl or 5-10 membered heteroaryl;
each of R₂ₐ, R_{2b}, R₃ₐ, R_{3b}, R₄ₐ, R_{4b}, R_{4c}, R_{4d}, R₄ₑ, R₆ₐ, R_{6b} and R_{6c} is independently selected from hydrogen, deuterium, halogen, aldehyde group, cyano, amino, hydroxyl, C₁₋₆ alkyl, C₁₋₆ deuterated alkyl, C₁₋₆ haloalkyl, C₁₋₆ alkoxy, C₂₋₆ alkenyl, C₂₋₆ alkynyl, or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl, or C₂₋₆ alkynyl, wherein the alkyl, alkoxy, alkenyl, alkynyl and cycloalkyl are optionally substituted with one or more substituents selected from deuterium, halogen, amino, C₁₋₆ alkyl, C₁₋₆ alkoxy, C₂₋₆ alkenyl, C₂₋₆ alkynyl and C₃₋₆ cycloalkyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl, C₁₋₆ haloalkyl, or C₃₋₆ cycloalkyl;
m is 0, 1, 2, or 3;
n is 1, 2, 3, or 4;
p is 0, 1, 2, or 3.

5. A compound as follows, or a stereoisomer, a tautomer or a pharmaceutically acceptable salt thereof,

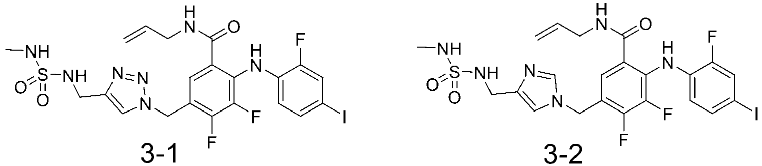

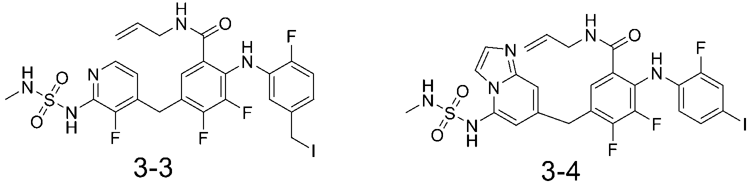

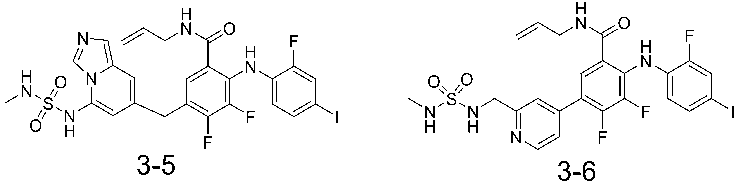

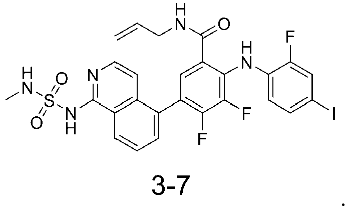

6. A compound represented by formula (IV), or a stereoisomer, a tautomer or a pharmaceutically acceptable salt thereof, 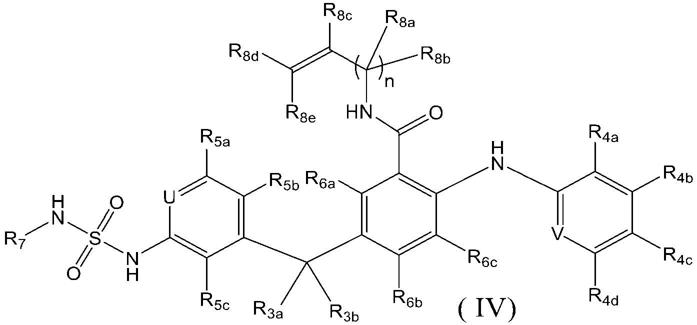
wherein, U is selected from nitrogen or CR_{5d};
V is selected from nitrogen or CR₄ₑ;
each of R₃ₐ, R_{3b}, R₄ₐ, R_{4b}, R_{4c}, R_{4d}, R₄ₑ, R₅ₐ, R_{5b}, R_{5c}, R_{5d}, R₆ₐ, R_{6b} and R_{6c} is independently selected from hydrogen, deuterium, halogen, aldehyde group, cyano, amino, hydroxyl, C₁₋₆ alkyl, C₁₋₆ deuterated alkyl, C₁₋₆ haloalkyl, C₁₋₆ alkoxy, C₂₋₆ alkenyl, C₂₋₆ alkynyl, or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl, or C₂₋₆ alkynyl, wherein the alkyl, alkoxy, alkenyl, alkynyl and cycloalkyl are optionally substituted with one or more substituents selected from deuterium, halogen, amino, C₁₋₆ alkyl, C₁₋₆ alkoxy, C₂₋₆ alkenyl, C₂₋₆ alkynyl, or C₃₋₆ cycloalkyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl, C₁₋₆ haloalkyl or C₃₋₆ cycloalkyl; and
n is 1, 2, 3, or 4.

7. A compound represented by formula (IV-1), or a stereoisomer, a tautomer or a pharmaceutically acceptable salt thereof, 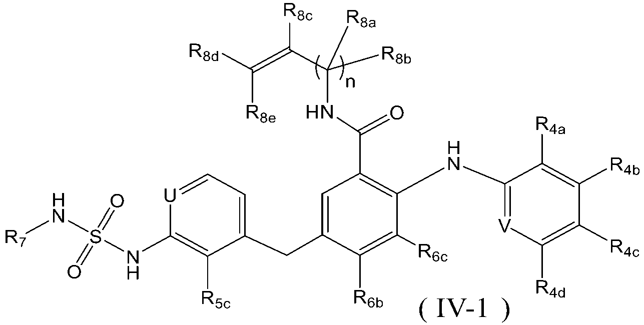
wherein, U is selected from nitrogen or CH;
V is selected from nitrogen or CR₄ₑ;
each of R₄ₐ, R_{4b}, R_{4c}, R_{4d} and R₄ₑ is independently selected from hydrogen, deuterium, halogen, aldehyde group, hydroxyl, C₁₋₆ alkyl, C₁₋₆ alkoxy, C₂₋₆ alkenyl, C₂₋₆ alkynyl or C₃₋₆ cycloalkyl;
each of R_{5c}, R_{6b} and R_{6c} is independently selected from hydrogen, halogen or C₁₋₆ alkyl;
R₇ is selected from hydrogen, C₁₋₆ alkyl, C₁₋₆ deuterated alkyl or C₃₋₆ cycloalkyl;
each of R₈ₐ, R_{8b}, R_{8c}, R_{8d} and R₈ₑ is independently selected from hydrogen, deuterium or C₁₋₆ alkyl; and
n is 1, 2, or 3.

8. A compound as follows, or a stereoisomer, a tautomer or a pharmaceutically acceptable salt thereof,

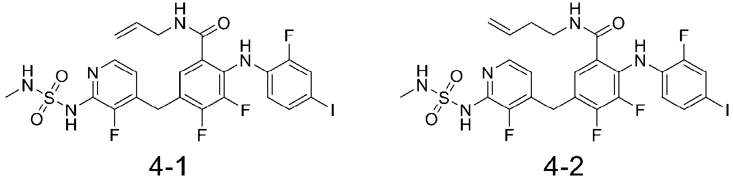

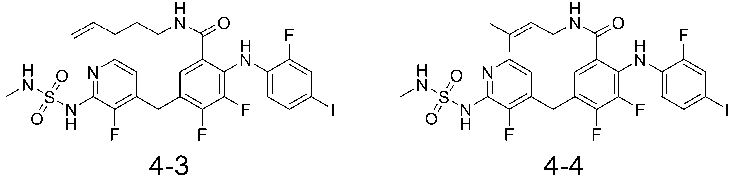

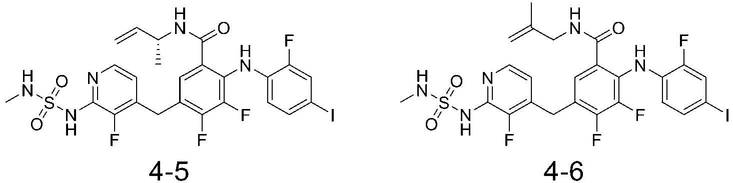

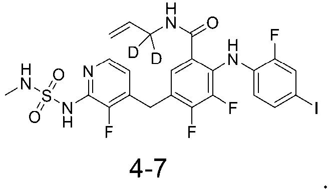

9. A compound as follows, or a stereoisomer, a tautomer or a pharmaceutically acceptable salt thereof,

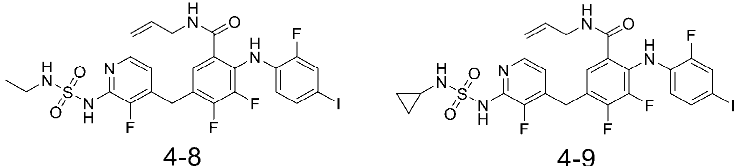

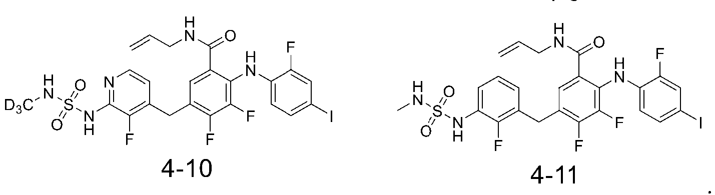

10. A compound as follows, or a stereoisomer, a tautomer or a pharmaceutically acceptable salt thereof,

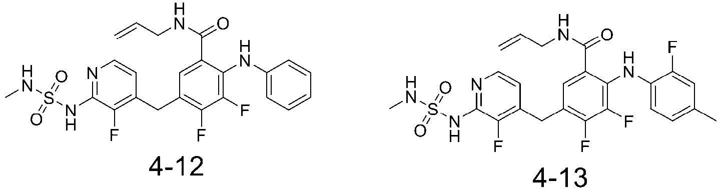

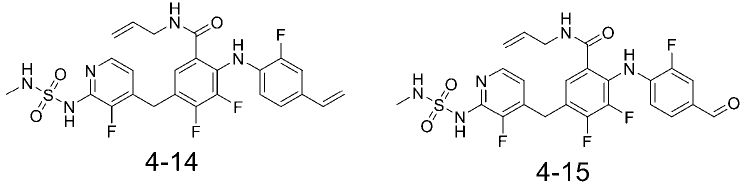

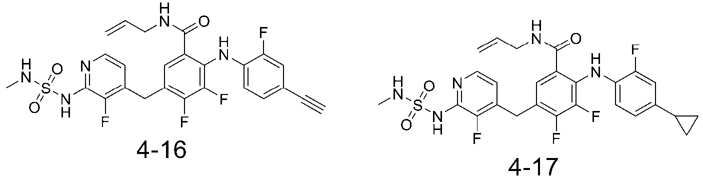

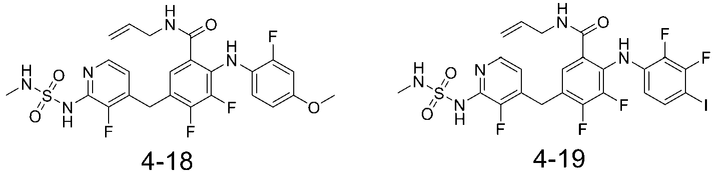

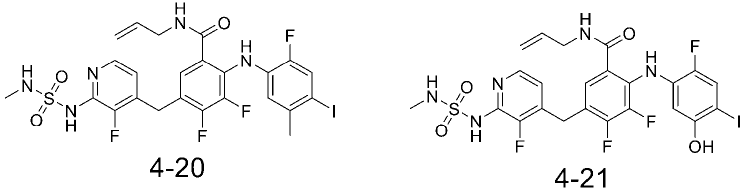

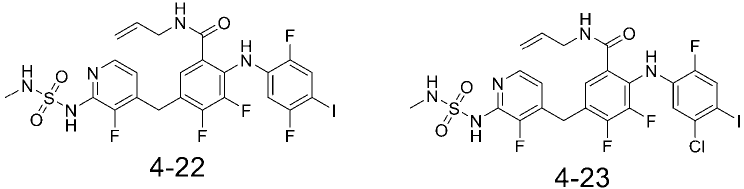

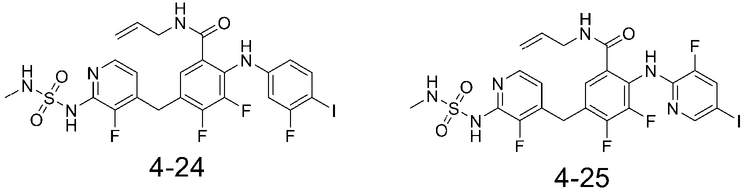

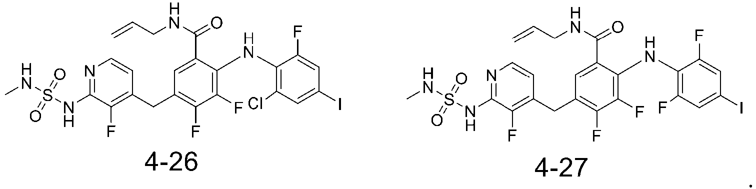

11. A compound represented by formula (V), or a stereoisomer, a tautomer or a pharmaceutically acceptable salt thereof, 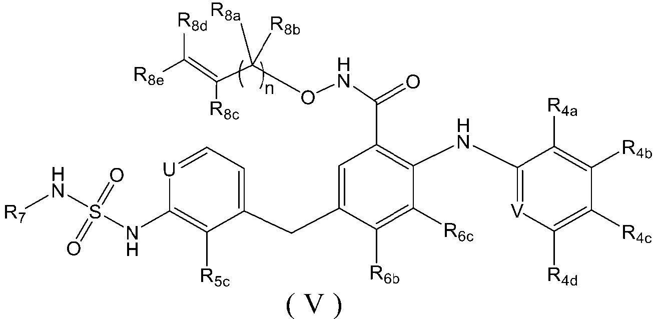
wherein, U is selected from nitrogen or CH;
V is selected from nitrogen or CR₄ₑ;
each of R₄ₐ, R_{4b}, R_{4c}, R_{4d}, R₄ₑ, R_{8c}, R_{6b} and R_{6c} is independently selected from hydrogen, deuterium, halogen, aldehyde group, cyano, amino, hydroxyl, C₁₋₆ alkyl, C₁₋₆ deuterated alkyl, C₁₋₆ haloalkyl, C₁₋₆ alkoxy, C₂₋₆ alkenyl, C₂₋₆ alkynyl or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl, or C₂₋₆ alkynyl, wherein the alkyl, alkoxy, alkenyl, alkynyl, and cycloalkyl are optionally substituted with one or more substituents selected from deuterium, halogen, amino, C₁₋₆ alkyl, C₁₋₆ alkoxy, C₂₋₆ alkenyl, C₂₋₆ alkynyl, and C₃₋₆ cycloalkyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl, C₁₋₆ haloalkyl or C₃₋₆ cycloalkyl; and
n is 1, 2, 3, or 4.

12. A compound as follows, or a stereoisomer, a tautomer or a pharmaceutically acceptable salt thereof,

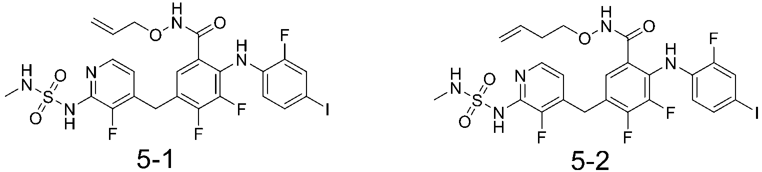

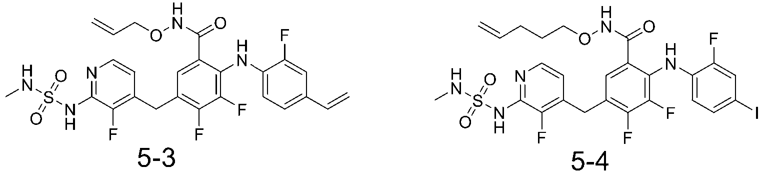

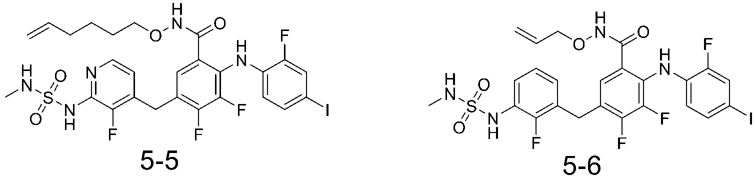

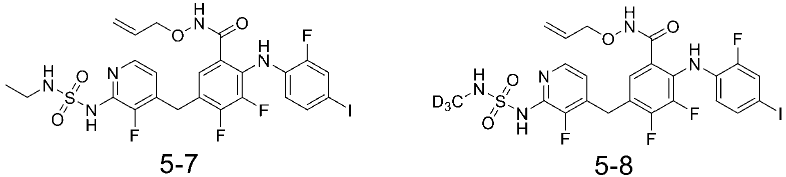

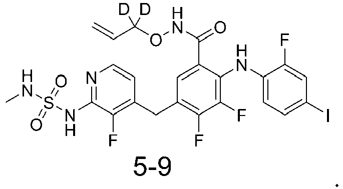

13. A pharmaceutical composition, comprising a therapeutically effective amount of the compound, or the stereoisomer, the tautomer or the pharmaceutically acceptable salt thereof according to any one of claims 1 to 12, and a pharmaceutically acceptable carrier.

14. Use of the compound, or the stereoisomer, the tautomer or the pharmaceutically acceptable salt thereof according to any one of claims 1 to 12, or the pharmaceutical composition according to claim 13 in the preparation of a medicament for preventing and/or treating a tumor.

15. Use of the compound, or the stereoisomer, the tautomer or the pharmaceutically acceptable salt thereof according to any one of claims 1 to 12, or the pharmaceutical composition according to claim 13 in the preparation of a medicament for preventing and/or treating a disease mediated by RAF and/or MEK, wherein the disease is preferably a tumor.

16. The use according to claim 14 or 15, wherein the tumor comprises kidney cancer, liver cancer, breast cancer, pancreatic cancer, prostate cancer, melanoma, leukemia, malignant lymphoma, ovarian cancer, head and neck cancer, lung cancer, colorectal cancer, bladder cancer and uterine cancer.
